# EUROPEAN PATENT APPLICATION

(11) **EP 2 813 499 A1**
(43) Date of publication of application: **17.12.2014**
(21) Application number: 14170051.8
(22) Date of filing: 27.05.2014
(51) Int. Cl.: C07D 403/06, A01N 43/50, A01N 43/653, A61K 31/4178, A61K 31/4196

(54) **Substituted [1,2,4]triazole and imidazole compounds**

(30) Priority: 12.06.2013 EP 13171712
(71) Applicant: BASF SE, 67056 Ludwigshafen (DE)
(72) Inventor: Grammenos, Wassilios, 67071 Ludwigshafen (DE); Craig, Ian Robert, 67063 Ludwigshafen (DE); Boudet, Nadege, 69493 Hirschberg (DE); Müller, Bernd, 67227 Frankenthal (DE); Lauterwasser, Erica May Wilson, 67157 Wachenheim (DE); Lohmann, Jan Klaas, 67245 Lambsheim (DE); Haden, Egon, 67346 Speyer (DE); Escribano Cuesta, Ana, 68161 Mannheim (DE); Grote, Thomas, 67157 Wachenheim (DE)

(57) **Abstract**

The present invention relates to compounds of the formula I 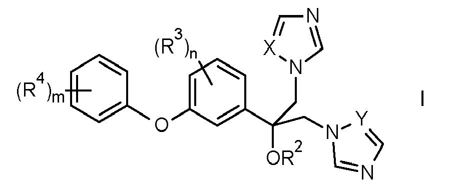 wherein the substituents are described in the specification and claims; to the use of said compounds, to processes for the preparation of the compounds and to intermediates and their use and preparation.

## Description

The present invention relates to substituted [1,2,4]triazole and imidazole compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one inventive compound.

J. of Computer-Aided Molecular Design, 7 (1993), 349-362 describes certain bistriazole-substituted para-biphenyl compounds. EP 0 044 605 relates to fungicidal bisazolyls, in particular bis triazolyl compounds. CN 1923819 relates to bistriazoles. J. Agric. Food Chem. 2009, 57, 4854-4860 describes certain bistriazoles and triazole-imidazoles with para phenoxyphenyl unit and their use as fungicide. CN 1923819 relates to certain para-phenoxyphenyl-bistriazoles.

Using known pesticidal compounds, in many cases, in particular at low application rates, the fungicidal activity of the known fungicidal compounds is unsatisfactory. Based on this, it was an object of the present invention to provide compounds having improved activity and/or a broader activity spectrum against phytopathogenic fungi.

Surprisingly, this object is achieved by the use of the inventive substituted [1,2,4]triazole and imidazole compounds having favorable fungicidal activity against phytopathogenic fungi.

Accordingly, the present invention relates to compounds of the formula I 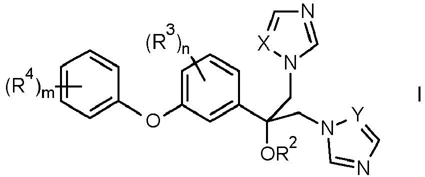 wherein

X is CH or N; Y is CH or N;
R² is C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl;
wherein the aliphatic moieties of R² are not further substituted or carry one, two, three or up to the maximum possible number of identical or different groups R^{2a} which independently of one another are selected from:
R^{2a} halogen, OH, CN, nitro, C₁-C₄-alkoxy, C₃-C₈-cycloalkyl, C₃-C₈-halocycloalkyl and C₁-C₄-halogenalkoxy;
wherein the cycloalkyl and/or phenyl moieties of R² are not further substituted or carry one, two, three, four, five or up to the maximum number of identical or different groups R^{2b} which independently of one another are selected from:
R^{2b} halogen, OH, CN, nitro, C₁-C₄-alkyl, C₁-C₄-alkoxy, C₁-C₄-halogenalkyl, C₃-C₈-cycloalkyl,
C₃-C₈-halocycloalkyl and C₁-C₄-halogenalkoxy;
n is 0, 1, 2, 3 or 4;
R³ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂; wherein each of R³ is unsubstituted or further substituted by one, two, three or four R^{3a}; wherein
R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
m is 0, 1, 2, 3, 4 or 5; and
R⁴ is in each case independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a} wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
p is 0, 1 or 2;
and the N-oxides and the agriculturally acceptable salts thereof.

A further aspect of the present invention are processes for the synthesis of compounds I and intermediates, in particular intermediates of formula II and the N-oxides and the salts thereof. The invention also relates to methods for combating phytopathogenic fungi, and to the use and methods for combating phytopathogenic fungi and to seeds coated with at least one such compound.

The compounds I and II can be obtained for example in analogy to prior art processes known (cf. J.Agric. Food Chem. (2009) 57, 4854-4860; EP 0 126 430 A2), EP 0 044 605, CN 1923819 and by the synthesis routes shown in the following schemes.

For example, phenoles IX are reacted, in a first step, with derivatives VIII, wherein X¹ stands for I or Br, in particular Br (=bromo derivatives VIIIa), preferably in the presence of a base to result in compounds VII. Optionally, this reaction can be carried out under catalysis conditions as known to the skilled person. For example, Di-halo compounds VIII are reacted in a crosscoupling reaction using a catalyst (e.g. Pd) and an organometallic species (e.g. M= MgX, SnR₃, BOR₃, wherein R is for example C₁-C₆-alkyl) in presence or absence of an appropriate additive known to the skilled person to obtain compounds VII.

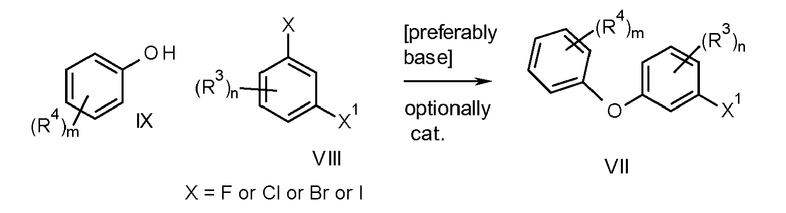

Thereafter, the resulting compounds VII, in particular VIIa (wherein X¹ is Br) are then transformed into Grignard reagents by the reaction with transmetallation reagents such as isopropylmagnesium halides and subsequently reacted with acetyl chloride preferably under anhydrous conditions and preferably in the presence of a catalyst such as CuCl₂, AlCl₃, LiCl and mixtures thereof, to obtain acetophenones VI.

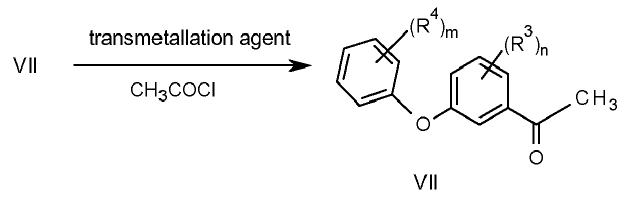

These compounds VI can be halogenated e.g. with bromine preferably in an organic solvent such as diethyl ether, methyl tert.-butyl ether (MTBE), methanol or acetic acid. In the resulting compounds V, "Hal" stands for "halogen" such as e.g. Br or CI. 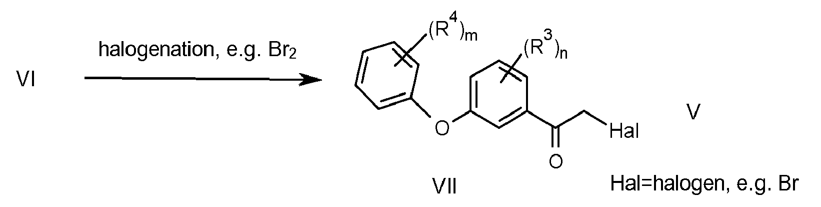 Compounds V can subsequently be reacted with 1 H-1,2,4-triazole or imidazole preferably in the presence of a solvent such as tetrahydrofuran (THF), dimethylformamide (DMF), toluene, and in the presence of a base such as potassium carbonate, sodium hydroxide or sodium hydride to obtain compounds IV.

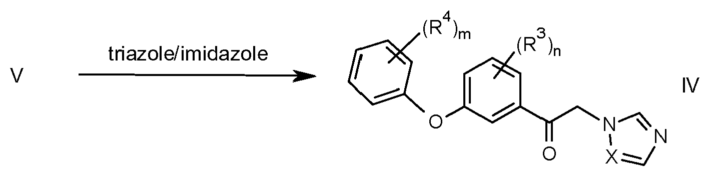

Thereafter, intermediates IV may be reacted with trimethylsulf(ox)onium halides, preferably iodide, preferably in the presence of a base such as sodium hydroxide, to result in epoxides III. 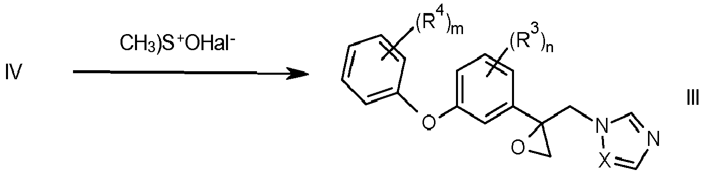 The epoxides II may then be reacted with 1H-1,2,4-triazole or imidazole, respectively, preferably in the presence of a base such as potassium carbonate and preferably in the presence of an organic solvent such as DMF to obtain alcohol compounds II.

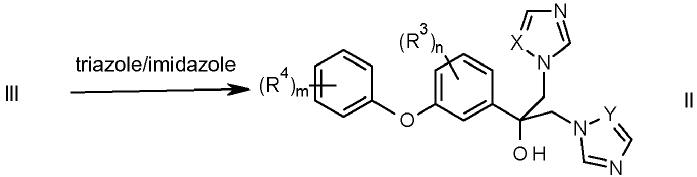

Compounds I can be obtained from compounds II by reacting the alcohol compound of formula II with R²-LG, wherein LG represents a nucleophilically replaceable leaving group, such as halogen, alkylsulfonyl, alkylsulfonyloxy and arylsulfonyloxy, preferably chloro, bromo or iodo, particularly preferably bromo, preferably in the presence of a base. X, Y, R², R³, R⁴, n and m are as defined herein.

A skilled person will readily understand that the preferences for the substituents given herein in connection with compounds I apply for the intermediates accordingly, in particular for compounds II. Thereby, the substituents in each case have independently of each other or more preferably in combination the meanings as defined herein. Some of the intermediates occurring in the synthesis of the inventive compounds are novel and therefore are also an object of the present invention.

In particular, a further object of the present invention are compounds of formula II and the N-oxides and the agriculturally acceptable salts thereof, wherein the variables R³, R⁴, X, Y, n and m are as defined and preferably defined for formula I herein. The substituents are specific embodiments independently of each other or in any combination. One particular embodiment relates to compounds II, wherein n+m is >= 1, namely, 1, 2, 3, 4, 5, 6, 7, 8 or 9, more specifically 1, 2, 3, 4 or 5. In specific embodiments thereof, n+m is 1, 2 or 3. Compounds II are also suitable as fungicides as described herein for compounds of formula I.

According to one embodiment of the intermediate II, X is N and Y is N, corresponding to compounds II.A:

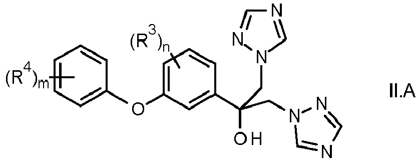

According to a further embodiment of the intermediate II, X is N and Y is CH, corresponding to compounds II.B:

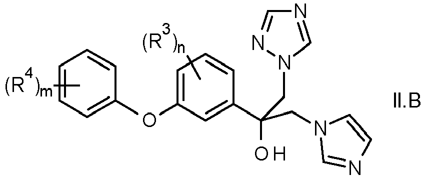

According to still a further embodiment of the intermediate II, X is CH and Y is CH, corresponding to compounds II.C:

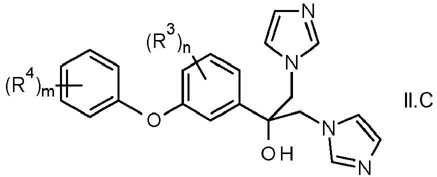

In particular with a view to their use, preference is given to the compounds of the formula II, in particular II.A, II.B and II.C, compiled in the Tables 1d, 1e and 1f below. Each of the groups mentioned for a substituent in the tables is furthermore per se, independently of the combination in which it is mentioned, a particularly preferred aspect of the substituent in question.

Table 1d Compounds of the formula I I.A in which the meaning for the combination of (R³)ₙ and (R⁴)ₘ for each individual compound corresponds in each case to one line of Table A (compounds II.A.A1 to II.A.A2112).

Table 1e Compounds of the formula II.B in which the meaning for the combination of (R³)ₙ and (R⁴)ₘ for each individual compound corresponds in each case to one line of Table A (compounds II.B.A1 to II.B.A2112).

Table 1f Compounds of the formula II.C in which the meaning for the combination of (R³)ₙ and (R⁴)ₘ for each individual compound corresponds in each case to one line of Table A (compounds II.C.A1 to II.C.A2112).

If individual inventive compounds cannot be directly obtained by the routes described above, they may be prepared by derivatization of other inventive compounds.

The N-oxides may be prepared from the inventive compounds according to conventional oxidation methods, e. g. by treating compounds I with an organic peracid such as metachloroper-benzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "Cₗ₁C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-haloalkyl" refers to an alkyl group having 1 or 6 carbon atoms as defined above, wherein some or all of the hydrogen atoms in these groups may be replaced by halogen atoms as mentioned above. Examples are "C₁-C₂-haloalkyl" groups such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₂-C₆-alkenyl" refers to a straight-chain or branched unsaturated hydrocarbon radical having 2 to 6 carbon atoms and a double bond in an position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-haloalkoxy" refers to a C₁-C₆-alkoxy radical as defined above, wherein some or all of the hydrogen atoms in these groups may be replaced by halogen atoms as mentioned above. Examples are "C₁-C₄-haloalkoxy" groups, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoro-ethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

Depending on the substitution pattern, the compounds of formula I and II, respectively, and their N-oxides may have one or more centers of chirality, in which case they are present as pure enantiomers or pure diastereomers or as enantiomer or diastereomer mixtures in any ratio. Both, the pure enantiomers or diastereomers and their mixtures in any ratio are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates, in particular to compounds II.

X according to the invention is N or CH. According to one embodiment X is N. According to a further embodiment X is CH.

Y according to the invention is N or CH. According to one embodiment Y is N. According to a further embodiment Y is CH.

According to one particular embodiment, X and Y are N.

According to a further particular embodiment, X is N and Y is CH.

According to still a further embodiment, X and Y are CH.

According to the invention, R² is C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl, wherein the aliphatic moieties of R² are not further substituted or carry one, two, three or up to the maximum possible number of identical or different groups R^{2a} which independently of one another are selected from halogen, OH, CN, nitro, C₁-C₄-alkoxy, C₃-C₈-cycloalkyl, C₃-C₈-halocycloalkyl and C₁-C₄-halogenalkoxy; and wherein the cycloalkyl and/or phenyl moieties of R² are not further substituted or carry one, two, three, four, five or up to the maximum number of identical or different groups R^{2b} which independently of one another are selected from halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy.

According to one embodiment of the invention, R² is selected from C₁-C₆-alkyl, C₂-C₆-alkenyl, C₂-C₆-alkynyl, wherein the R² are in each case unsubstituted or are substituted by R^{2a} as defined and preferably defined herein, in particular wherein each R^{2a} is independently selected from halogen, OH, CN, C₁-C₄-alkoxy and C₁-C₄-halogenalkoxy, more specifically selected from OH, CN, C₁-C₄-alkoxy and C₁-C₄-halogenalkoxy. Specific embodiments thereof can be found in the below Table P2.

In a further embodiment of the invention, R² is selected from C₁-C₆-alkyl, C₂-C₆-alkenyl and C₂-C₆-alkynyl, wherein the R² are in each case unsubstituted or are substituted by R^{2a} as defined and preferably defined herein. In each case, the substituents may also have the preferred meanings for the respective substituent as defined herein. Specific embodiments thereof can be found in the below Table P2.

According to one particular embodiment, R² is C₁-C₆-alkyl, in particular C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R² is C₁-C₆-alkyl, in particular C₁-C₄-alkyl, that is substituted by one, two or three or up to the maximum possible number of identical or different groups R^{2a}, as defined and preferably defined herein. According to a specific embodiment thereof, R² is C₁-C₆-haloalkyl, in particular C₁-C₄-haloalkyl, more particularly C₁-C₂-haloalkyl. According to a further specific embodiment thereof, R² is C₁-C₄-alkoxy-C₁-C₆-alkyl, in particular C₁-C₄-alkoxy-C₁-C₄-alkyl, such as CH₂OCH₃ or CH₂CH₂OCH₃. According to still a further specific embodiment thereof, R² is hydroxy-C₁-C₆-alkyl, in particular hydroxyl-C₁-C₄-alkyl, such as CH₂CH₂OH. According to a further specific embodiment thereof, R² is cyano-C₁-C₆-alkyl, in particular C₁-C₄-cyano-C₁-C₄-alkyl, such as CH₂CN or CH₂CH₂CN. Further specific embodiments thereof can be found in the below Table P2.

According to still another embodiment, R² is C₃-C₈-cycloalkyl-C₁-C₆-alkyl, in particular C₃-C₆-cycloalkyl-C₁-C₄-alkyl. A further embodiment relates to compounds, wherein R² is C₃-C₈-cycloalkyl-C₁-C₆-alkyl, in particular C₃-C₆-cycloalkyl-C₁-C₄-alkyl, more particularly C₃-C₆-cycloalkyl-C₁-C₂-alkyl, that is substituted by one, two or three or up to the maximum possible number of identical or different groups R^{2a} in the alkyl moiety and/or substituted by one, two, three four or five or up to the maximum possible number of identical or different groups R^{2b} in the cycloalkyl moiety. R^{2a} and R^{2b} are in each case as defined and preferably defined herein. Specific embodiments thereof can be found in the below Table P2.

According to another embodiment, R² is C₂-C₆-alkenyl, in particular C₂-C₄-alkenyl, such as CH₂CH=CH₂, CH₂C(CH₃)=CH₂ or CH₂CH=CHCH₃. A further embodiment relates to compounds, wherein R² is C₂-C₆-alkenyl, in particular C₂-C₄-alkenyl, that is substituted by one, two or three or up to the maximum possible number of identical or different groups R^{2a} as defined and preferably defined herein. According to a specific embodiment thereof, R² is C₂-C₆-haloalkenyl, in particular C₂-C₄-haloalkenyl, such as CH₂C(Cl)=CH₂ and CH₂C(H)=CHCl. According to a further specific embodiment thereof, R² is C₃-C₈-cycloalkyl-C₂-C₆-alkenyl or C₃-C₈-halocycloalkyl-C₂-C₆-alkenyl, in particular C₃-C₆-cycloalkyl-C₂-C₄-alkenyl or C₃-C₆-halocycloalkyl-C₂-C₄-alkenyl. Further specific embodiments thereof can be found in the below Table P2.

According to still another embodiment, R² is C₂-C₆-alkynyl, in particular C₂-C₄-alkynyl, such as CH₂C≡CH or CH₂C≡CCH₃. A further embodiment relates to compounds, wherein R² is C₂-C₆-alkynyl, in particular C₂-C₄-alkynyl, that is substituted by one, two or three or up to the maximum possible number of identical or different groups R^{2a}, as defined and preferably defined herein. According to a specific embodiment thereof, R² is C₂-C₆-haloalkynyl, in particular C₂-C₄-haloalkynyl. According to a further specific embodiment thereof, R² is C₃-C₈-cycloalkyl-C₂-C₆-alkynyl or C₃-C₈-halocycloalkyl-C₂-C₆-alkynyl, in particular C₃-C₆-cycloalkyl-C₂-C₄-alkynyl or C₃-C₆-halocycloalkyl-C₂-C₄-alkynyl. Specific embodiments thereof can be found in the below Table P2.

According to still another embodiment, R² is phenyl-C₁-C₄-alkyl, in particular phenyl-C₁-C₂-alkyl, such as benzyl, wherein the alkyl moiety in each case is unsubstituted or carries one, two or three R^{2a} as defined and preferably defined herein, in particular selected from halogen, in particular F and Cl, C₁-C₄-alkoxy, in particular OCH₃, and CN, and wherein the phenyl in each case is unsubstituted or carries one, two or three R^{2b} as as defined and preferably defined herein, in particular selected from halogen, in particular Cl and F, C₁-C₄-alkoxy, in particular OCH₃, C₁-C₄-alkyl, in particular CH₃ or C₂H₅, and CN. Specific embodiments thereof can be found in the below Table P2.

According to still another embodiment, R² is phenyl-C₂-C₄-alkenyl, in particular phenyl-C₂-C₃-alkenyl, such as phenylethenyl, wherein the alkenyl moiety in each case is unsubstituted or carries one, two or three R^{2a} as defined and preferably defined herein, in particular selected from halogen, in particular F and Cl, C₁-C₄-alkoxy, in particular OCH₃, and CN, and wherein the phenyl in each case is unsubstituted or carries one, two or three R^{2b} as defined and preferably defined herein, in particular selected from halogen, in particular Cl and F, C₁-C₄-alkoxy, in particular OCH₃, C₁-C₄-alkyl, in particular CH₃ or C₂H₅, and CN.

According to still another embodiment, R² is phenyl-C₂-C₄-alkynyl, in particular phenyl-C₂-C₃-alkynyl, such as phenylethinyl, wherein the alkynyl moiety in each case is unsubstituted or carries one, two or three R^{2a}, as defined and preferably defined herein, in particular selected from halogen, in particular F and Cl, C₁-C₄-alkoxy, in particular OCH₃, and CN, and wherein the phenyl in each case is unsubstituted or carries one, two or three R^{2b} as defined and preferably defined herein, in particular selected from halogen, in particular Cl and F, C₁-C₄-alkoxy, in particular OCH₃, C₁-C₄-alkyl, in particular CH₃ or C₂H₅, and CN.

According to still another embodiment, R² is C₃-C₈-cycloalkyl, in particular C₃-C₆-cycloalkyl, such as C₃H₅ (cyclopropyl), C₄H₇ (cyclobutyl), cyclopentyl or cyclohexyl. A further embodiment relates to compounds, wherein R² is C₃-C₈-cycloalkyl, in particular C₃-C₆-cycloalkyl, such as C₃H₅ (cyclopropyl) or C₄H₇ (cyclobutyl), that is substituted by one, two, three four or five or up to the maximum possible number of identical or different groups R^{2b} as defined and preferably defined herein. According to a specific embodiment thereof, R² is C₃-C₈-halocycloalkyl, in particular C₃-C₆-halocycloalkyl, such as halocyclopropyl, in particular 1-F-cyclopropyl or 1-Cl-cyclopropyl. According to a further specific embodiment thereof, R² is C₃-C₈-cycloalkyl-C₃-C₈-cycloalkyl, in particular C₃-C₆-cycloalkyl-C₃-C₆-cycloalkyl, wherein each of said cycloalkyl-cycloalkyl moieties is unsubstituted or carries one, two or three R^{2b} as defined and preferably defined herein.

According to still another embodiment, R² is phenyl, wherein the phenyl is unsubstituted or carries one, two, three, four or five independently selected R^{2b} as defined and preferably defined herein, in particular selected from halogen, in particular Cl and F, C₁-C₄-alkoxy, in particular OCH₃, C₁-C₄-alkyl, in particular CH₃ or C₂H₅, and CN.

Particularly preferred embodiments of R² according to the invention are in Table P2 below, wherein each line of lines P2-1 to P2-87 corresponds to one particular embodiment of the invention, wherein P2-1 to P2-87 are also in any combination a preferred embodiment of the present invention.

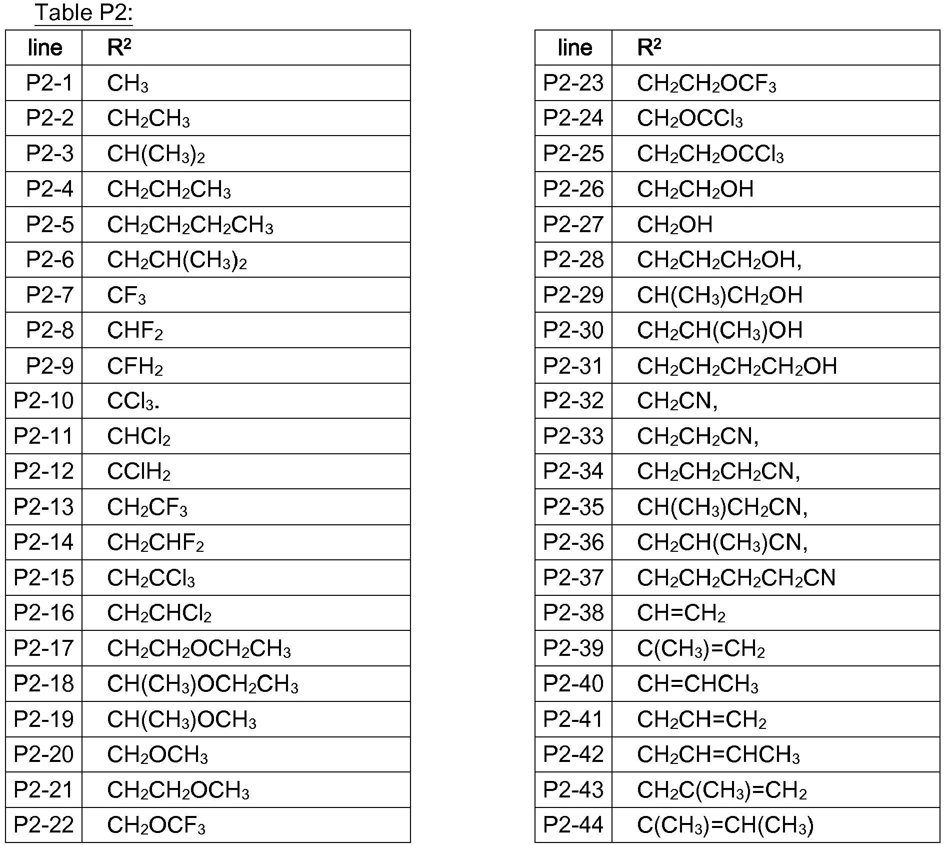

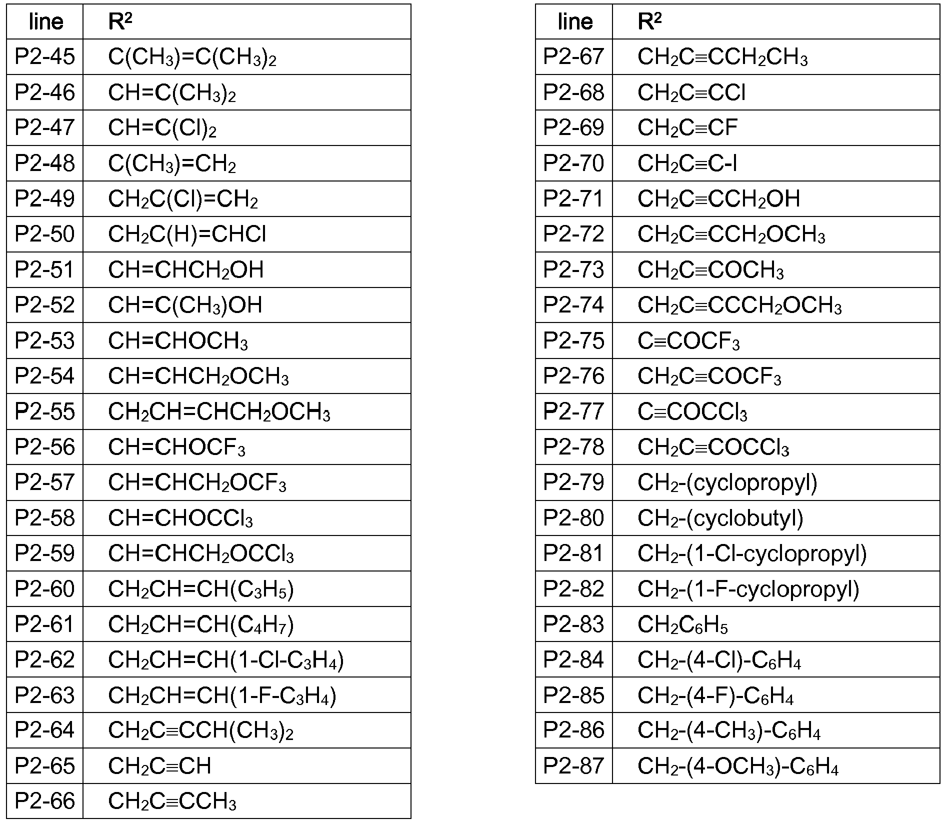

More specific embodiments of R² according to the invention are in Table B below, wherein each line of lines B-1 to B-13 corresponds to one particular embodiment of the invention, wherein B-1 to B-13 are also in any combination preferred embodiments of the present invention.

Each R³ according to the present invention is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R³ is unsubstituted or further substituted by one, two, three or four R^{3a}; wherein R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy.

According to the invention, there can be zero, one, two, three or four R³ present, namely for n is 0, 1, 2, 3 or 4.

According to one embodiment, n is 0.

According to a further embodiment, n is 1.

According to a further embodiment, n is 2 or 3. According to one specific embodiment thereof, n is 2, according to a further specific embodiment, n is 3.

According to one embodiment of the invention, one R³ is attached to the 2-position in relation to the position of the attachment of the tertiary ether group(R³¹). According to one specific embodiment thereof, n is 1, according to a further specific embodiment, n is 2.

According to one embodiment of the invention, one R³ is attached to the 4-position in relation to the position of the attachment of the tertiary ether group (R³²). According to one specific embodiment thereof, n is 1, according to a further specific embodiment, n is 2.

According to one embodiment of the invention, one R³ is attached to the 5-position in relation to the position of the attachment of the tertiary ether group (R³³). According to one specific embodiment thereof, n is 1, according to a further specific embodiment, n is 2.

According to one embodiment of the invention, one R³ is attached to the 6-position in relation to the position of the attachment of the tertiary ether group (R³⁴). According to one specific embodiment thereof, n is 1, according to a further specific embodiment, n is 2.

According to still a further embodiment, n is 1, 2 or 3 and one R³ is in 2-position in relation to the position of the attachment of the tertiary ether group.

According to a further embodiment of the invention, two R³ are attached in 2,4-position in relation to the position of the attachment of the tertiary ether group. According to one specific embodiment thereof, n is 2, according to a further specific embodiment, n is 3.

According to a further embodiment of the invention, two R³ are attached in 2,5-position in relation to the position of the attachment of the tertiary ether group. According to one specific embodiment thereof, n is 2, according to a further specific embodiment, n is 3.

According to a further embodiment of the invention, two R³ are attached in 2,6-position in relation to the position of the attachment of the tertiary ether group. According to one specific embodiment thereof, n is 2, according to a further specific embodiment, n is 3.

According to a further embodiment of the invention, two R³ are attached in 4,5-position in relation to the position of the attachment of the tertiary ether group. According to one specific embodiment thereof, n is 2, according to a further specific embodiment, n is 3.

According to a further embodiment of the invention, two R³ are attached in 4,6-position in relation to the position of the attachment of the tertiary ether group. According to one specific embodiment thereof, n is 2, according to a further specific embodiment, n is 3.

For every R³ (or R³¹, R³², R³³, R³⁴, respectively) that is present in the inventive compounds, the following embodiments and preferences apply independently of the meaning of any other R³ (or R³¹, R³², R³³, R³⁴ respectively) that may be present in the phenyl ring. Furthermore, the particular embodiments and preferences given herein for R³ (or R³¹, R³², R³³, R³⁴, respectively) apply independently for each of n=1, n=2, n=3 and n=4.

According to one embodiment, R³ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R³ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R³ is unsubstituted or further substituted by one, two, three or four R^{3a}, wherein R^{3a} is as defined and preferably defined herein.

According to still a further embodiment, R³ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to still a further embodiment, R³ is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to one specific embodiment, R³ is halogen, in particular Br, F or Cl, more specifically F or Cl.

According to a further specific embodiment, R³ is CN.

According to a further specific embodiment, R³ is C₁-C₆-alkyl, in particular C₁-C₄-alkyl, such as CH₃.

According to a further specific embodiment, R³ is C₁-C₆-haloalkyl, in particular C₁-C₄-haloalkyl, such as CF₃, CHF₂, CH₂F, CCl₃, CHCl₂ or CH₂Cl.

According to a further specific embodiment, R³ is C₁-C₆-alkoxy, in particular C₁-C₄-alkoxy, more specifically C₁-C₂-alkoxy such as OCH₃ or OCH₂CH₃.

According to a further specific embodiment, R³ is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R³ is C₂-C₆-alkenyl or C₂-C₆-haloalkenyl, in particular C₂-C₄-alkenyl or C₂-C₄-haloalkenyl, such as CH=CH₂.

According to still a further embodiment, R³ is C₂-C₆-alkynyl or C₂-C₆-haloalkynyl, in particular C₂-C₄-alkynyl or C₂-C₄-haloalkynyl, such as CΞCH.

According to still a further embodiment, R³ is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R³ is C(=O)(OH) or C(=O)(O-C₁-C₄-alkyl), in particular C(=O)(OCH₃).

According to still a further embodiment, R³ is selected from S(C₁-C₂-alkyl), S(O)(C₁-C₂-alkyl) and S(O)₂(C₁-C₂-alkyl), in particular SCH₃, S(O)(CH₃) and S(O)₂(CH₃).

According to still a further embodiment, R³ is unsubstituted phenyl or phenyl that is substituted by one, two, three or four R^{3a}, as defined herein.

According to still a further embodiment, R³ is unsubstituted phenoxy or phenoxy that is substituted by one, two, three or four R^{3a}, as defined herein.

According to still a further embodiment, R³ is unsubstituted 5- or 6-membered heteroaryl. According to still a further embodiment, R³ is 5- or 6-membered heteroaryl that is substituted by one, two or three R^{3a}, as defined herein. According to one specific embodiment, the heteroaryl in each case is 5-membered such as . According to a further specific embodiment, the heteroaryl in each case is 6-membered such as .

According to still a further embodiment, R³ is unsubstituted 5- or 6-membered heteroaryloxy. According to still a further embodiment, R³ is 5- or 6-membered heteroaryloxy that is substituted by one, two or three R^{3a}, as defined herein. According to one specific embodiment, the heteroaryloxy in each case is 5-membered. According to a further specific embodiment, the heteroaryloxy in each case is 6-membered.

R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy, in particular selected from halogen, CN, C₁-C₂-alkyl, C₁-C₂-haloalkyl, C₃-C₆-cycloalkyl, C₃-C₆-halocycloalkyl, C₁-C₂-alkoxy and C₁-C₂-halogenalkoxy. Specifically, R^{3a} is independently selected from F, Cl, CN, OH, CH₃, halomethyl, cyclopropyl, halocyclopropyl, OCH₃ and halogenmethoxy.

Particularly preferred embodiments of R³ according to the invention are in Table P3 below, wherein each line of lines P3-1 to P3-16 corresponds to one particular embodiment of the invention, wherein P3-1 to P3-16 are also in any combination with one another a preferred embodiment of the present invention. Thereby, for every R³ that is present in the inventive compounds, these specific embodiments and preferences apply independently of the meaning of any other R³ that may be present in the phenyl ring:

**Table P3:**

| **No.** | **R³** | | **No.** | **R³** | | **No.** | **R³** |
|---|---|---|---|---|---|---|---|
| P3-1 | Cl | | P3-7 | CF₃ | | P3-13 | SCH₃ |
| P3-2 | F | | P3-8 | CHF₂ | | P3-14 | SOCH₃ |
| P3-3 | CN | | P3-9 | OCH₃ | | P3-15 | SO₂CH₃ |
| P3-4 | NO₂ | | P3-10 | OCH₂CH₃ | | P3-16 | CO₂CH₃ |
| P3-5 | CH₃ | | P3-11 | OCF₃ | | | |
| P3-6 | CH₂CH₃ | | P3-12 | OCHF₂ | | | |

Particularly preferred embodiments of (R³)ₙ according to the invention are in Table P31 below, wherein each line of lines P31-1 to P31-103 corresponds to one particular embodiment of the invention, wherein P31-1 to P31-103 are also in any combination preferred embodiments of the present invention. The positions of the R³ are given in relation to the position of the attachment of the tertiary ether group.

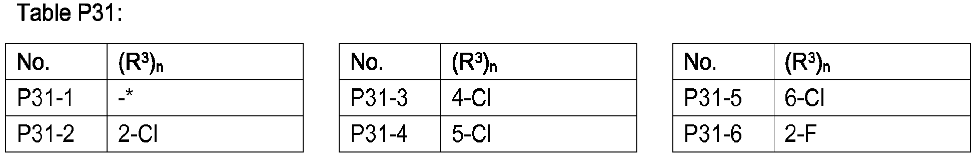

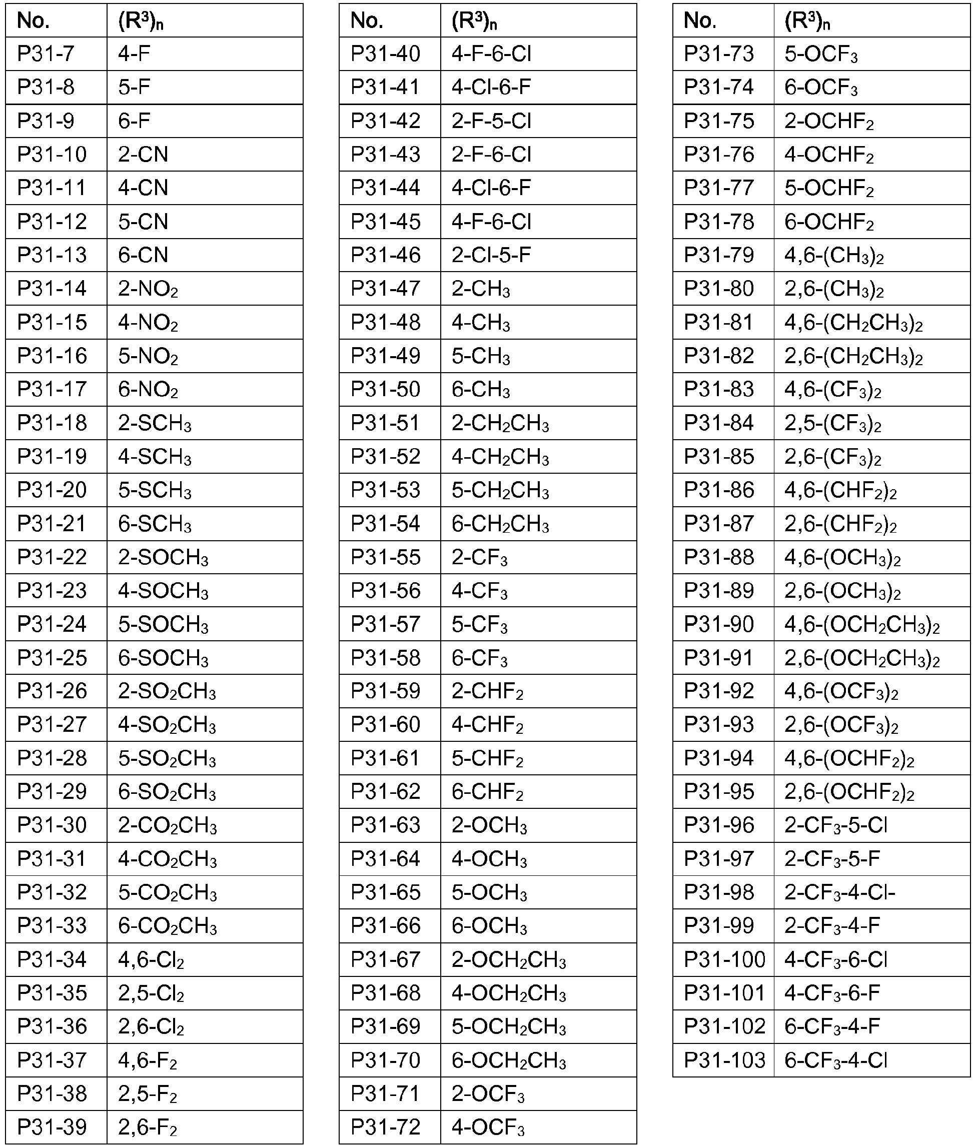

More specific embodiments of (R³)ₙ according to the invention are in Table P32 below, wherein each line of lines P32-1 to P32-47 corresponds to one particular embodiment of the invention, wherein P32-1 to P32-47 are also in any combination preferred embodiments of the present invention. The positions of the R³ are given in relation to the position of the attachment of the tertiary ether group.

**Table P32:**

| **No.** | **(R³)ₙ** | | **No.** | **(R³)ₙ** | | **No.** | **(R³)ₙ** |
|---|---|---|---|---|---|---|---|
| P32-1 | -***** | | P32-17 | 4,6-F₂ | | P32-33 | 4-CHF₂ |
| P32-2 | 2-Cl | | P32-18 | 2,5-F₂ | | P32-34 | 5-CHF₂ |
| P32-3 | 4-Cl | | P32-19 | 2,6-F₂ | | P32-35 | 6-CHF₂ |
| P32-4 | 5-Cl | | P32-20 | 4-F-6-Cl | | P32-36 | 2-OCH₃ |
| P32-5 | 6-Cl | | P32-21 | 4-Cl-6-F | | P32-37 | 4-OCH₃ |
| P32-6 | 2-F | | P32-22 | 2-F-6-Cl | | P32-38 | 5-OCH₃ |
| P32-7 | 4-F | | P32-23 | 2-Cl-5-F | | P32-39 | 6-OCH₃ |
| P32-8 | 5-F | | P32-24 | 2-CH₃ | | P32-40 | 4,6-(CF₃)₂ |
| P32-9 | 6-F | | P32-25 | 4-CH₃ | | P32-41 | 2,5-(CF₃)₂ |
| P32-10 | 2-CN | | P32-26 | 5-CH₃ | | P32-42 | 2,4-(CF₃)₂ |
| P32-11 | 4-CN | | P32-27 | 6-CH₃ | | P32-43 | 2,6-(CF₃)₂ |
| P32-12 | 5-CN | | P32-28 | 2-CF₃ | | P32-44 | 4-CF₃-6-Cl |
| P32-13 | 6-CN | | P32-29 | 4-CF₃ | | P32-45 | 4-CF₃-6-F |
| P32-14 | 4,6-Cl₂ | | P32-30 | 5-CF₃ | | P32-46 | 4-Cl-6-CF₃ |
| P32-15 | 2,5-Cl₂ | | P32-31 | 6-CF₃ | | P32-47 | 4-F-6-CF₃ |
| P32-16 | 2,6-Cl₂ | | P32-32 | 2-CHF₂ | | | |

| | | | | | | | |
|---|---|---|---|---|---|---|---|
| -* means that n=0 | | | | | | | |

Each R⁴ according to the present invention is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a} independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy and p is 0, 1 or 2.

According to the invention, there can be zero, one, two, three, four or five R⁴ present, namely for m is 0, 1, 2, 3, 4 or 5. In particular, m is 0, 1, 2, 3 or 4.

According to one embodiment, m is 0.

According to a further embodiment, m is 1, 2, 3 or 4, in particular 1, 2 or 3, more specifically 1 or 2. According to one specific embodiment thereof, m is 1, according to a further specific embodiment, m is 2.

According to still a further embodiment, m is 2, 3 or 4.

According to still a further embodiment, m is 3.

According to one embodiment of the invention, one R⁴ is attached to the para-position (4-position).

According to a further embodiment of the invention, one R⁴ is attached to the meta-position (3-position).

According to a further embodiment of the invention, one R⁴ is attached to the ortho-position (2-position).

According to a further embodiment of the invention, two R⁴ are attached in 2,4-position.

According to a further embodiment of the invention, two R⁴ are attached in 2,3-position.

According to a further embodiment of the invention, two R⁴ are attached in 2,5-position.

According to a further embodiment of the invention, two R⁴ are attached in 2,6-position.

According to a further embodiment of the invention, two R⁴ are attached in 3,4-position.

According to a further embodiment of the invention, two R⁴ are attached in 3,5-position.

According to a further embodiment of the invention, three R⁴ are attached in 2,4,6-position.

For every R⁴ that is present in the inventive compounds, the following embodiments and preferences apply independently of the meaning of any other R⁴ that may be present in the phenyl ring. Furthermore, the particular embodiments and preferences given herein for R⁴ apply independently for each of m=1, m=2, m=3, m= 4 and m=5.

According to one embodiment, R⁴ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four independently selected R^{4a}, wherein R^{4a} is as defined and preferably defined herein.

According to a further embodiment, R⁴ is independently selected from halogen, CN, NO₂, C₁-C₄-alkyl, C₁-C₄-alkoxy, C₂-C₄-alkenyl, C₂-C₄-alkynyl, C₃-C₆-cycloalkyl, C₃-C₆-cycloalkyloxy, NH₂, NH(C₁-C₄₂-alkyl), N(C₁-C₂-alkyl)₂, S(O)ₚ(C₁-C₂-alkyl) (p=0, 1 or 2), C(=O)(C₁-C₂-alkyl), C(=O)(OH) and C(=O)(O-C₁-C₂-alkyl), wherein each of R⁴ is unsubstituted or further substituted by one, two, three or four independently selected R^{4a}, wherein R^{4a} is as defined and preferably defined herein.

According to a further embodiment, R⁴ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R⁴ is independently selected from halogen, CN, NO₂, C₁-C₂-alkyl, C₁-C₂-haloalkyl, C₁-C₂-alkoxy, C₁-C₂-haloalkoxy, S(C₁-C₂-alkyl), S(O)(C₁-C₂-alkyl), S(O)₂(C₁-C₂-alkyl), C(=O)(OH) and C(=O)(O-C₁-C₂-alkyl).

According to a further embodiment, R⁴ is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to still a further specific embodiment, R⁴ is independently selected from halogen, in particular from Br, F and Cl, more specifically from F and Cl.

According to a further specific embodiment, R⁴ is CN.

According to one further embodiment R⁴ is NO₂.

According to one further embodiment R⁴ is OH.

According to one further embodiment R⁴ is SH.

According to a further specific embodiment, R⁴ is C₁-C₆-alkyl, in particular C₁-C₄-alkyl, such as CH₃. Further appropriate alkyls are ethyl, n-propyl, i-propyl, n-butyl, i-butyl and t-butyl.

According to a further specific embodiment, R⁴ is C₁-C₆-haloalkyl, in particular C₁-C₄-haloalkyl, such as CF₃, CHF₂, CH₂F, CCl₃, CHCl₂ or CH₂Cl.

According to a further specific embodiment R⁴ is C₁-C₆-alkyl, preferably C₁-C₄-alkyl, substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R⁴ is CH₂OH. According to a further specific embodiment R⁴ is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH₂CH₂CH₂CN. In a special embodiment R⁴ is CH₂CH₂CN. In a further special embodiment R⁴ is CH(CH₃)CN. According to a further specific embodiment R⁴ is C₁-C₄-alkoxy-C₁-C₆-alkyl, more preferably C₁-C₄-alkoxy-C₁-C₄-alkyl. In a special embodiment R⁴ is CH₂OCH₃. In a further special embodiment R⁴ is CH₂CH₂OCH₃. In a further special embodiment R⁴ is CH(CH₃)OCH₃. In a further special embodiment R⁴ is CH(CH₃)OCH₂CH₃. In a further special embodiment R⁴ is CH₂CH₂OCH₂CH₃. According to a further specific embodiment R⁴ is C₁-C₄-haloalkoxy-C₁-C₆-alkyl, more preferably C₁-C₄-alkoxy-C₁-C₄-alkyl. In a special embodiment R⁴ is CH₂OCF₃. In a further special embodiment R⁴ is CH₂CH₂OCF₃. In a further special embodiment R⁴ is CH₂OCCl₃. In a further special embodiment R⁴ is CH₂CH₂OCCl₃.

According to a further specific embodiment, R⁴ is C₁-C₆-alkoxy, in particular C₁-C₄-alkoxy, more specifically C₁-C₂-alkoxy such as OCH₃ or OCH₂CH₃.

According to a further specific embodiment, R⁴ is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R⁴ is C₂-C₆-alkenyl or C₂-C₆-haloalkenyl, in particular C₂-C₄-alkenyl or C₂-C₄-haloalkenyl, such as CH=CH₂, CH₂CH=CH₂, CH=CHCH₃ or C(CH₃)=CH₂.

According to a further specific embodiment R⁴ is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R⁴ is CH=CHOH. In a further special embodiment R⁴ is CH=CHCH₂OH. According to a further specific embodiment R⁴ is C₁-C₄-alkoxy-C₂-C₆-alkenyl, more preferably C₁-C₄-alkoxy-C₂-C₄-alkenyl. In a special embodiment R⁴ is CH=CHOCH₃. In a further special embodiment R⁴ is CH=CHCH₂OCH₃. According to a further specific embodiment R⁴ is C₁-C₄-haloalkoxy-C₂-C₆-alkenyl, more preferably C₁-C₄-haloalkoxy-C₂-C₄-alkenyl. In a special embodiment R⁴ is CH=CHOCF₃. In a further special embodiment R⁴ is CH=CHCH₂OCF₃. In a further special embodiment R⁴ is CH=CHOCCl₃. In a further special embodiment R⁴ is CH=CHCH₂OCCl₃. According to a further specific embodiment R⁴ is C₃-C₈-cycloalkyl-C₂-C₆-alkenyl, preferably C₃-C₆-cycloalkyl-C₂-C₄-alkenyl. According to a further specific embodiment R⁴ is C₃-C₆-halocycloalkyl-C₂-C₄-alkenyl, preferably C₃-C₈-halocycloalkyl-C₂-C₆-alkenyl.

According to still a further embodiment, R⁴ is C₂-C₆-alkynyl or C₂-C₆-haloalkynyl, in particular C₂-C₄-alkynyl or C₂-C₄-haloalkynyl, such as CΞCH, CH₂CCH or CH₂CCCH₃.

According to a further specific embodiment R⁴ is C₂-C₆-alkynyl, preferably C₂-C₄-alkynyl, substituted by OH, more preferably, CCOH, CH₂CCOH. In a special embodiment R⁴ is CCOH. In a further special embodiment R⁴ is CH₂CCOH. According to a further specific embodiment R⁴ is C₁-C₄-alkoxy-C₂-C₆-alkynyl, more preferably C₁-C₄-alkoxy-C₂-C₄-alkynyl. In a special embodiment R⁴ is CCOCH₃. In a further special embodiment R⁴ is CH₂CCOCH₃. According to a further specific embodiment R⁴ is C₁-C₄-haloalkoxy-C₂-C₆-alkynyl, more preferably C₁-C₄-haloalkoxy-C₂-C₄-alkynyl. In a special embodiment R⁴ is CCOCF₃. In a further special embodiment R⁴ is CH₂CCOCF₃. In a further special embodiment R⁴ is CCOCCl₃. In a further special embodiment

R⁴ is CH₂CCOCCl₃. According to a further specific embodiment R⁴ is C₃-C₈-cycloalkyl-C₂-C₆-alkynyl, preferably C₃-C₆-cycloalkyl-C₂-C₄-alkynyl. According to a further specific embodiment R⁴ is C₃-C₆-halocycloalkyl-C₂-C₄-alkynyl, preferably C₃-C₈-halocycloalkyl-C₂-C₆-alkynyl.

According to one another embodiment R⁴ is C₃-C₈-cycloalkyl, preferably cyclopropyl, cyclobutyl, cyclopentyl or cyclohexyl, in particular cyclopropyl or cyclobutyl. In a special embodiment R⁴ is cyclopropyl. In a further special embodiment R⁴ is cyclobutyl. In a further special embodiment R⁴ is cyclopentyl. In a further special embodiment R⁴ is cyclohexyl.

According to one another embodiment R⁴ is C₃-C₈-cycloalkoxy, preferably C₃-C₆-cycloalkoxy. In a special embodiment R⁴ is O-cyclopropyl.

According to a specific embodiment R⁴ is C₃-C₈-halocycloalkyl, more preferably fully or partially halogenated C₃-C₆-cycloalkyl. In a special embodiment R⁴ is fully or partially halogenated cyclopropyl. In a further special embodiment R⁴ is 1-Cl-cyclopropyl. In a further special embodiment R⁴ is 2-Cl-cyclopropyl. In a further special embodiment R⁴ is 1-F-cyclopropyl. In a further special embodiment R⁴ is 2-F-cyclopropyl. In a further special embodiment R⁴ is fully or partially halogenated cyclobutyl. In a further special embodiment R⁴ is 1-Cl-cyclobutyl. In a further special embodiment R⁴ is 1-F-cyclobutyl. In a further special embodiment R⁴ is 3,3-Cl₂-cyclobutyl. In a further special embodiment R⁴ is 3,3-F₂-cyclobutyl. According to a specific embodiment R⁴ is C₃-C₈-cycloalkyl substituted by C₁-C₄-alkyl, more preferably is C₃-C₆-cycloalkyl substituted by C₁-C₄-alkyl. In a special embodiment R⁴ is 1-CH₃-cyclopropyl. According to a specific embodiment R⁴ is C₃-C₈-cycloalkyl substituted by CN, more preferably is C₃-C₆-cycloalkyl substituted by CN. In a special embodiment R⁴ is 1-CN-cyclopropyl.According to a further specific embodiment R⁴ is C₃-C₈-cycloalkyl-C₃-C₈-cycloalkyl, preferably C₃-C₆-cycloalkyl-C₃-C₆-cycloalkyl. In a special embodiment R⁴ is cyclopropyl-cyclopropyl. In a special embodiment R⁴ is 2-cyclopropyl-cyclopropyl. According to a further specific embodiment R⁴ is C₃-C₈-cycloalkyl-C₃-C₈-halocycloalkyl, preferably C₃-C₆-cycloalkyl-C₃-C₆-halocycloalkyl.

According to one another embodiment R⁴ is C₃-C₈-cycloalkyl-C₁-C₄-alkyl, preferably C₃-C₆-cycloalkyl-C₁-C₄-alkyl. In a special embodiment R⁴ is CH(CH₃)(cyclopropyl). In a further special embodiment R⁴ is CH₂-(cyclopropyl).

According to a further preferred embodiment R⁴ is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R⁴ is C₃-C₈-cycloalkyl-C₁-C₄-haloalkyl, C₃-C₆-cycloalkyl-C₁-C₄-haloalkyl. According to a specific embodiment R⁴ is C₃-C₈-halocycloalkyl-C₁-C₄-alkyl, C₃-C₆-halocycloalkyl-C₁-C₄-alkyl. In a special embodiment R⁴ is fully or partially halogenated cyclopropyl-C₁-C₄-alkyl. In a further special embodiment R⁴ is 1-Cl-cyclopropyl-C₁-C₄-alkyl. In a further special embodiment R⁴ is 1-F-cyclopropyl-C₁-C₄-alkyl.

According to one another embodiment R⁴ is NH₂.

According to one another embodiment R⁴ is NH(C₁-C₄-alkyl). According to a specific embodiment R⁴ is NH(CH₃). According to a specific embodiment R⁴ is NH(CH₂CH₃). According to a specific embodiment R⁴ is NH(CH₂CH₂CH₃). According to a specific embodiment R⁴ is NH(CH(CH₃)₂). According to a specific embodiment R⁴ is NH(CH₂CH₂CH₂CH₃). According to a specific embodiment R⁴ is NH(C(CH₃)₃).

According to one another embodiment R⁴ is N(C₁-C₄-alkyl)₂. According to a specific embodiment R⁴ is N(CH₃)₂. According to a specific embodiment R⁴ is N(CH₂CH₃)₂. According to a specific embodiment R⁴ is N(CH₂CH₂CH₃)₂. According to a specific embodiment R⁴ is N(CH(CH₃)₂)₂. According to a specific embodiment R⁴ is N(CH₂CH₂CH₂CH₃)₂. According to a specific embodiment R⁴ is NH(C(CH₃)₃)₂.

According to one another embodiment R⁴ is NH(C₃-C₈-cycloalkyl) preferably NH(C₃-C₆-cycloalkyl). According to a specific embodiment R⁴ is NH(cyclopropyl). According to a specific embodiment R⁴ is NH(cyclobutyl). According to a specific embodiment R⁴ is NH(cyclopentyl). According to a specific embodiment R⁴ is NH(cyclohexyl).

According to one another embodiment R⁴ is N(C₃-C₈-cycloalkyl)₂ preferably N(C₃-C₆-cycloalkyl)₂. According to a specific embodiment R⁴ is N(cyclopropyl)₂. According to a specific embodiment R⁴ is N(cyclobutyl)₂. According to a specific embodiment R⁴ is N(cyclopentyl)₂. According to a specific embodiment R⁴ is N(cyclohexyl)₂.

According to still a further embodiment, R⁴ is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R⁴ is C(=O)(OH) or C(=O)(O-C₁-C₄-alkyl), in particular C(=O)(OCH₃).

According to one another embodiment R⁴ is C(=O)(-C₁-C₄-alkyl). According to a specific embodiment R⁴ is C(=O)CH₃.. According to a further specific embodiment R⁴ is C(=O)CH₂CH₃. According to a further specific embodiment R⁴ is C(=O)CH₂CH₂CH₃. According to a further specific embodiment R⁴ is C(=O)CH(CH₃)₂. According to a further specific embodiment R⁴ is C(=O)C(CH₃)₃.

According to one another embodiment R⁴ is C(=O)OH.

According to one another embodiment R⁴ is C(=O)(-O-C₁-C₄-alkyl). According to a specific embodiment R⁴ is C(=O)OCH₃.. According to a further specific embodiment R⁴ is C(=O)OCH₂CH₃. According to a further specific embodiment R⁴ is C(=O)OCH₂CH₂CH₃. According to a further specific embodiment R⁴ is C(=O)OCH(CH₃)₂. According to a further specific embodiment R⁴ is C(=O)OC(CH₃)₃.

According to one another embodiment R⁴ is C(=O)-NH(C₁-C₄-alkyl). According to a specific embodiment R⁴ is C(=O)NHCH₃.. According to a further specific embodiment R⁴ is C(=O)NHCH₂CH₃. According to a further specific embodiment R⁴ is C(=O)NHCH₂CH₂CH₃. According to a further specific embodiment R⁴ is C(=O)NHCH(CH₃)₂. According to a further specific embodiment R⁴ is C(=O)NHC(CH₃)₃.

According to one another embodiment R⁴ is C(=O)-N(C₁-C₄-alkyl)₂. According to a specific embodiment R⁴ is C(=O)N(CH₃)₂. According to a further specific embodiment R⁴ is C(=O)N(CH₂CH₃)₂. According to a further specific embodiment R⁴ is C(=O)N(CH₂CH₂CH₃)₂.According to a further specific embodiment R⁴ is C(=O)N(CH(CH₃)₂)₂. According to a further specific embodiment R⁴ is C(=O)N(C(CH₃)₃)₂.

According to one another embodiment R⁴ is C(=O)-NH(C₃-C₆-cycloalkyl). According to a specific embodiment R⁴ is C(=O)NH(cyclopropyl).. According to a further specific embodiment R⁴ is C(=O)NH(cyclobutyl).According to a further specific embodiment R⁴ is C(=O)NH(cyclopentyl).Ac-cording to a further specific embodiment R⁴ is C(=O)NH(cyclohexyl).

According to one another embodiment R⁴ is C(=O)-N(C₃-C₆-cycloalkyl)₂. According to a specific embodiment R⁴ is C(=O)N(cyclopropyl)₂. According to a further specific embodiment R⁴ is C(=O)N(cyclobutyl)₂. According to a further specific embodiment R⁴ is C(=O)N(cyclopentyl)₂. According to a further specific embodiment R⁴ is C(=O)N(cyclohexyl)₂.

According to still a further embodiment, R⁴ is selected from S(C₁-C₂-alkyl), S(O)(C₁-C₂-alkyl) and S(O)₂(C₁-C₂-alkyl), in particular SCH₃, S(O)(CH₃) and S(O)₂(CH₃). According to a specific embodiment R⁴ is selected from S(C₁-C₂-haloalkyl), S(O)(C₁-C₂-haloalkyl) and S(O)₂(C₁-C₂-haloalkyl), such as SO₂CF₃.

Particularly preferred embodiments of R⁴ according to the invention are in Table P4 below, wherein each line of lines P4-1 to P4-16 corresponds to one particular embodiment of the invention, wherein P4-1 to P4-16 are also in any combination with one another preferred embodiments of the present invention. Thereby, for every R⁴ that is present in the inventive compounds, these specific embodiments and preferences apply independently of the meaning of any other R⁴ that may be present in the phenyl ring:

**Table P4:**

| **No.** | **R⁴** | | **No.** | **R⁴** | | **No.** | **R⁴** |
|---|---|---|---|---|---|---|---|
| P4-1 | Cl | | P4-7 | CF₃ | | P4-13 | SCH₃ |
| P4-2 | F | | P4-8 | CHF₂ | | P4-14 | SOCH₃ |
| P4-3 | CN | | P4-9 | OCH₃ | | P4-15 | SO₂CH₃ |
| P4-4 | NO₂ | | P4-10 | OCH₂CH₃ | | P4-16 | CO₂CH₃ |
| P4-5 | CH₃ | | P4-11 | OCF₃ | | | |
| P4-6 | CH₂CH₃ | | P4-12 | OCHF₂ | | | |

Particularly preferred embodiments of (R⁴)ₘ according to the invention are in Table P41 below, wherein each line of lines P41-1 to P41-156 corresponds to one particular embodiment of the invention, wherein P41-1 to P41-156 are also in any combination preferred embodiments of the present invention. The positions of the R⁴ are given in relation to the position of the attachment of the phenoxy-moiety (= in 1-position).

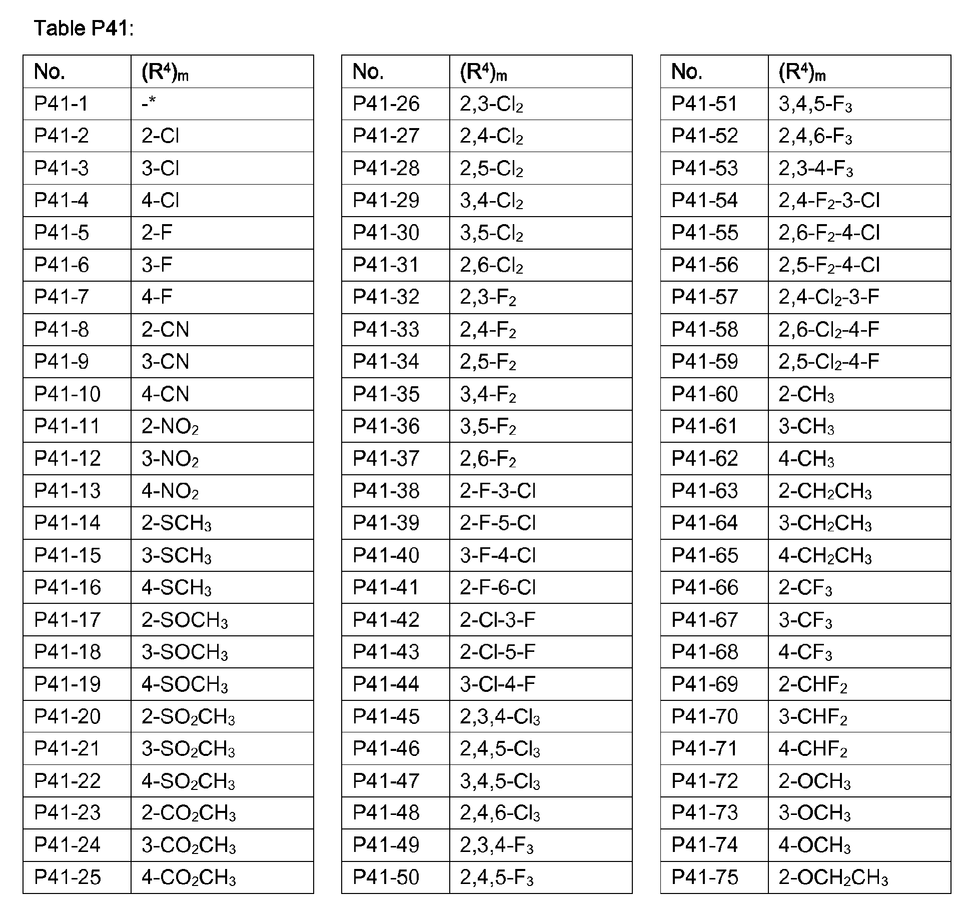

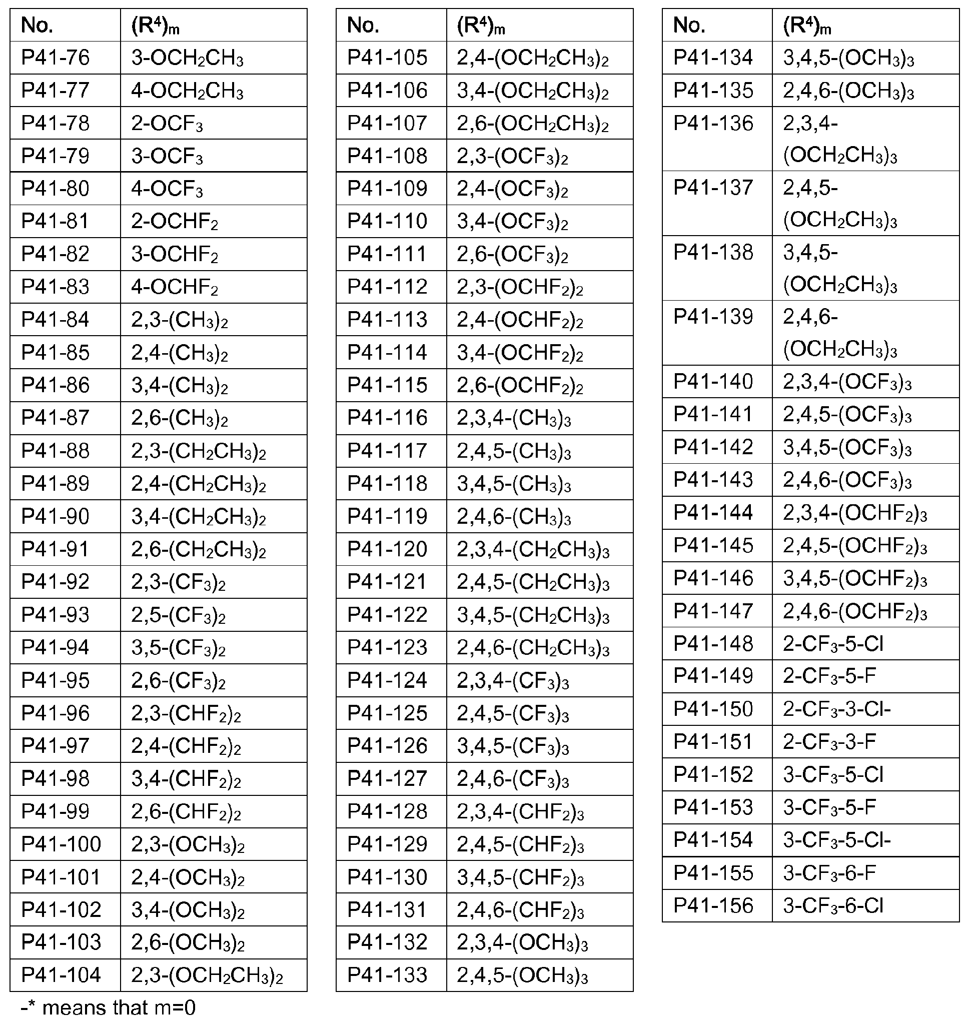

More particularly preferred embodiments of (R⁴)ₘ according to the invention are in Table P42 below, wherein each line of lines P42-1 to P42-64 corresponds to one particular embodiment of the invention, wherein P42-1 to P42-64 are also in any combination preferred embodiments of the present invention. The positions of the R⁴ are given in relation to the position of the attachment of the phenoxy-moiety (= in 1-position).

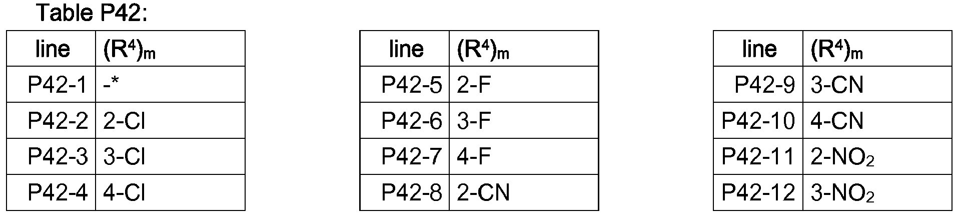

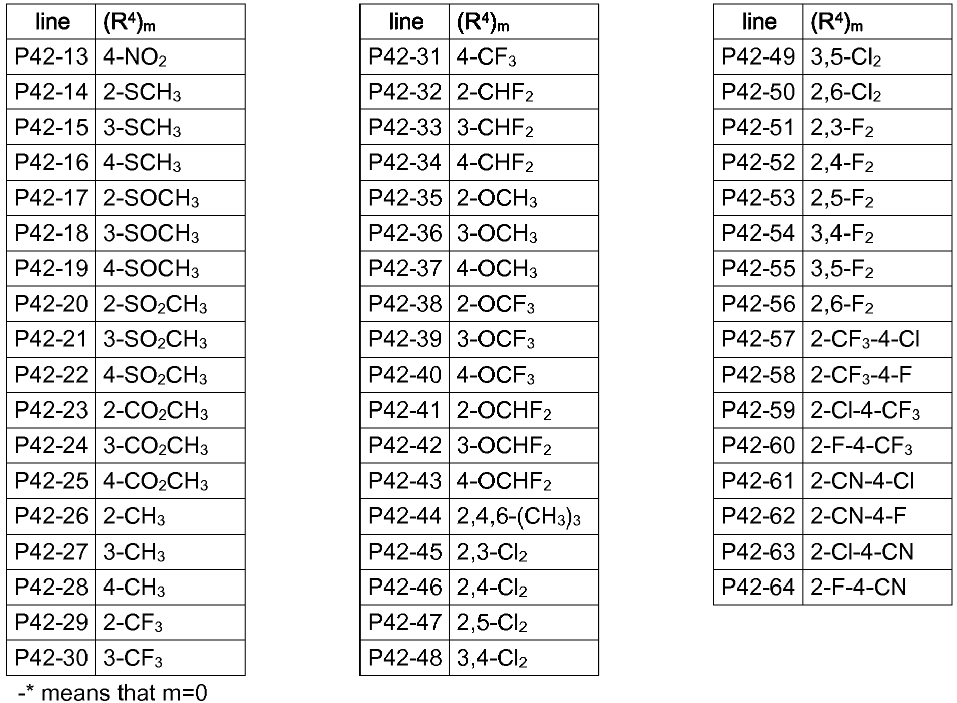

One particular embodiment relates to compounds I, wherein n+m is >= 1, namely, 1, 2, 3, 4, 5, 6, 7, 8 or 9, more specifically 1, 2, 3, 4 or 5. In specific embodiment thereof, n+m is 1, 2 or 3.

According to one embodiment, X is N and Y is N, corresponding to compounds I.A:

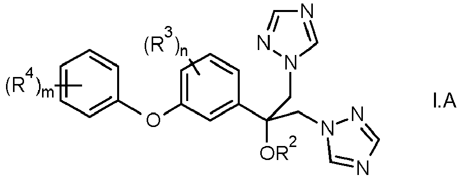

According to a further embodiment, X is N and Y is CH, corresponding to compounds I.B:

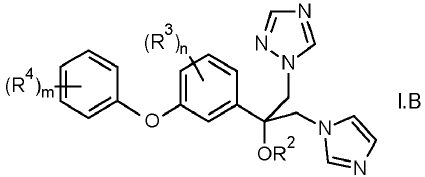

According to still a further embodiment, X is CH and Y is CH, corresponding to compounds I.C:

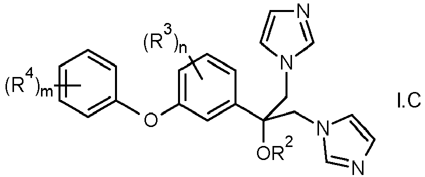

In particular with a view to their use, preference is given to the compounds of the formula I, in particular I.A, I.B and I.C, compiled in the Tables 1a to 13a, Tables 1b to 13b and Tables 1c to 13c below. Each of the groups mentioned for a substituent in the tables is furthermore per se, independently of the combination in which it is mentioned, a particularly preferred aspect of the substituent in question.
Table 1 a Compounds of the formula I.A in which R² corresponds to line B-1 of Table B and the meaning for the combination of (R³)ₙ and (R⁴)ₘ for each individual compound corresponds in each case to one line of Table A (compounds I.A.B-1.A1 to I.A.B-1.A2112).
Table 2a Compounds of the formula I.A in which R² corresponds to line B-2 of Table B and the meaning for the combination of (R³)ₙ and (R⁴)ₘ for each individual compound corresponds in each case to one line of Table A (compounds I.A.B-2.A1 to I.A.B-2.A2112).
Table 3a Compounds of the formula I.A in which R² corresponds to line B-3 of Table B and the meaning for the combination of (R³)ₙ and (R⁴)ₘ for each individual compound corresponds in each case to one line of Table A (compounds I.A.B-3.A1 to I.A.B-3.A2112).
Table 4a Compounds of the formula I.A in which R² corresponds to line B-4 of Table B and the meaning for the combination of (R³)ₙ and (R⁴)ₘ for each individual compound corresponds in each case to one line of Table A (compounds I.A.B-4.A1 to I.A.B-4.A2112).
Table 5a Compounds of the formula I.A in which R² corresponds to line B-5 of Table B and the meaning for the combination of (R³)ₙ and (R⁴)ₘ for each individual compound corresponds in each case to one line of Table A (compounds I.A.B-5.A1 to I.A.B-5.A2112).
Table 6a Compounds of the formula I.A in which R² corresponds to line B-6 of Table B and the meaning for the combination of (R³)ₙ and (R⁴)ₘ for each individual compound corresponds in each case to one line of Table A (compounds I.A.B-6.A1 to I.A.B-6.A2112).
Table 7a Compounds of the formula I.A in which R² corresponds to line B-7 of Table B and the meaning for the combination of (R³)ₙ and (R⁴)ₘ for each individual compound corresponds in each case to one line of Table A (compounds I.A.B-7.A1 to I.A.B-7.A2112).
Table 8a Compounds of the formula I.A in which R² corresponds to line B-8 of Table B and the meaning for the combination of (R³)ₙ and (R⁴)ₘ for each individual compound corresponds in each case to one line of Table A (compounds I.A.B-8.A1 to I.A.B-8.A2112).
Table 9a Compounds of the formula I.A in which R² corresponds to line B-9 of Table B and the meaning for the combination of (R³)ₙ and (R⁴)ₘ for each individual compound corresponds in each case to one line of Table A (compounds I.A.B-9.A1 to I.A.B-9.A2112).
Table 10a Compounds of the formula I.A in which R² corresponds to line B-10 of Table B and the meaning for the combination of (R³)ₙ and (R⁴)ₘ for each individual compound corresponds in each case to one line of Table A (compounds I.A.B-10.A1 to I.A.B-10.A2112).
Table 11 a Compounds of the formula I.A in which R² corresponds to line B-11 of Table B and the meaning for the combination of (R³)ₙ and (R⁴)ₘ for each individual compound corresponds in each case to one line of Table A (compounds I.A.B-11.A1 to I.A.B-11.A2112).
Table 12a Compounds of the formula I.A in which R² corresponds to line B-12 of Table B and the meaning for the combination of (R³)ₙ and (R⁴)ₘ for each individual compound corresponds in each case to one line of Table A (compounds I.A.B-12.A1 to I.A.B-12.A2112).
Table 13a Compounds of the formula I.A in which R² corresponds to line B-13 of Table B and the meaning for the combination of (R³)ₙ and (R⁴)ₘ for each individual compound corresponds in each case to one line of Table A (compounds I.A.B-13.A1 to I.A.B-13.A2112).
Table 1 b Compounds of the formula I.B in which R² corresponds to line B-1 of Table B and the meaning for the combination of (R³)ₙ and (R⁴)ₘ for each individual compound corresponds in each case to one line of Table A (compounds I.B.B-1.A1 to I.B.B-1.A2112).
Table 2b Compounds of the formula I.B in which R² corresponds to line B-2 of Table B and the meaning for the combination of (R³)ₙ and (R⁴)ₘ for each individual compound corresponds in each case to one line of Table A (compounds I.B.B-2.A1 to I.B.B-2.A2112).
Table 3b Compounds of the formula I.B in which R² corresponds to line B-3 of Table B and the meaning for the combination of (R³)ₙ and (R⁴)ₘ for each individual compound corresponds in each case to one line of Table A (compounds I.B.B-3.A1 to I.B.B-3.A2112).
Table 4b Compounds of the formula I.B in which R² corresponds to line B-4 of Table B and the meaning for the combination of (R³)ₙ and (R⁴)ₘ for each individual compound corresponds in each case to one line of Table A (compounds I.B.B-4.A1 to I.B.B-4.A2112).
Table 5b Compounds of the formula I.B in which R² corresponds to line B-5 of Table B and the meaning for the combination of (R³)ₙ and (R⁴)ₘ for each individual compound corresponds in each case to one line of Table A (compounds I.B.B-5.A1 to I.B.B-5.A2112).
Table 6b Compounds of the formula I.B in which R² corresponds to line B-6 of Table B and the meaning for the combination of (R³)ₙ and (R⁴)ₘ for each individual compound corresponds in each case to one line of Table A (compounds I.B.B-6.A1 to I.B.B-6.A2112).
Table 7b Compounds of the formula I.B in which R² corresponds to line B-7 of Table B and the meaning for the combination of (R³)ₙ and (R⁴)ₘ for each individual compound corresponds in each case to one line of Table A (compounds I.B.B-7.A1 to I.B.B-7.A2112).
Table 8b Compounds of the formula I.B in which R² corresponds to line B-8 of Table B and the meaning for the combination of (R³)ₙ and (R⁴)ₘ for each individual compound corresponds in each case to one line of Table A (compounds I.B.B-8.A1 to I.B.B-8.A2112).
Table 9b Compounds of the formula I.B in which R² corresponds to line B-9 of Table B and the meaning for the combination of (R³)ₙ and (R⁴)ₘ for each individual compound corresponds in each case to one line of Table A (compounds I.B.B-9.A1 to I.B.B-9.A2112).
Table 10b Compounds of the formula I.B in which R² corresponds to line B-10 of Table B and the meaning for the combination of (R³)ₙ and (R⁴)ₘ for each individual compound corresponds in each case to one line of Table A (compounds I.B.B-10.A1 to I.B.B-10.A2112).
Table 11 b Compounds of the formula I.B in which R² corresponds to line B-11 of Table B and the meaning for the combination of (R³)ₙ and (R⁴)ₘ for each individual compound corresponds in each case to one line of Table A (compounds I.B.B-11.A1 to I.B.B-11.A2112).
Table 12b Compounds of the formula I.B in which R² corresponds to line B-12 of Table B and the meaning for the combination of (R³)ₙ and (R⁴)ₘ for each individual compound corresponds in each case to one line of Table A (compounds I.B.B-12.A1 to I.B.B-12.A2112).
Table 13b Compounds of the formula I.B in which R² corresponds to line B-13 of Table B and the meaning for the combination of (R³)ₙ and (R⁴)ₘ for each individual compound corresponds in each case to one line of Table A (compounds I.B.B-13.A1 to I.B.B-13.A2112).
Table 1c Compounds of the formula I.C in which R² corresponds to line B-1 of Table B and the meaning for the combination of (R³)ₙ and (R⁴)ₘ for each individual compound corresponds in each case to one line of Table A (compounds I.C.B-1.A1 to I.C.B-1.A2112).
Table 2c Compounds of the formula I.C in which R² corresponds to line B-2 of Table B and the meaning for the combination of (R³)ₙ and (R⁴)ₘ for each individual compound corresponds in each case to one line of Table A (compounds I.C.B-2.A1 to I.C.B-2.A2112).
Table 3c Compounds of the formula I.C in which R² corresponds to line B-3 of Table B and the meaning for the combination of (R³)ₙ and (R⁴)ₘ for each individual compound corresponds in each case to one line of Table A (compounds I.C.B-3.A1 to I.C.B-3.A2112).
Table 4c Compounds of the formula I.C in which R² corresponds to line B-4 of Table B and the meaning for the combination of (R³)ₙ and (R⁴)ₘ for each individual compound corresponds in each case to one line of Table A (compounds I.C.B-4.A1 to I.C.B-4.A2112).
Table 5c Compounds of the formula I.C in which R² corresponds to line B-5 of Table B and the meaning for the combination of (R³)ₙ and (R⁴)ₘ for each individual compound corresponds in each case to one line of Table A (compounds I.C.B-5.A1 to I.C.B-5.A2112).
Table 6c Compounds of the formula I.C in which R² corresponds to line B-6 of Table B and the meaning for the combination of (R³)ₙ and (R⁴)ₘ for each individual compound corresponds in each case to one line of Table A (compounds I.C.B-6.A1 to I.C.B-6.A2112).
Table 7c Compounds of the formula I.C in which R² corresponds to line B-7 of Table B and the meaning for the combination of (R³)ₙ and (R⁴)ₘ for each individual compound corresponds in each case to one line of Table A (compounds I.C.B-7.A1 to I.C.B-7.A2112).
Table 8c Compounds of the formula I.C in which R² corresponds to line B-8 of Table B and the meaning for the combination of (R³)ₙ and (R⁴)ₘ for each individual compound corresponds in each case to one line of Table A (compounds I.C.B-8.A1 to I.C.B-8.A2112).
Table 9c Compounds of the formula I.C in which R² corresponds to line B-9 of Table B and the meaning for the combination of (R³)ₙ and (R⁴)ₘ for each individual compound corresponds in each case to one line of Table A (compounds I.C.B-9.A1 to I.C.B-9.A2112).
Table 10c Compounds of the formula I.C in which R² corresponds to line B-10 of Table B and the meaning for the combination of (R³)ₙ and (R⁴)ₘ for each individual compound corresponds in each case to one line of Table A (compounds I.C.B-10.A1 to I.C.B-10.A2112).
Table 11c Compounds of the formula I.C in which R² corresponds to line B-11 of Table B and the meaning for the combination of (R³)ₙ and (R⁴)ₘ for each individual compound corresponds in each case to one line of Table A (compounds I.C.B-11.A1 to I.C.B-11.A2112).
Table 12c Compounds of the formula I.C in which R² corresponds to line B-12 of Table B and the meaning for the combination of (R³)ₙ and (R⁴)ₘ for each individual compound corresponds in each case to one line of Table A (compounds I.C.B-12.A1 to I.C.B-12.A2112).
Table 13c Compounds of the formula I.C in which R² corresponds to line B-13 of Table B and the meaning for the combination of (R³)ₙ and (R⁴)ₘ for each individual compound corresponds in each case to one line of Table A (compounds I.C.B-13.A1 to I.C.B-13.A2112).

**Table B**

| **line** | **R²** | | **line** | **R²** |
|---|---|---|---|---|
| B-1 | CH₃ | | B-8 | CH₂CN, |
| B-2 | CH₂CH₃ | | B-9 | CH₂CH₂CN, |
| B-3 | CH(CH₃)₂ | | B-10 | CH₂CH=CH₂ |
| B-4 | CH₂CH₂CH₃ | | B-11 | CH₂CH=CHCH₃ |
| B-5 | CH₂OCH₃ | | B-12 | CH₂C≡CH |
| B-6 | CH₂CH₂OCH₃ | | B-13 | CH₂C≡CCH₃ |
| B-7 | CH₂CH₂OH | | | |

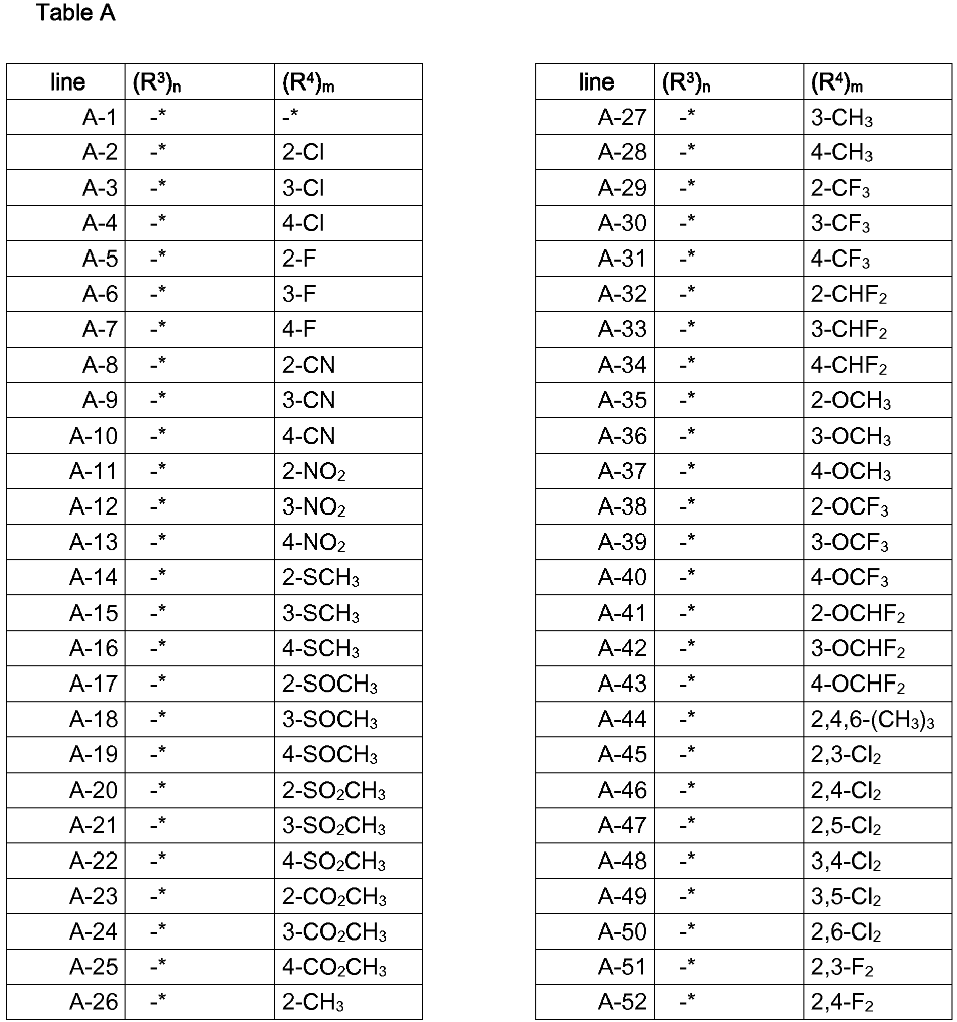

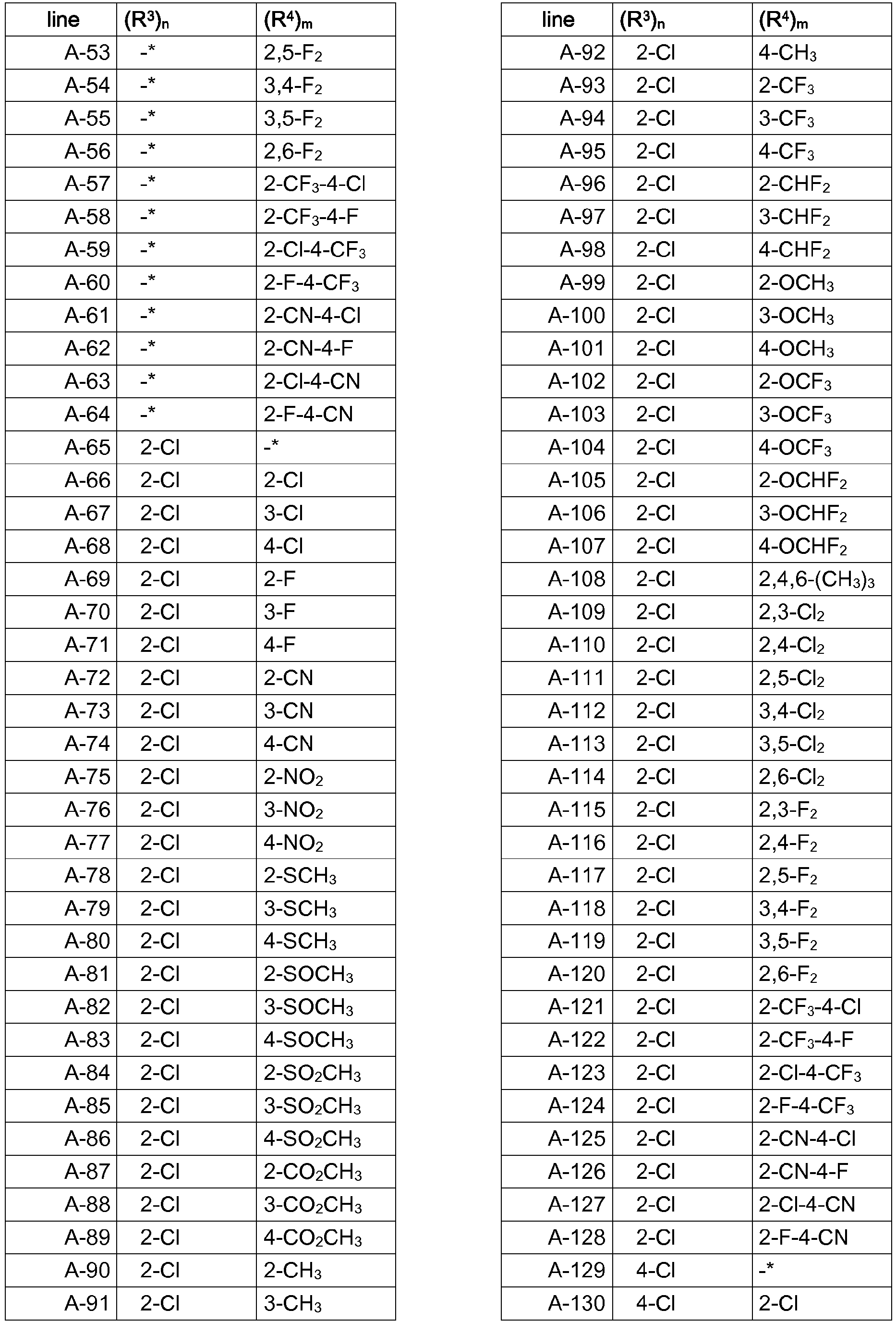

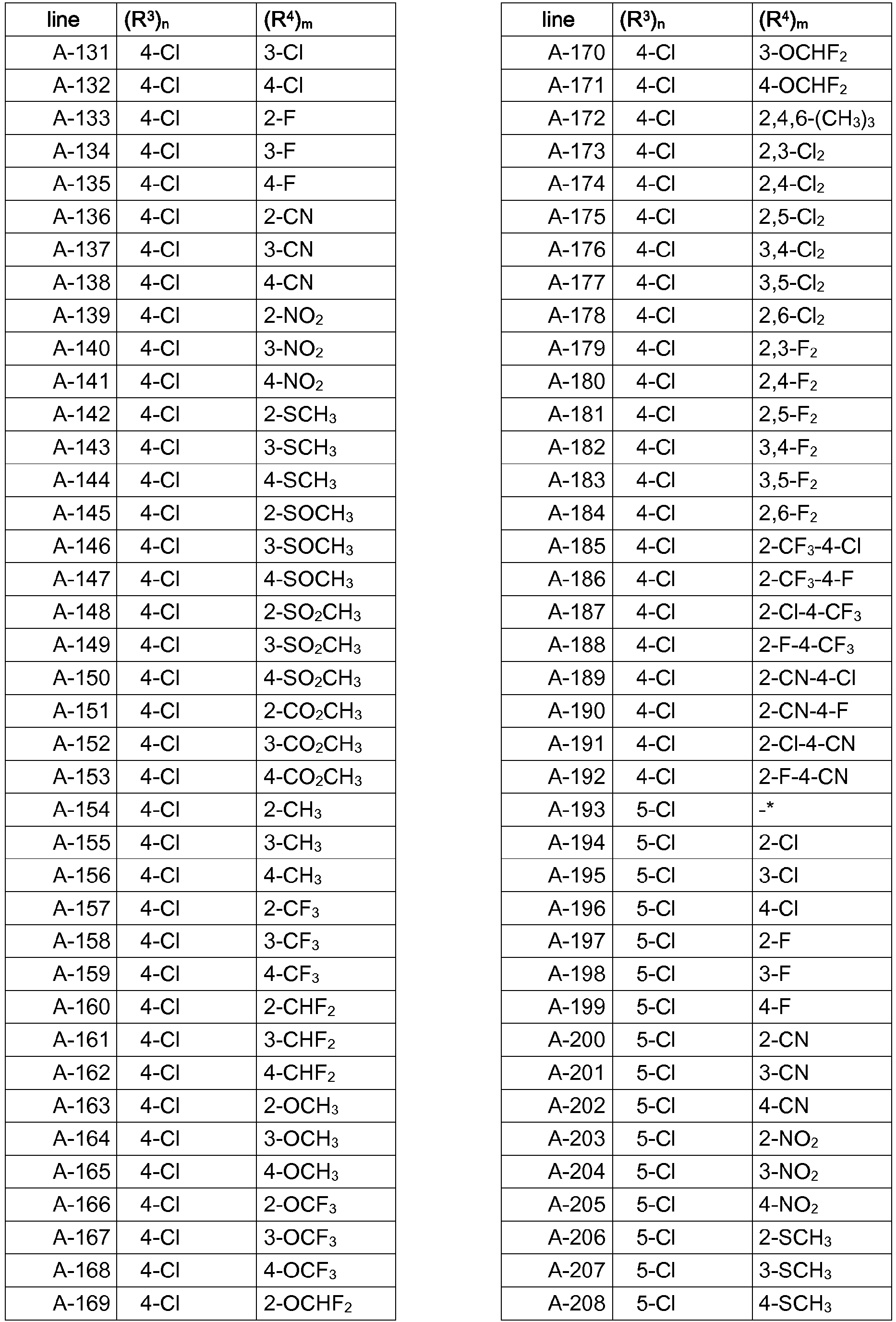

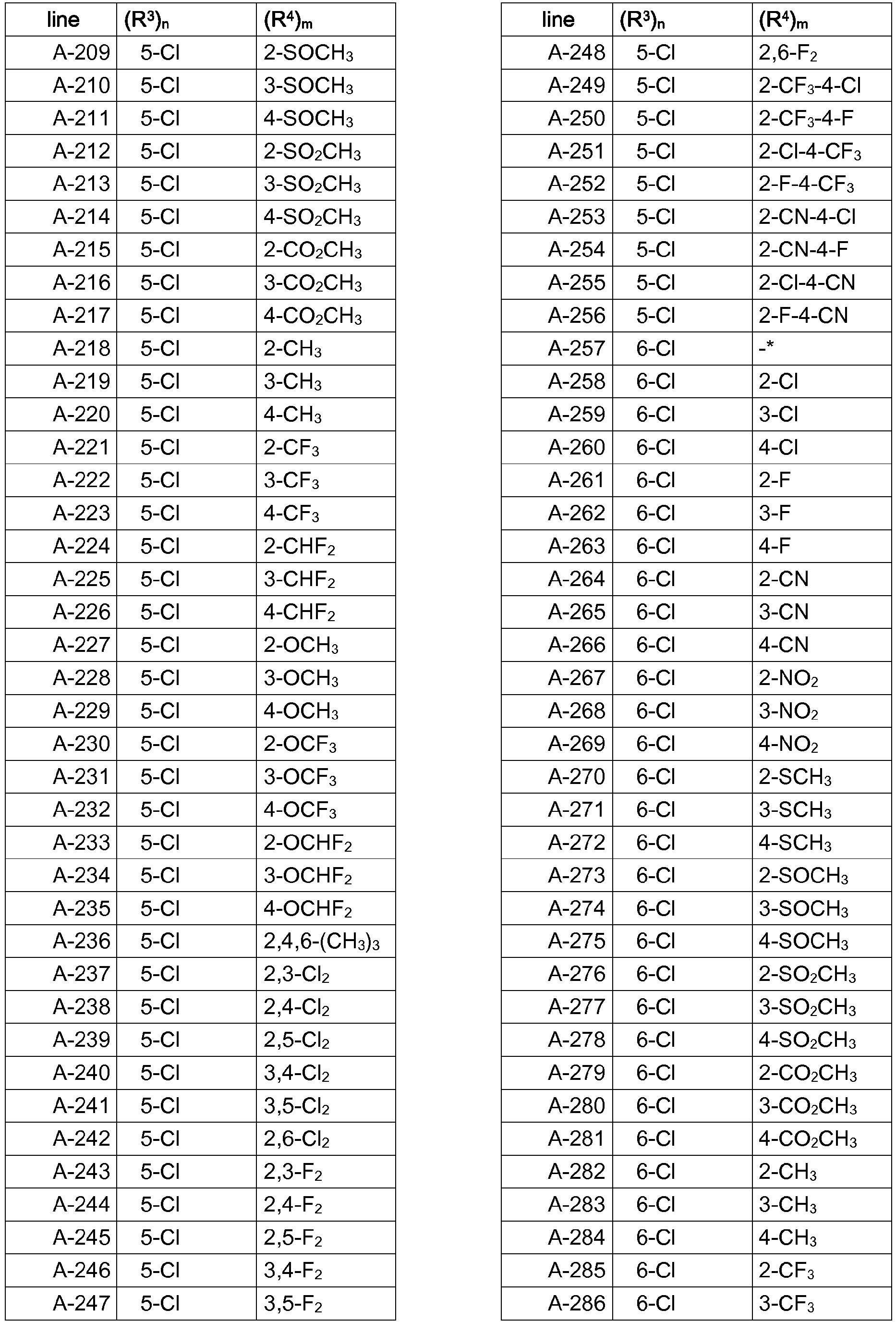

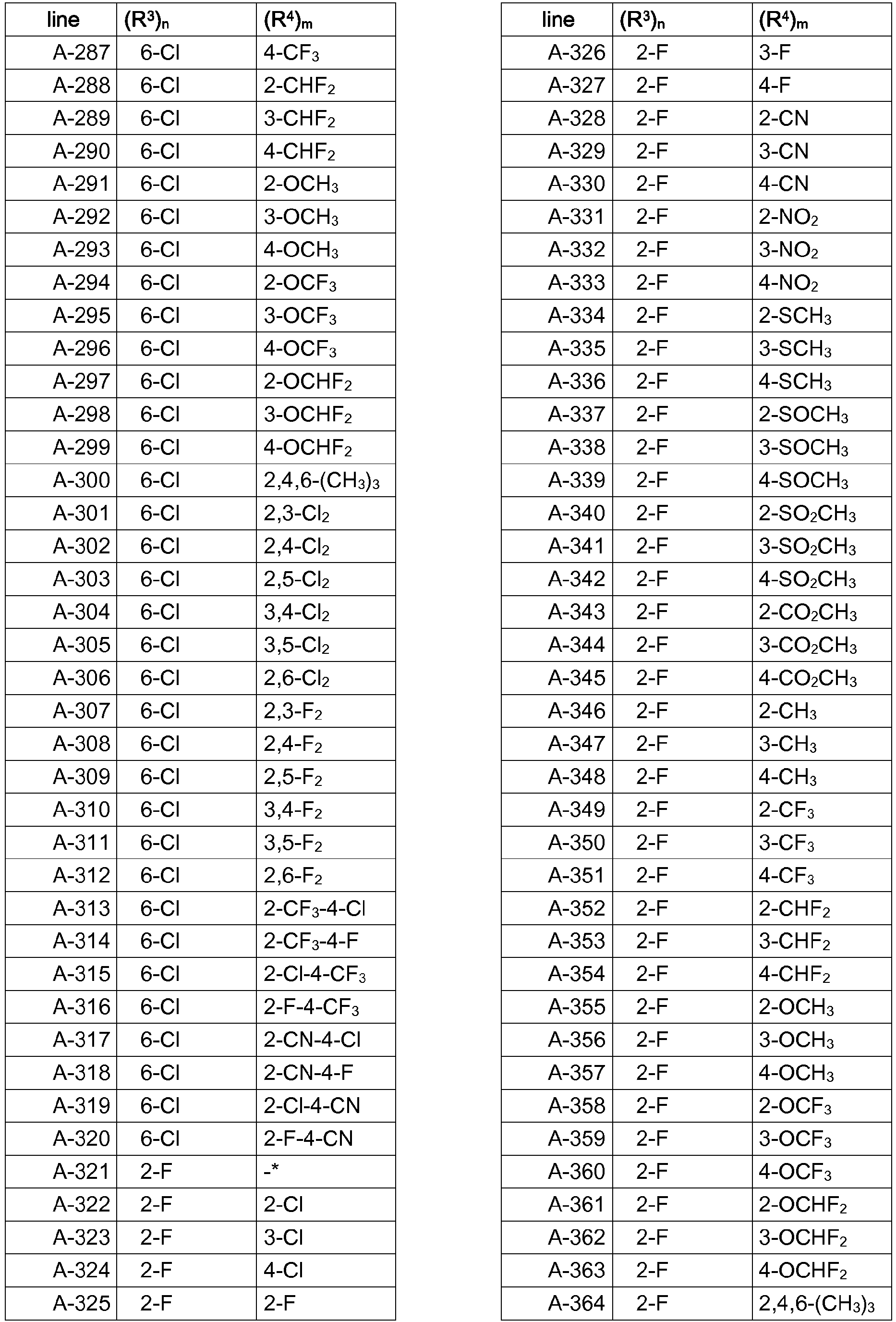

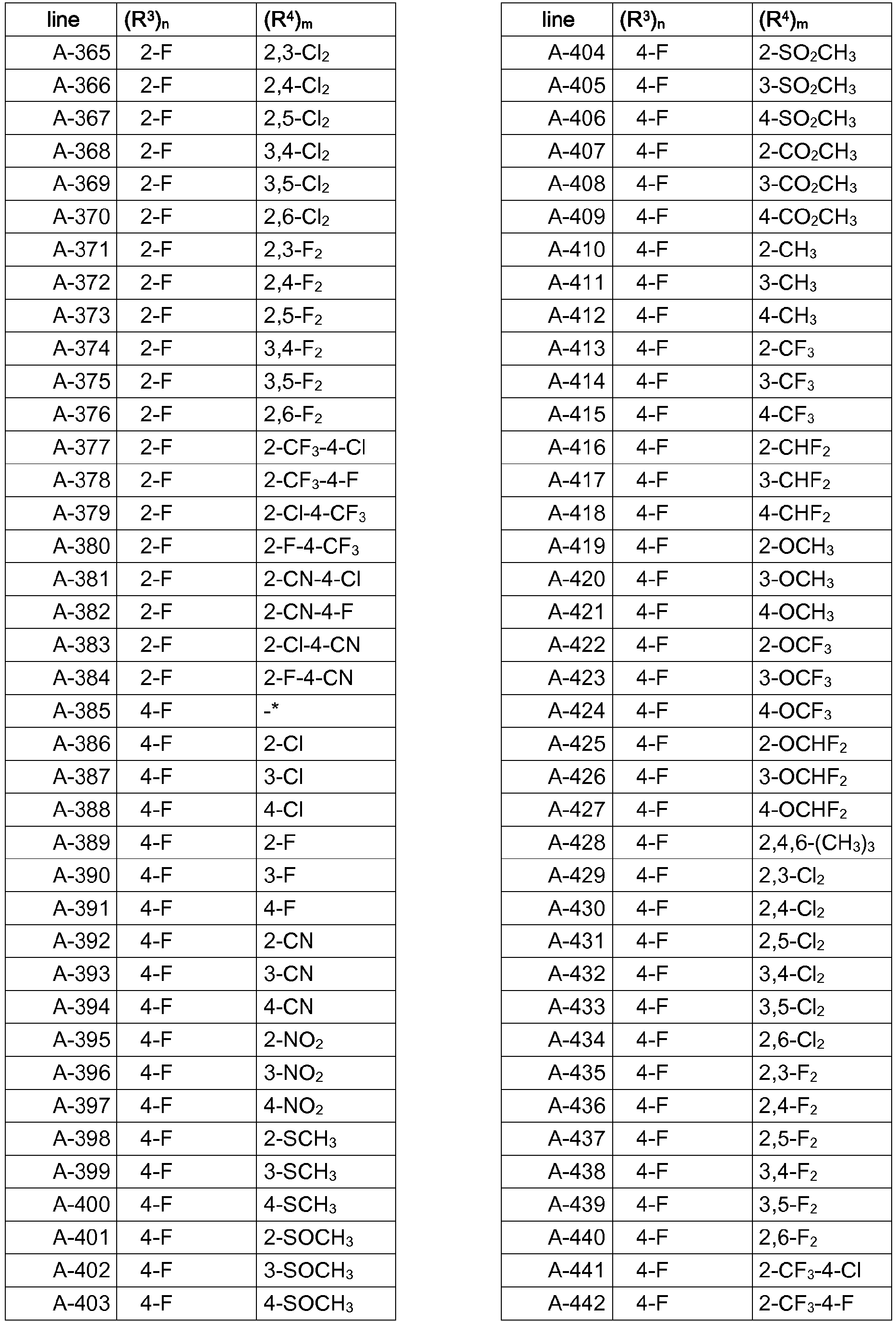

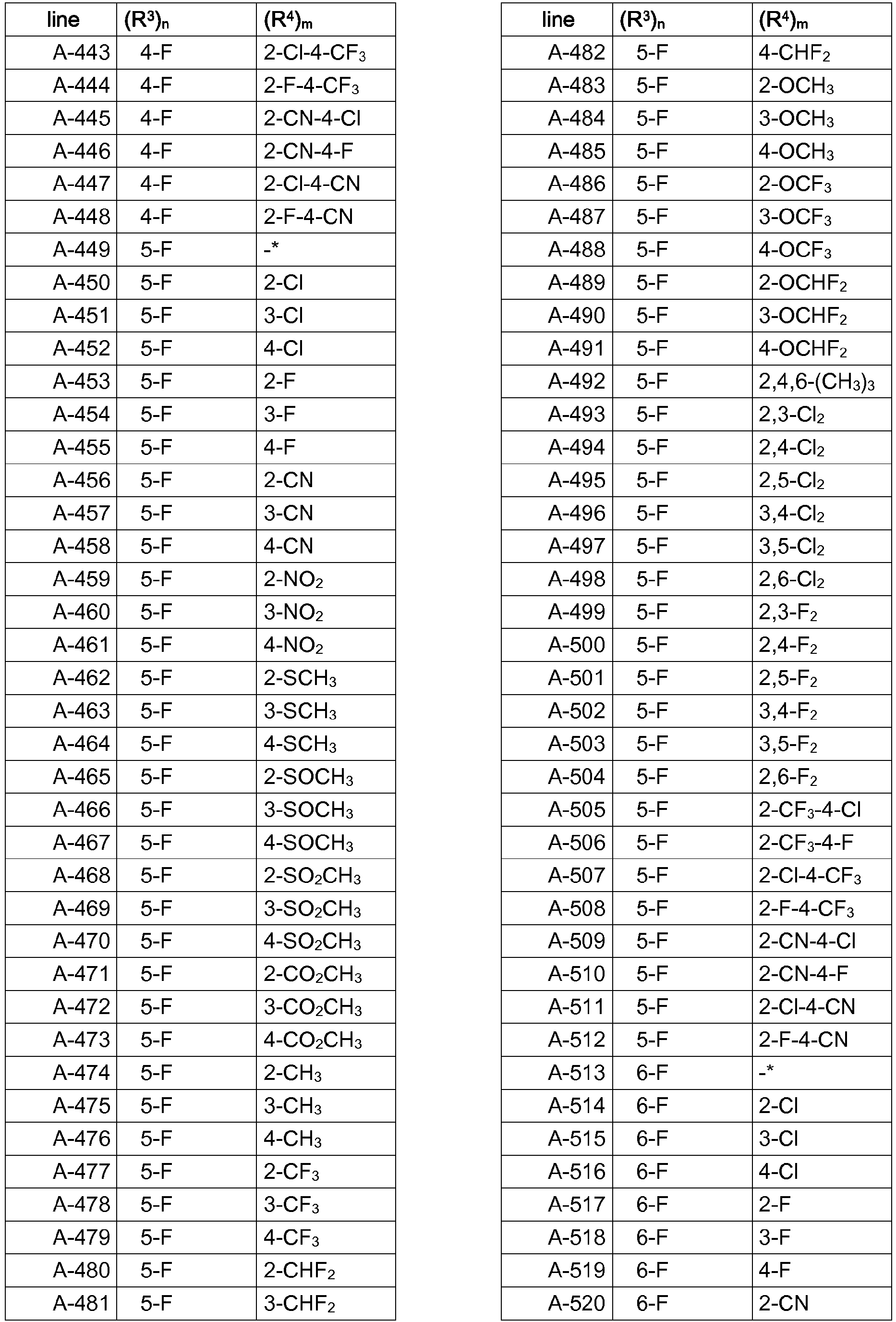

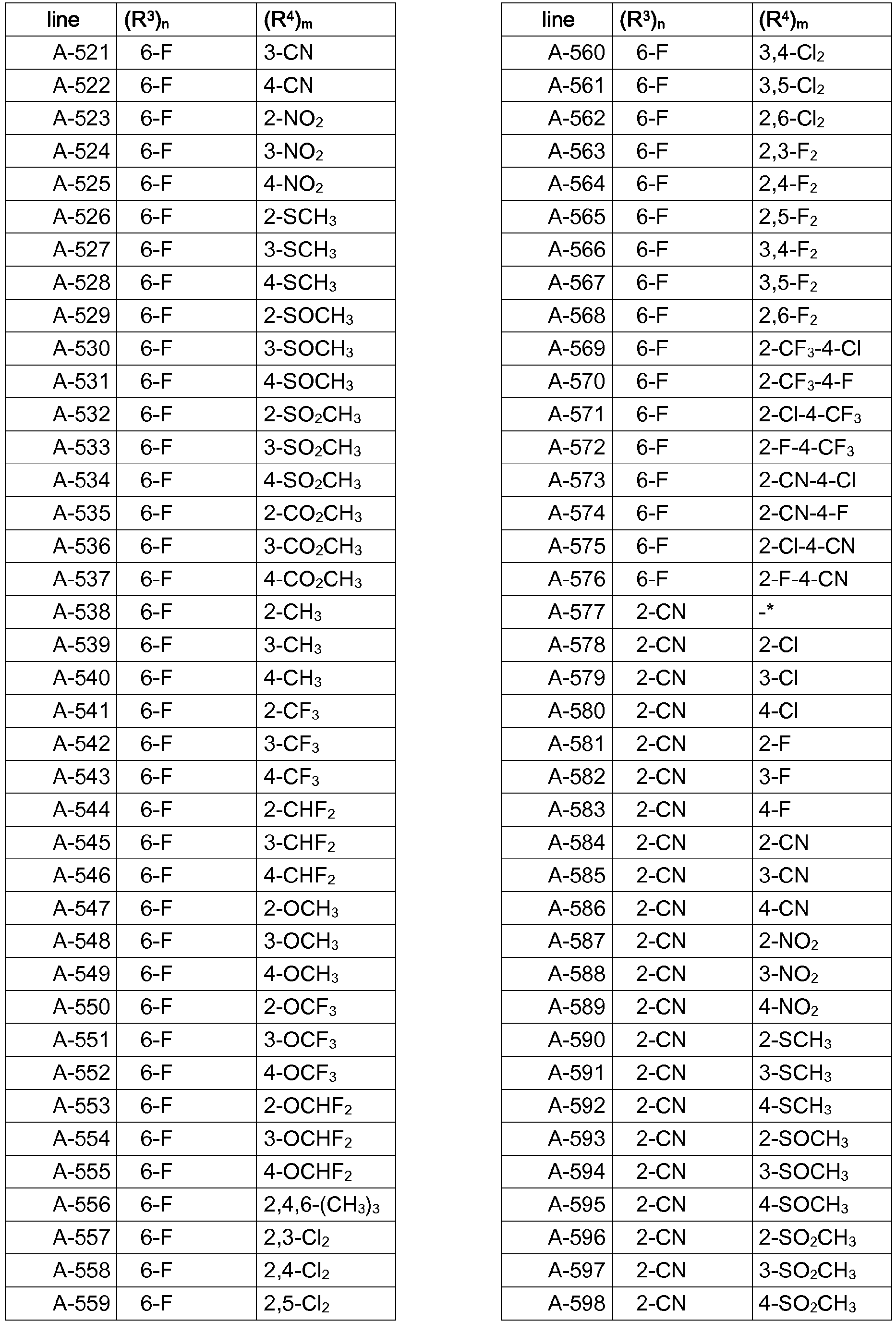

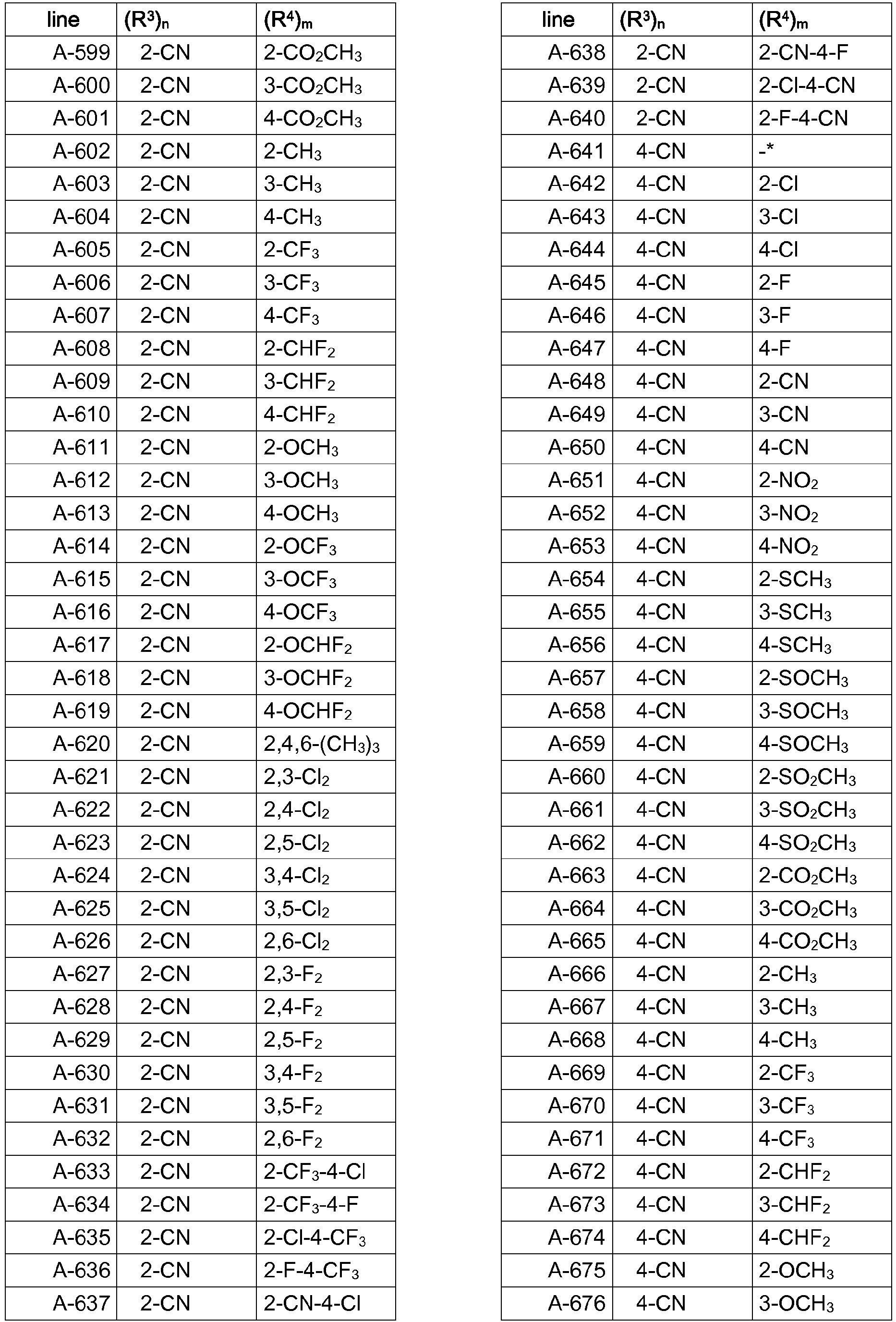

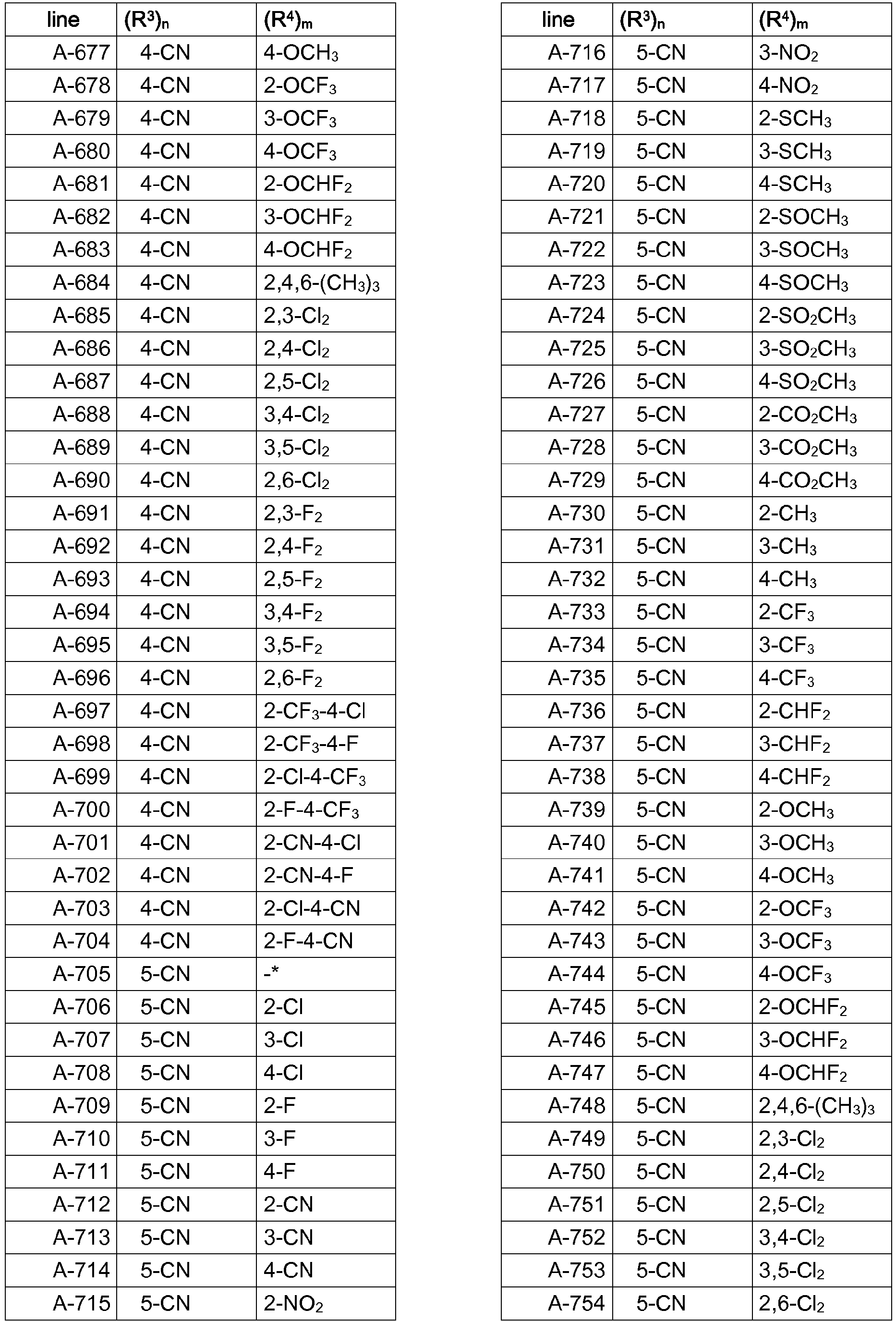

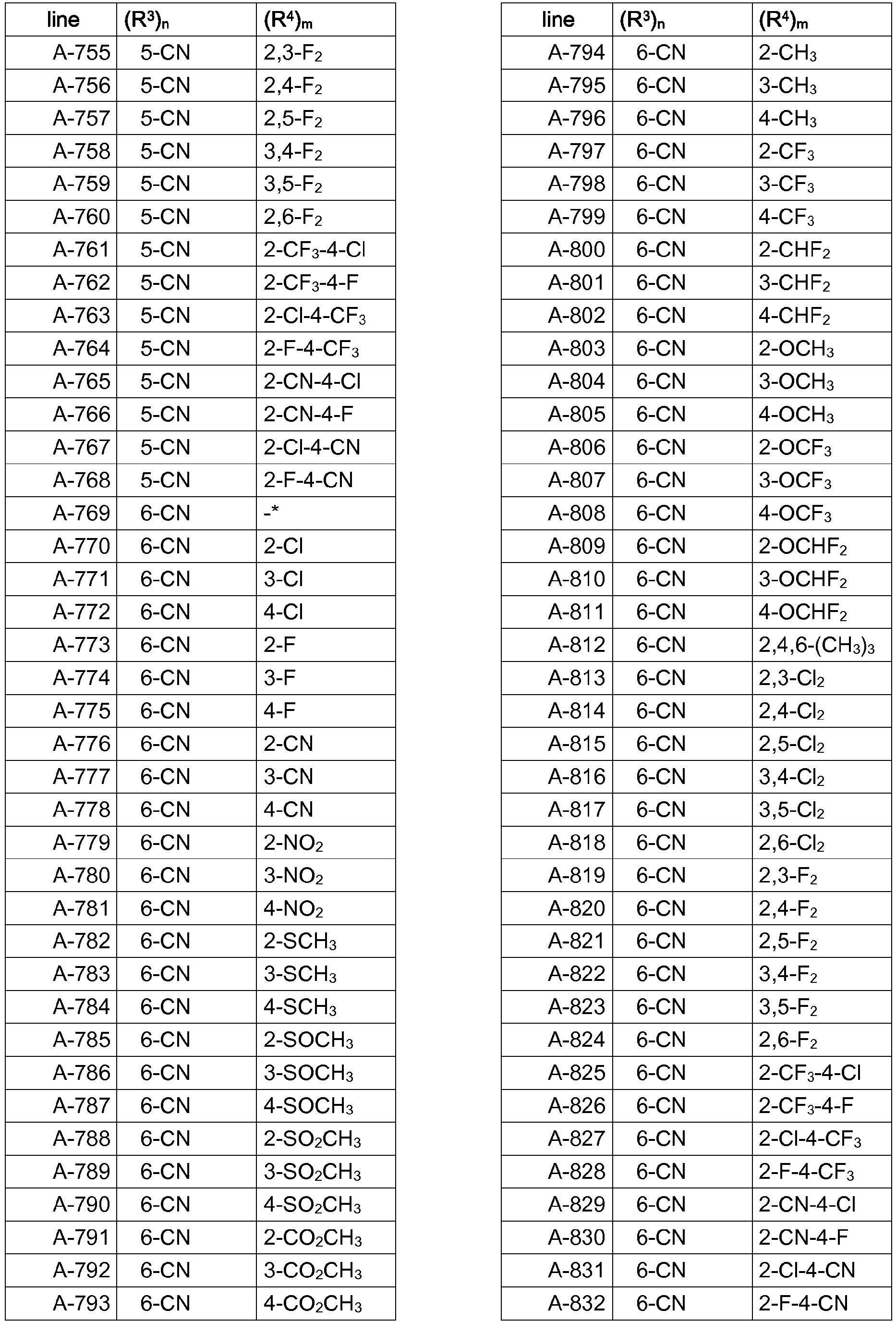

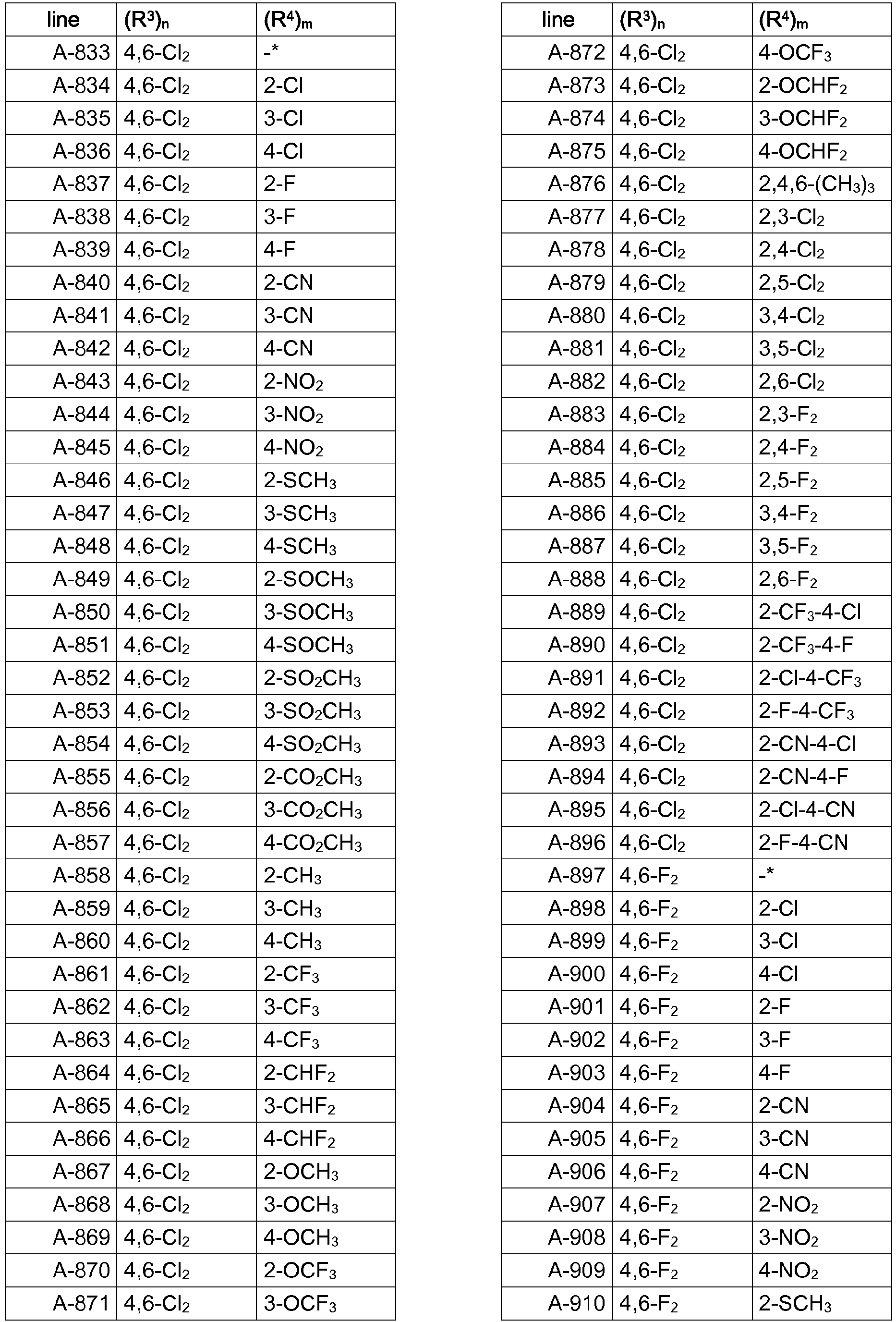

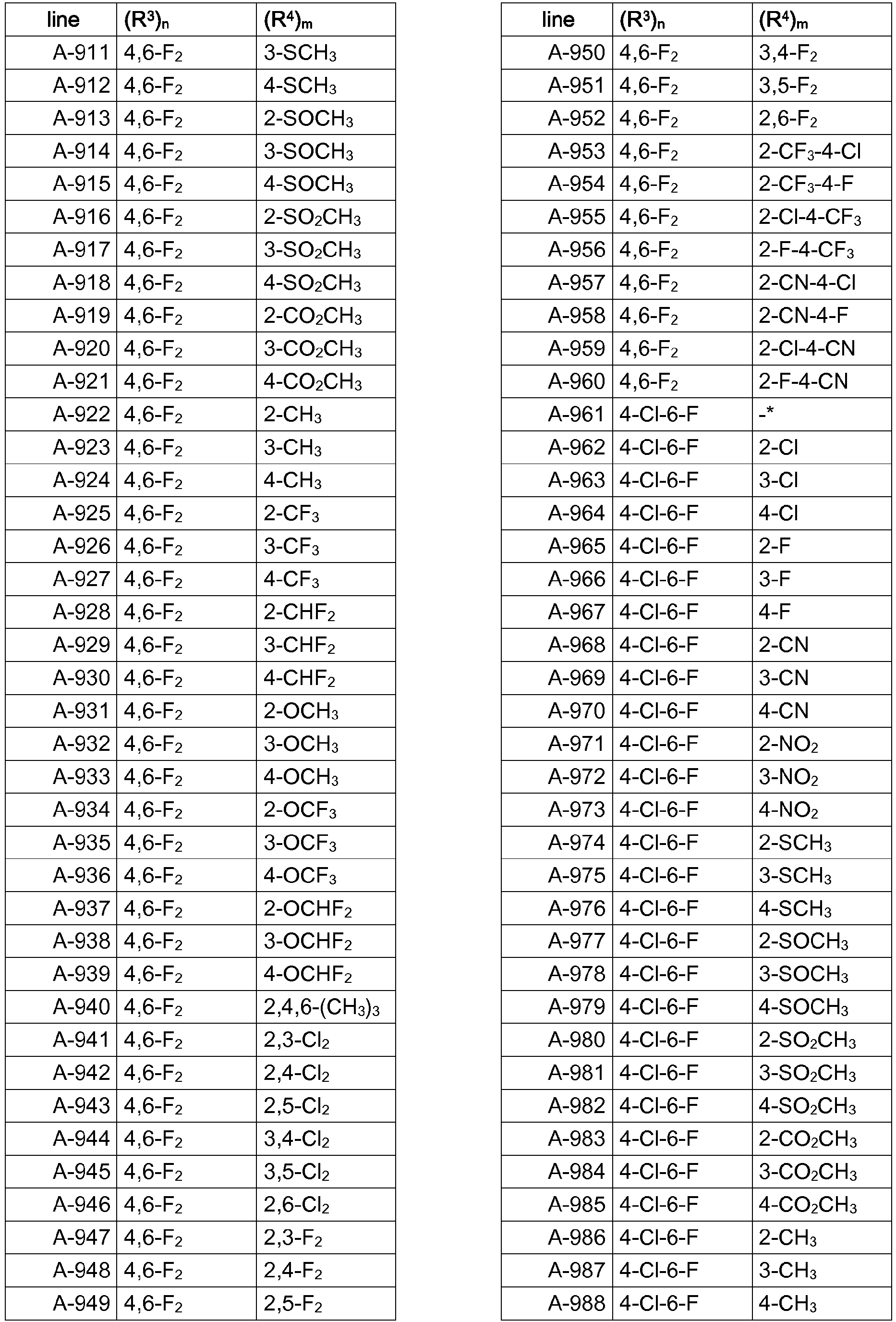

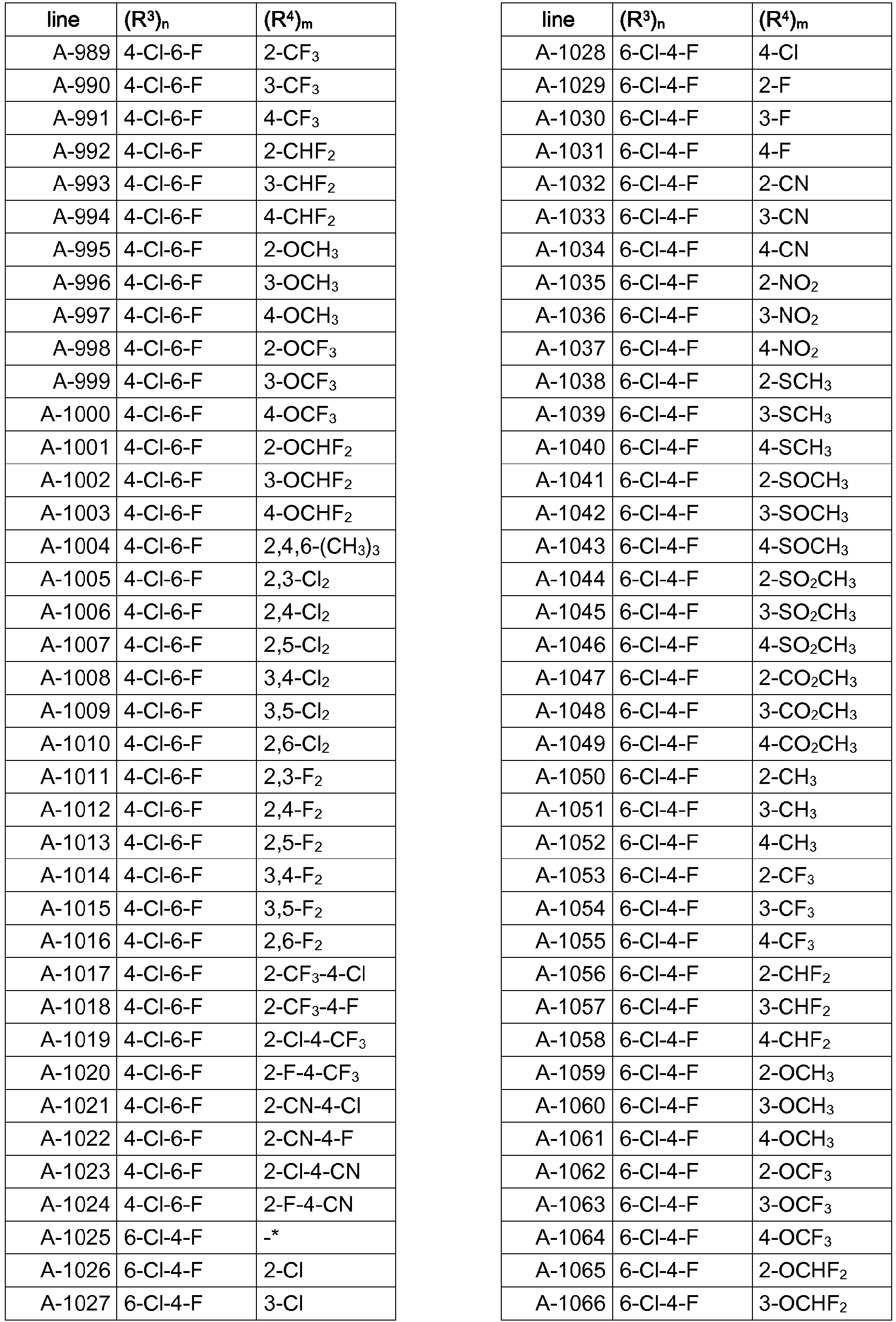

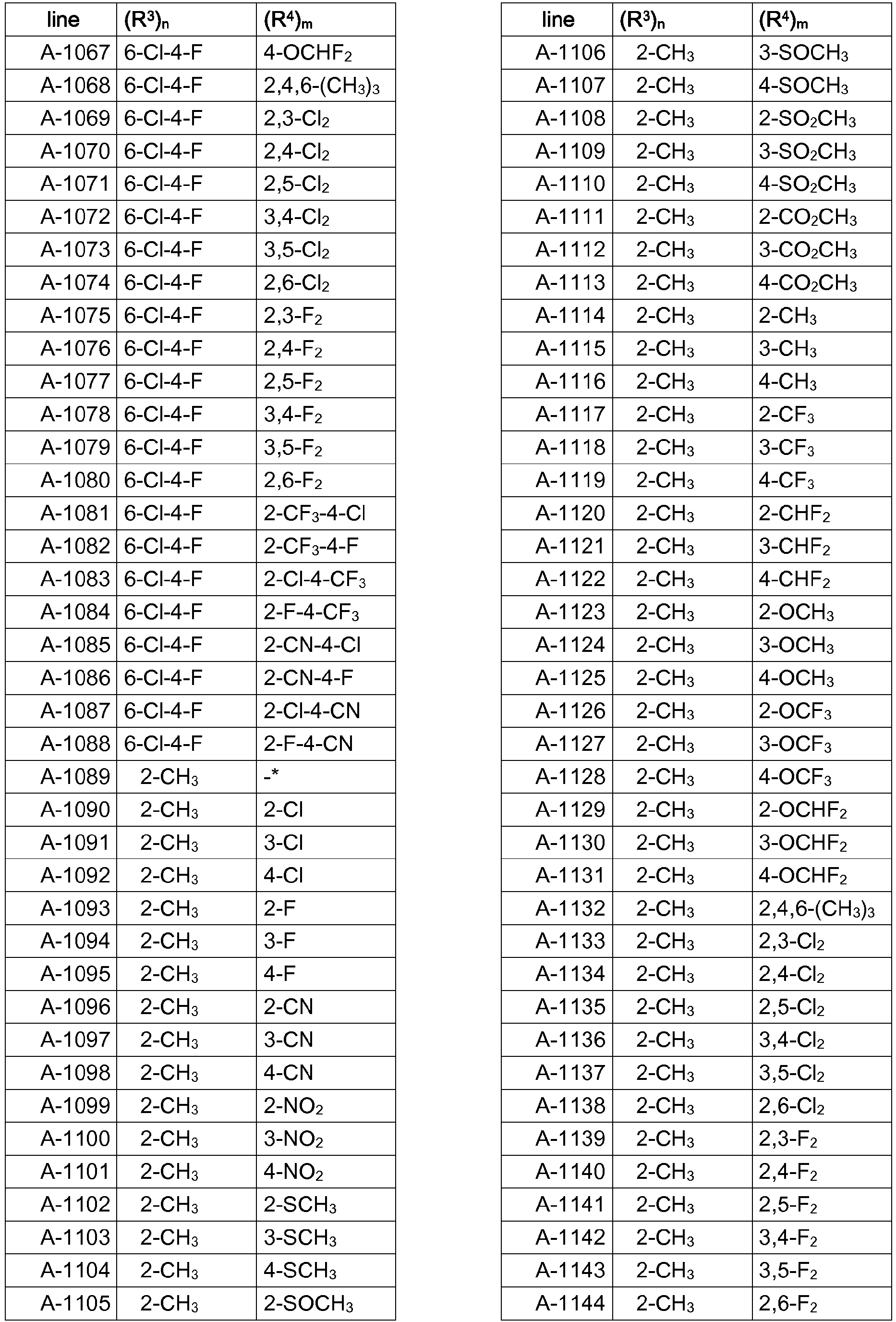

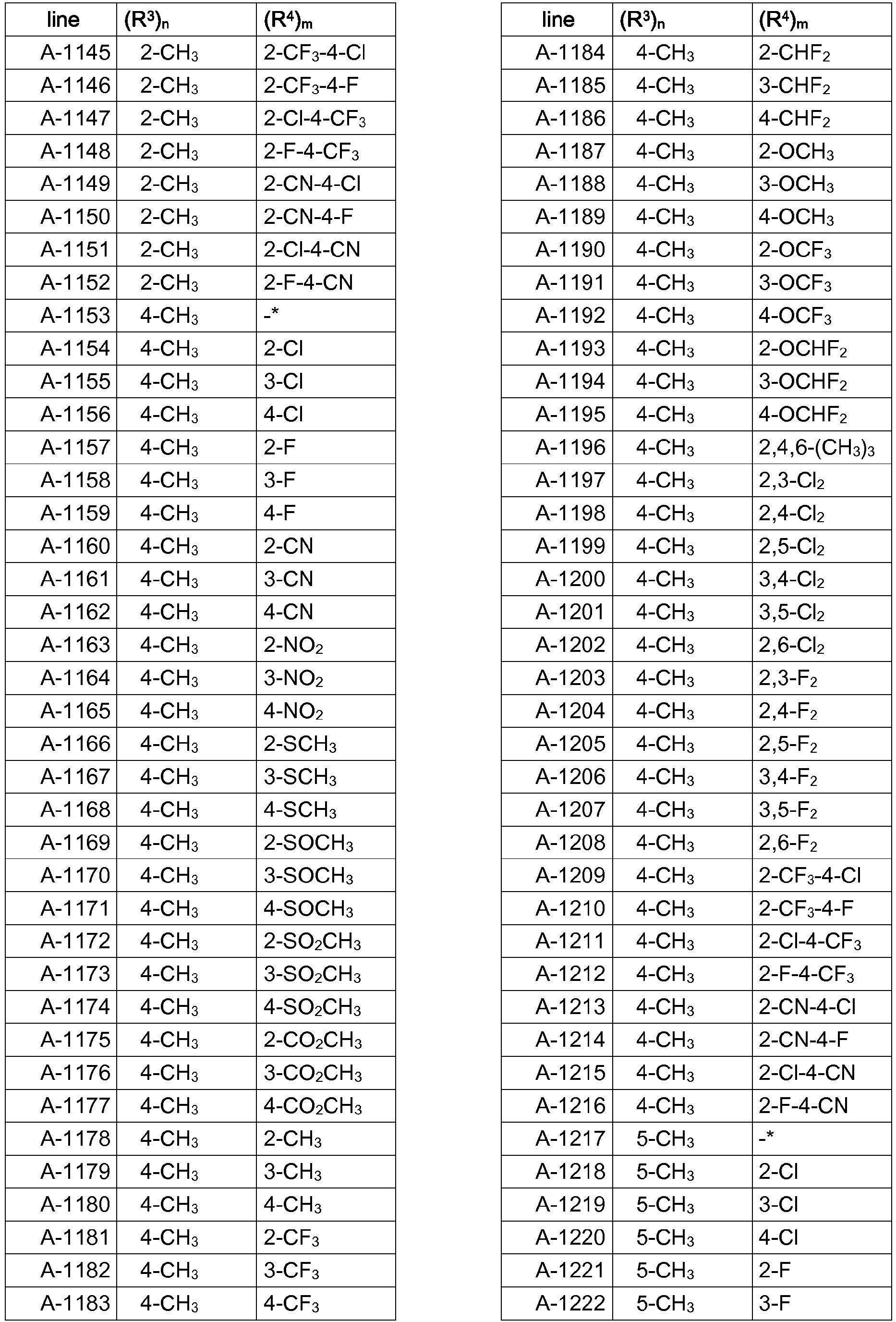

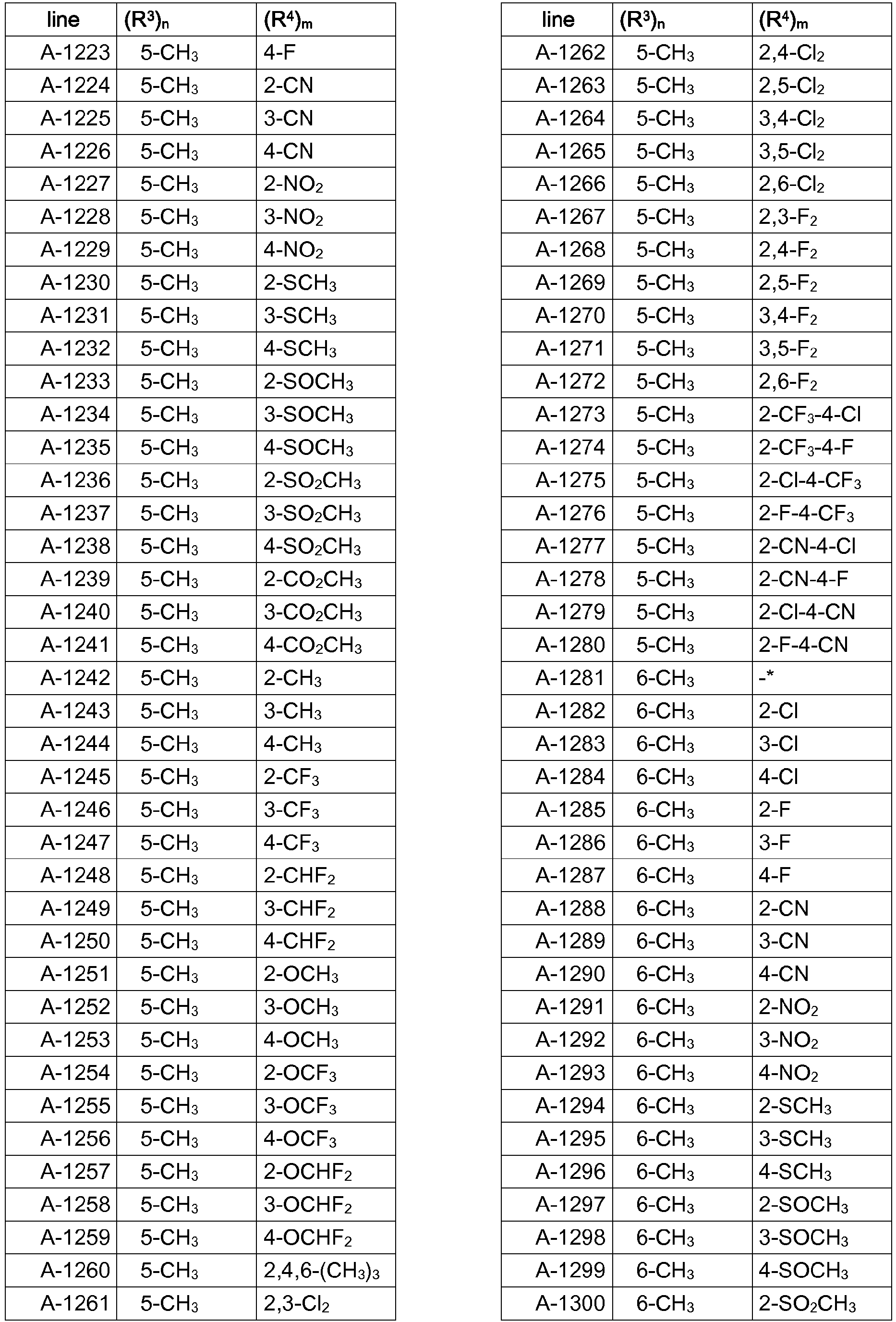

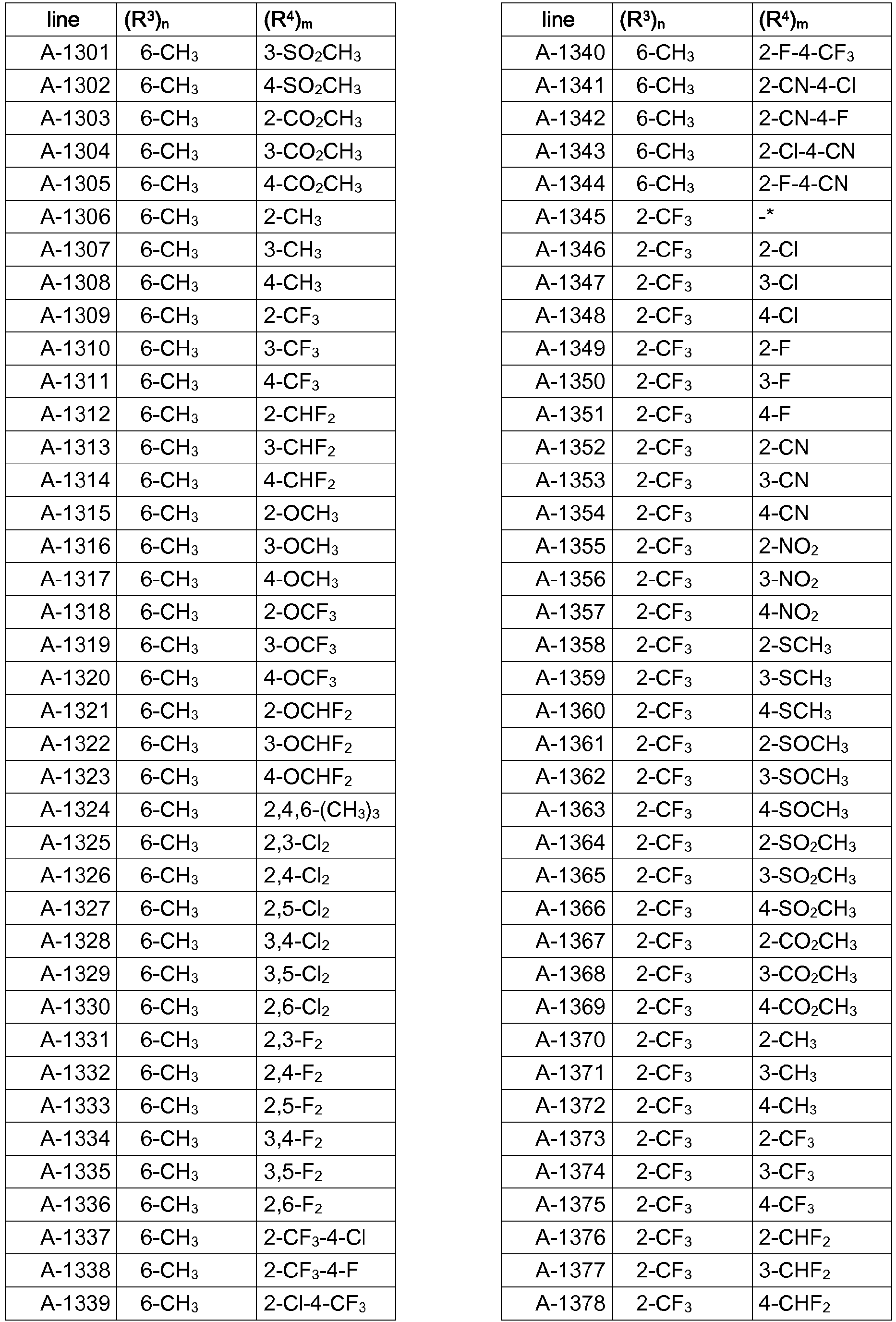

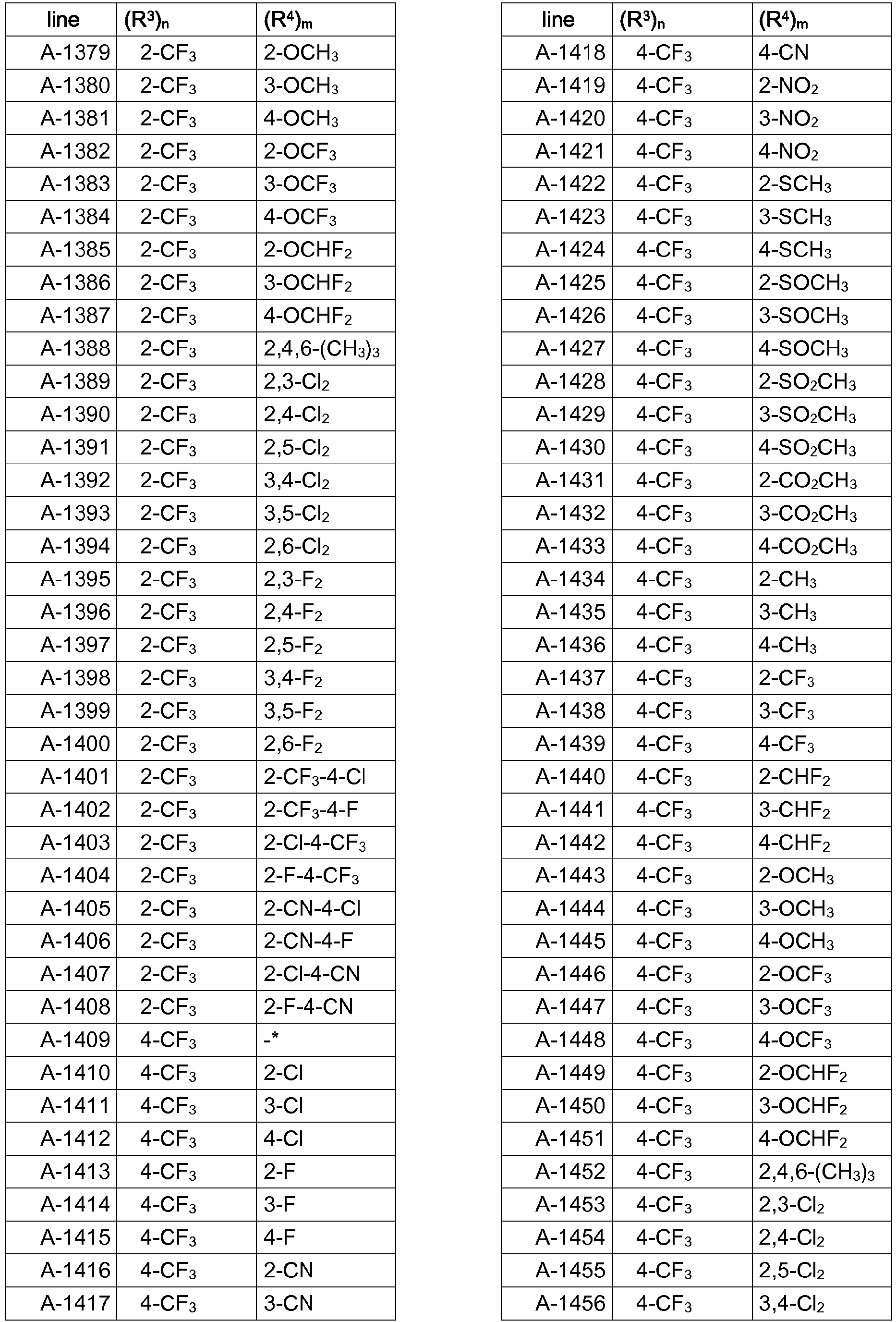

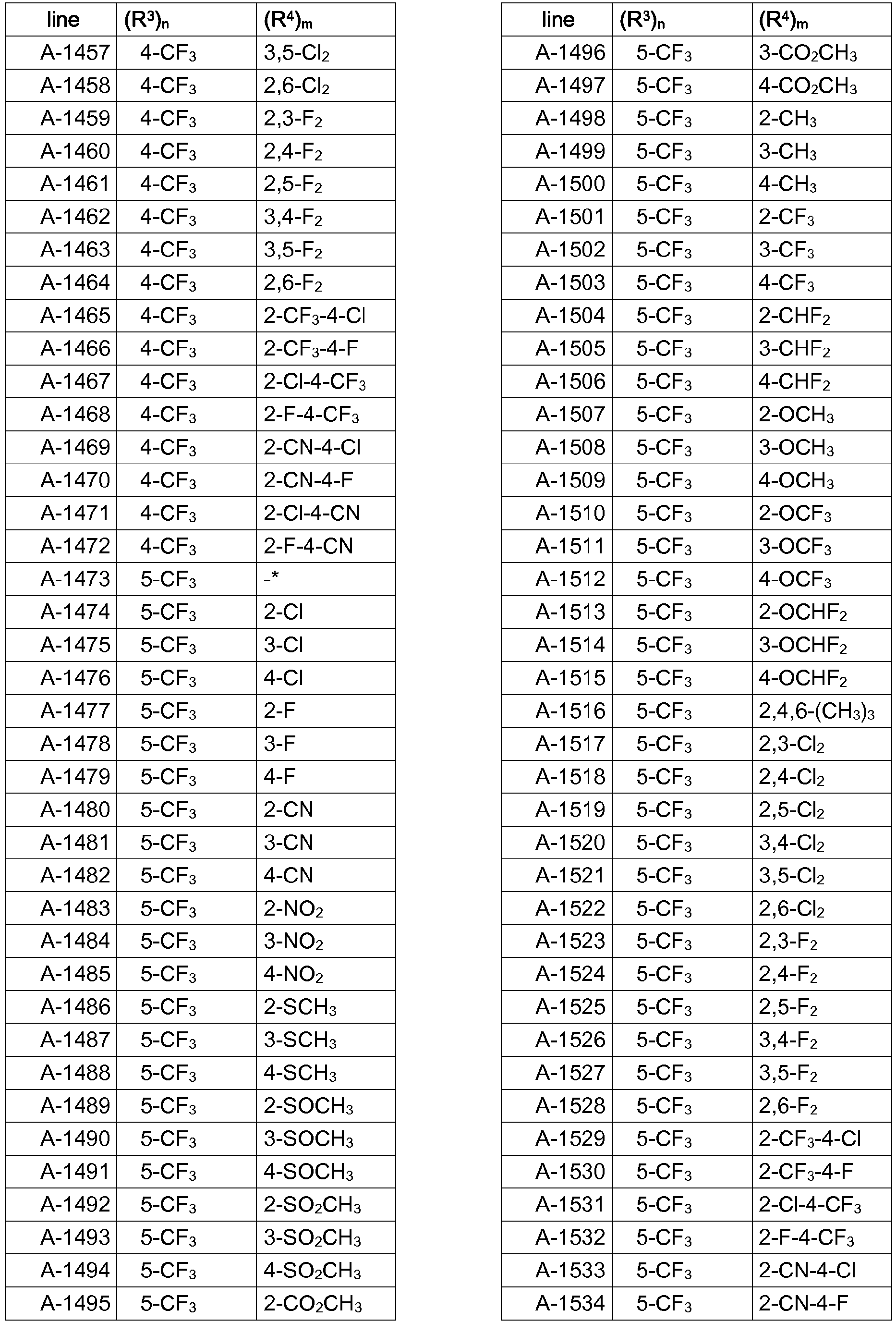

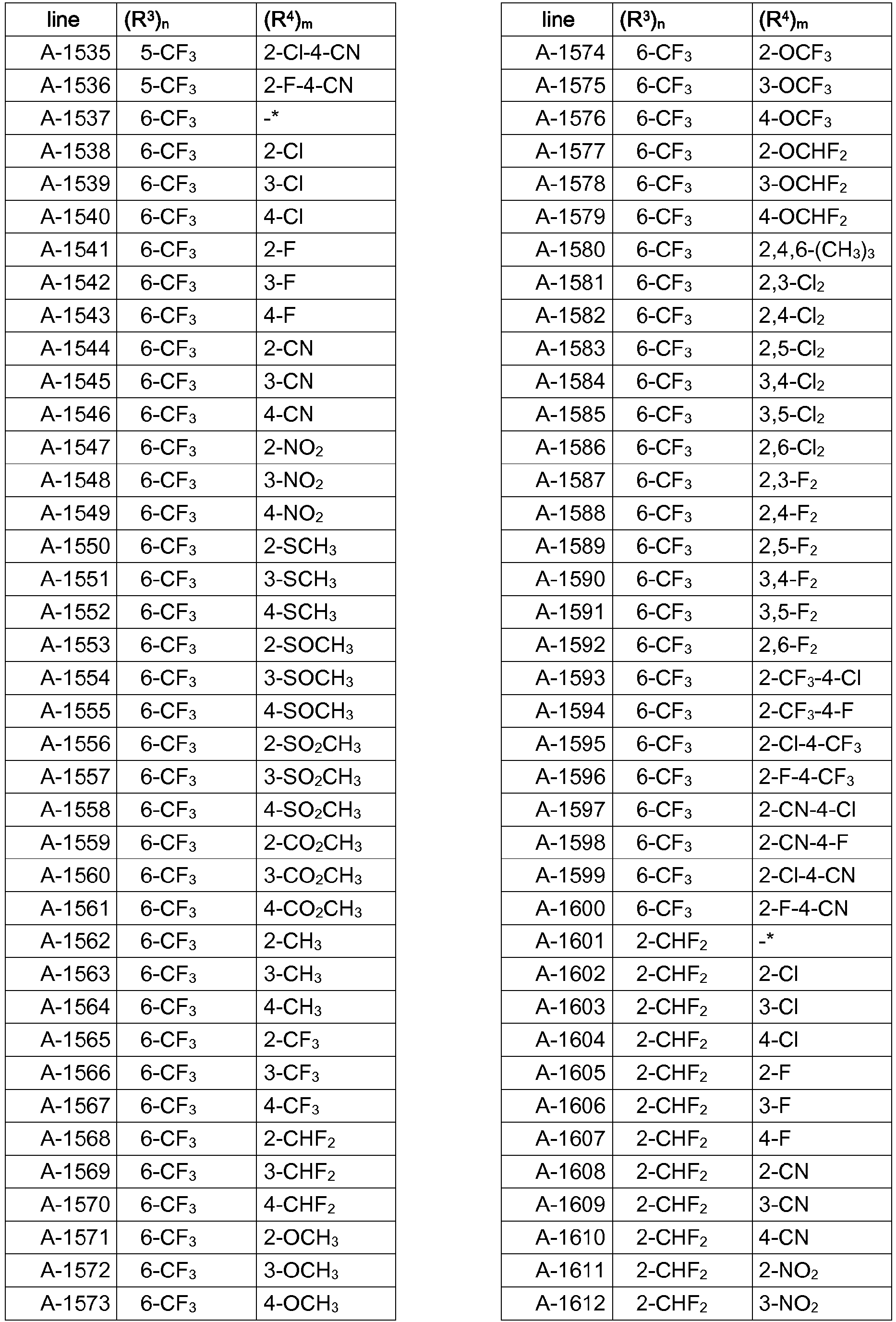

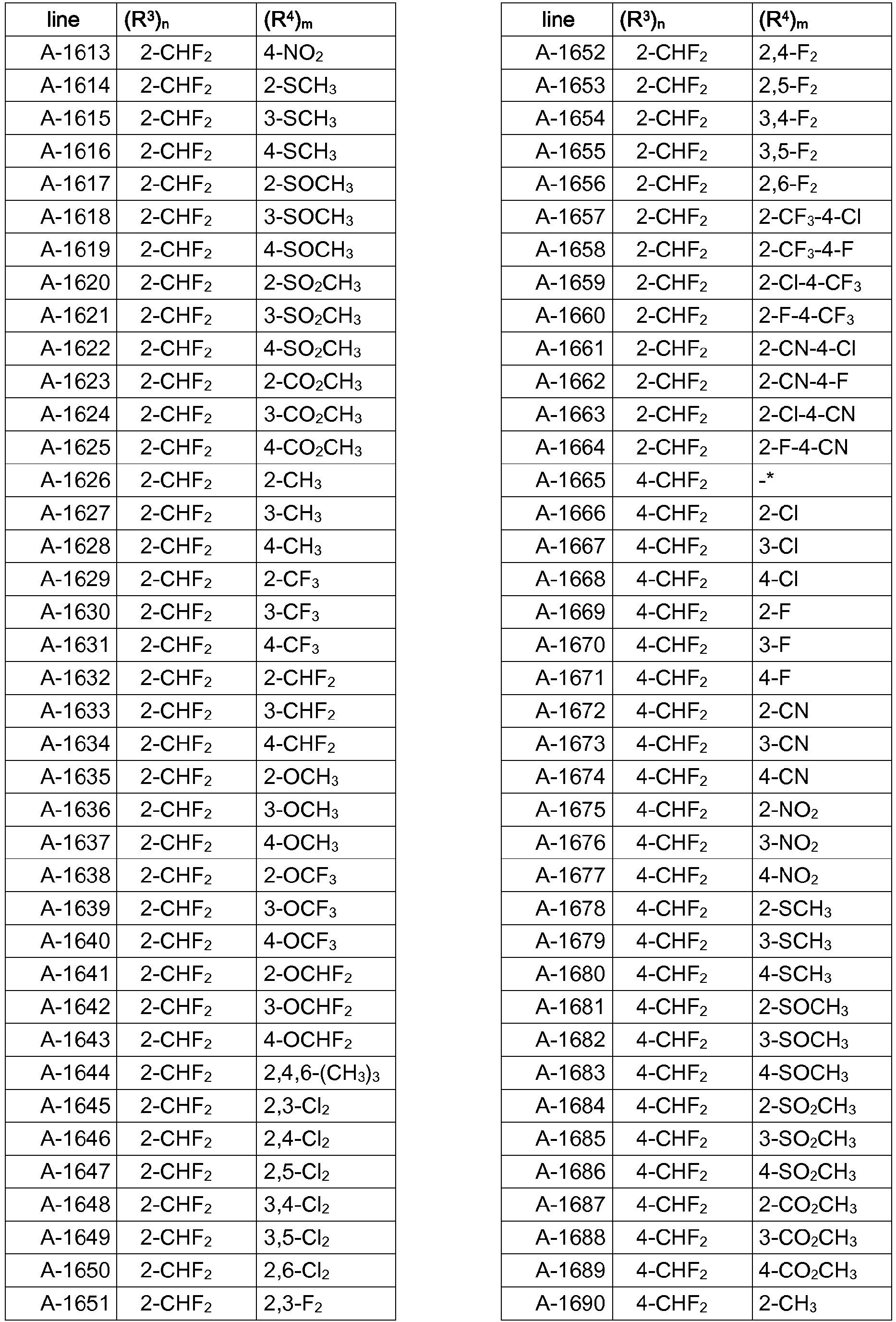

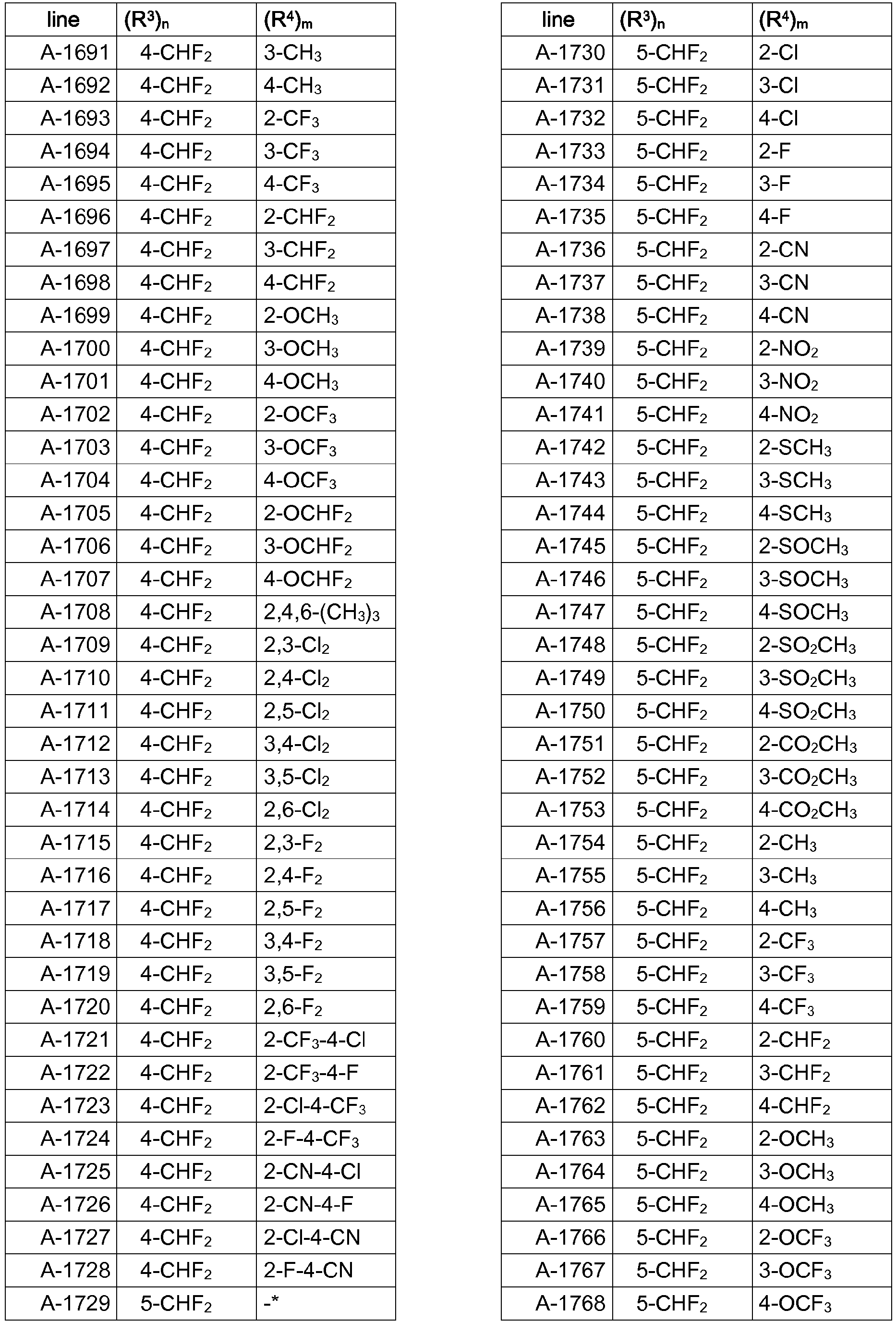

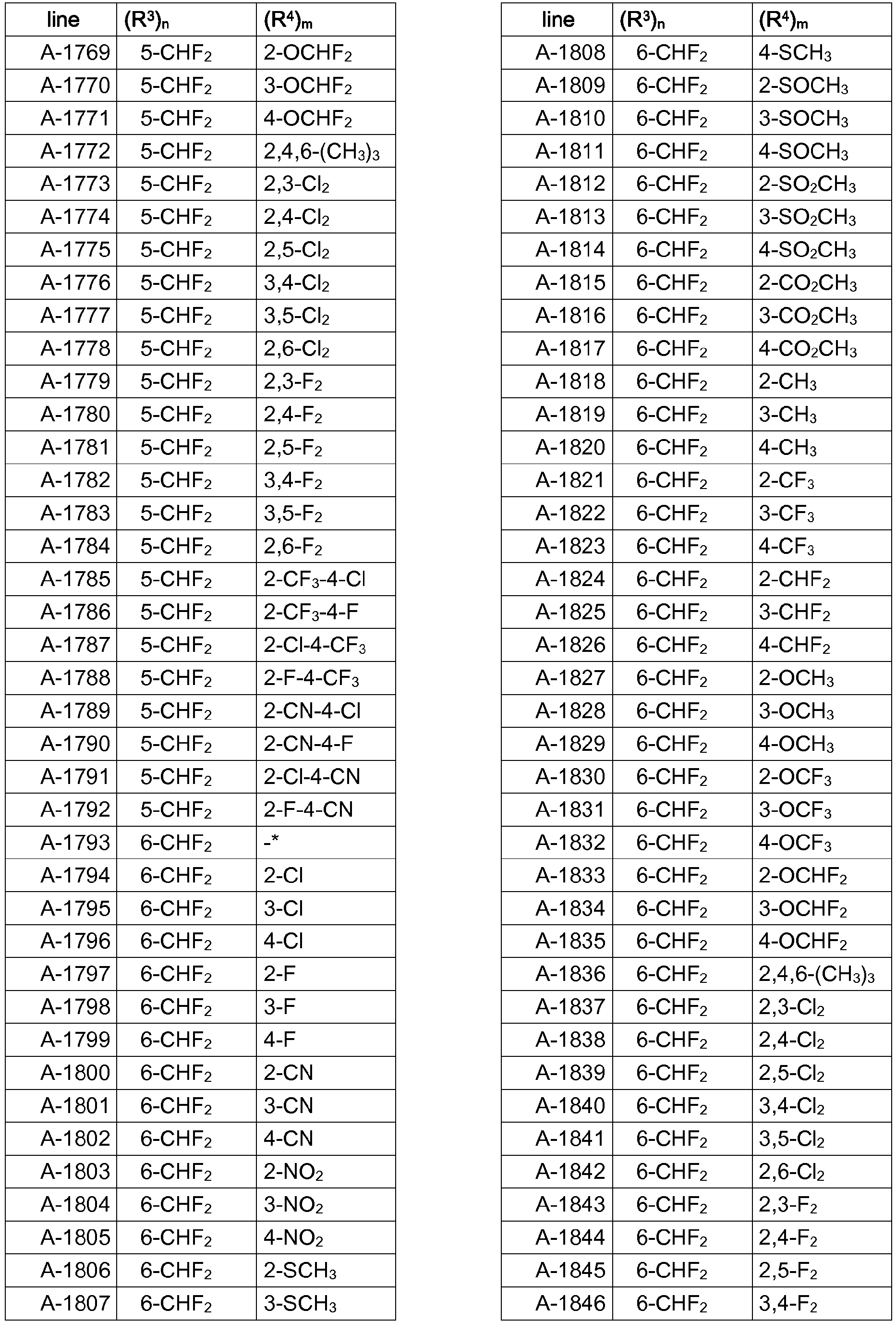

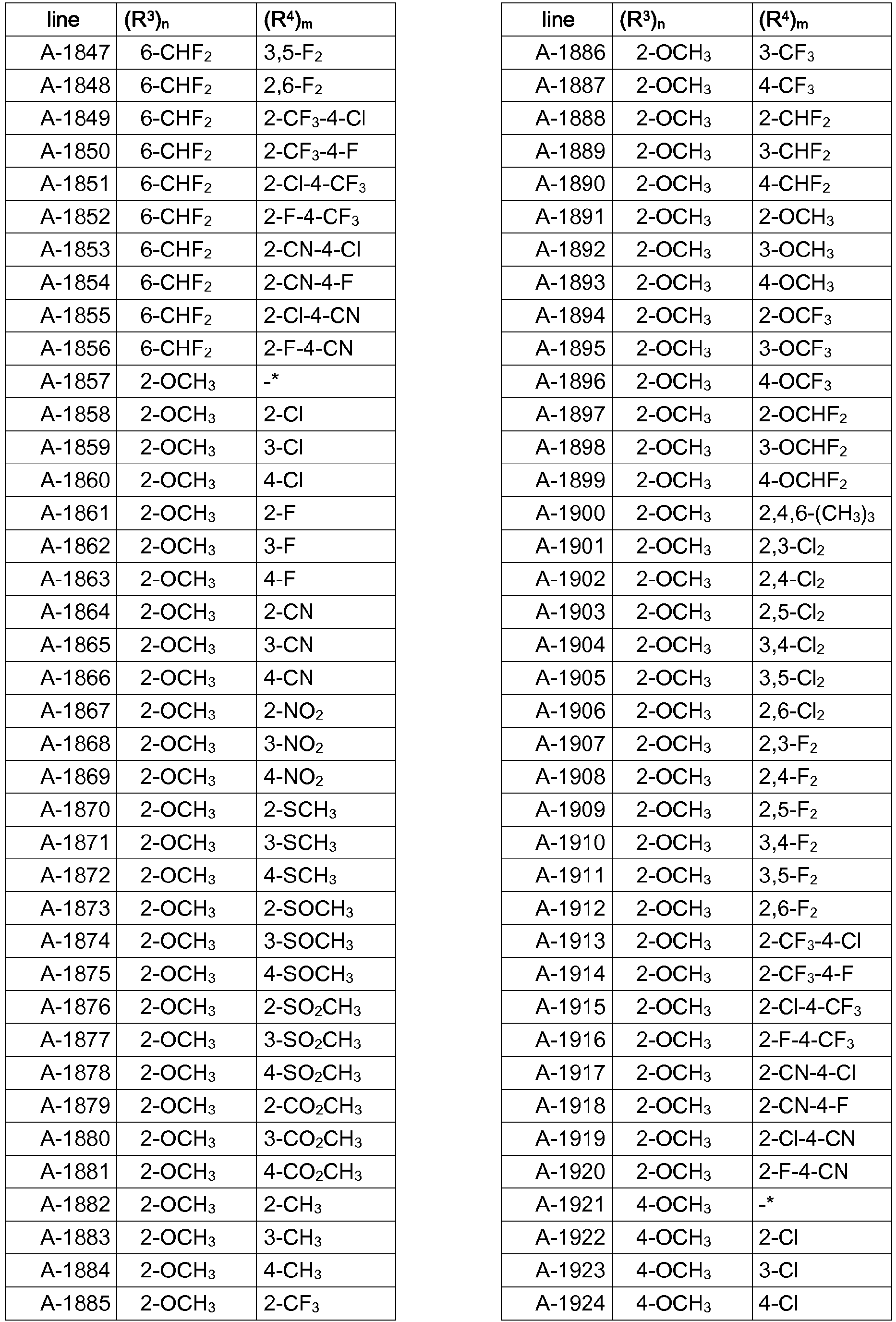

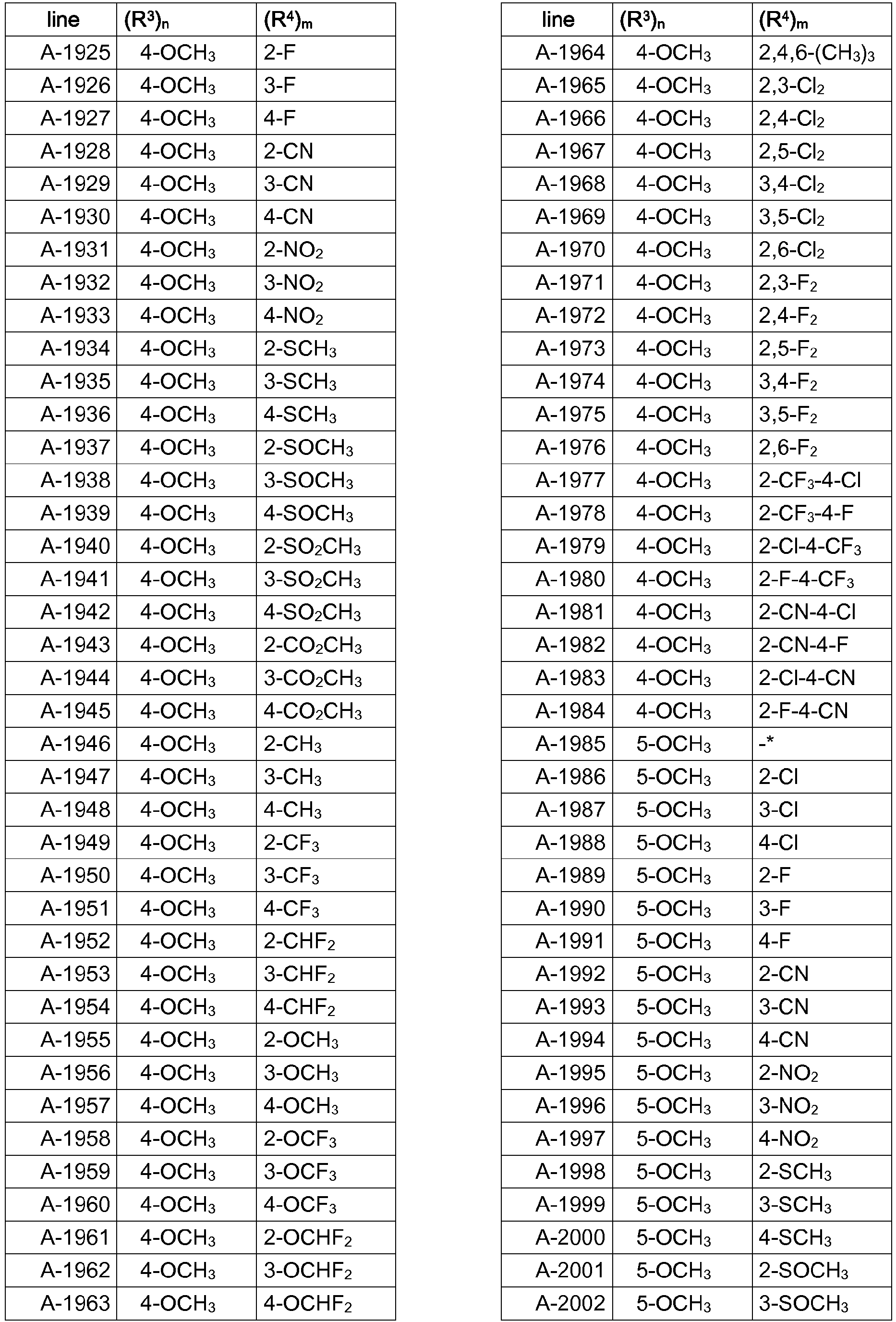

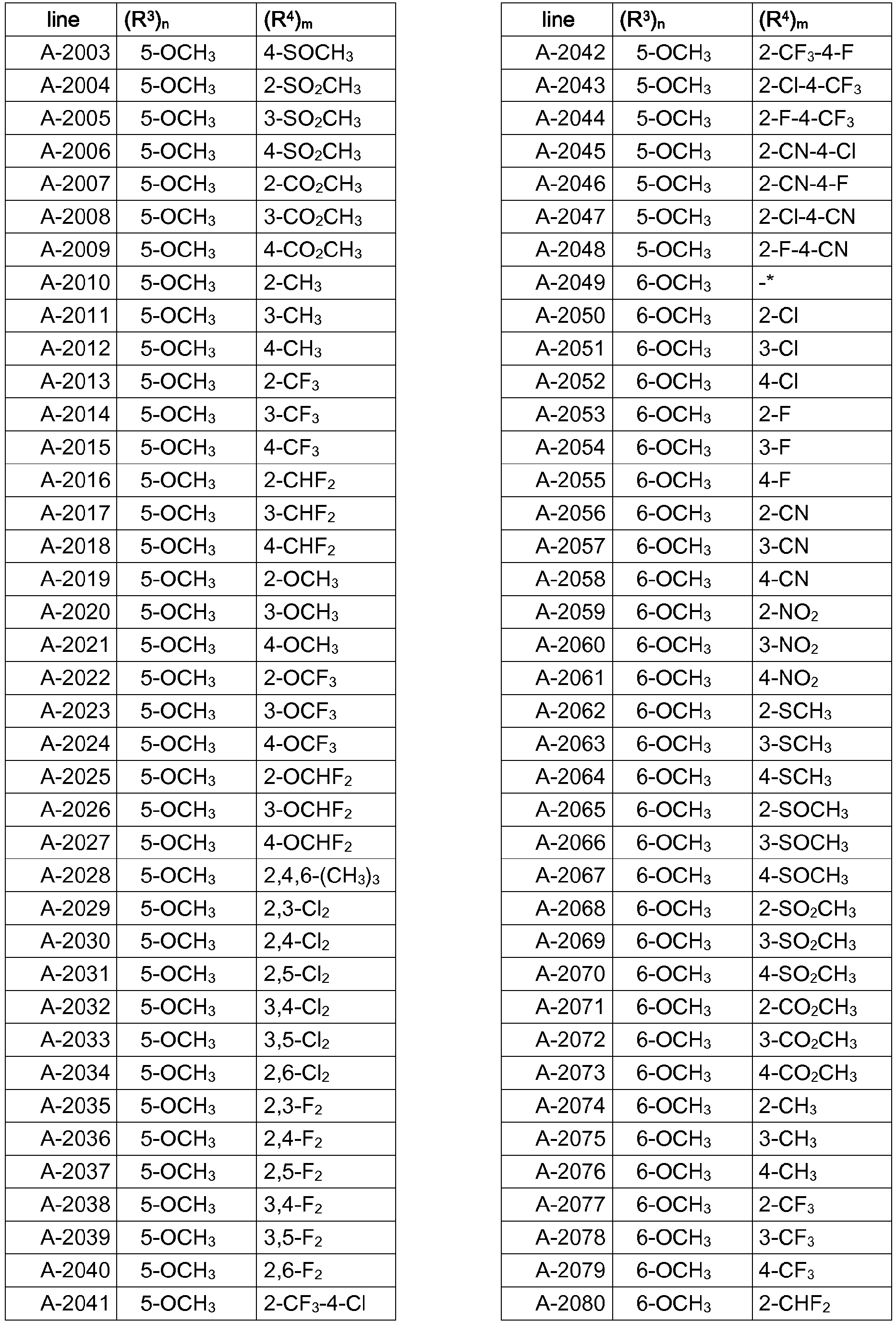

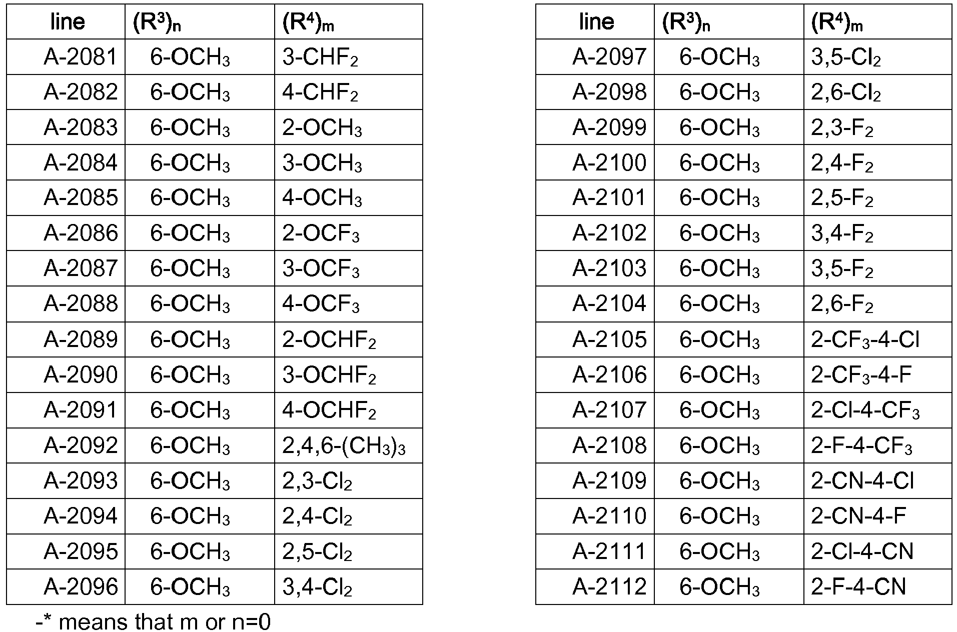

The compounds I and II, respectively, and the compositions according to the invention, respectively, are suitable as fungicides.

Consequently, according to a further aspect, the present invention relates to the use of compounds of formula I, the N-oxides and the agriculturally acceptable salts thereof or of the compositions of the invention for combating phytopathogenic fungi.

Accordingly, the present invention also encompasses a method for combating harmful fungi, comprising treating the fungi or the materials, plants, the soil or seeds to be protected against fungal attack with an effective amount of at least one compound of formula I or with a composition comprising according to the invention.

They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and II, respectively, and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and II, respectively, and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and II, respectively, and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenlated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfoₙl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfoₙI ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), CulItivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdyster-oid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278,

WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT eₙzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum)* or T4-lysozym (e. g. potato cultivars capable of synthesizing these proteins with increased resistance against bacteria such as *Erwinia aₙIvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and II, respectively, and compositions thereof, respectively, are particularly suitable for controlling the following plant diseases:

*Albugo* spp. (white rust) on ornamentals, vegetables (e. g. A. *candida)* and sunflowers (e. g. A. *tragopogonis*); *Alternaria* spp. (Alternaria leaf spot) on vegetables, rape (A. *brassicola* or *brassicae),* sugar beets (A. *tenuis),* fruits, rice, soybeans, potatoes (e. g. A. *solani* or A. *alternata*), tomatoes (e. g. A. *solani* or A. *alternata*) and wheat; *Aphanomyces* spp. on sugar beets and vegetables; *Ascochyta* spp. on cereals and vegetables, e. g. A. *tritici* (anthracnose) on wheat and A. *hordei* on barley; *Bipolaris* and *Drechslera* spp. (teleomorph: *Cochliobolus* spp.), e. g. Southern leaf blight *(D. maydis)* or Northern leaf blight (*B. zeicola)* on corn, e. g. spot blotch (*B. sorokiniana*) on cereals and e.g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (dowₙ mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e.g. Gray leaf spot: *C. zeae-maydis),* rice, sugar beets (e. g. *C. beticola*), sugar cane, vegetables, coffee, soybeans (e. g. *C. sojina* or *C. kikuchii)* and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn *(C. carbonum),* cereals (e. g. *C. sativus,* anamorph: *B. sorokiniana)* and rice (e. g. *C. miyabeanus,* anamorph: *H. oryzae*); *Colletotrichum* (teleomorph: *Glomerella*) spp. (anthracnose) on cotton (e. g. *C. gossypii*)*,* corn (e. g. *C. graminicola:* Anthracnose stalk rot), soft fruits, potatoes (e. g. *C. coccodes:* black dot), beans (e. g. *C. lindemu-thianum)* and soybeans (e. g. *C. truncatum* or C. *gloeosporioides*); *Corticium* spp., e. g. *C*. *sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri:* Black Foot Disease) and ornamentals; *Dematophora* (teleomorph: *Rosellinia)* necatrix (root and stem rot) on soybeans; *Diaporthe* spp., e. g. *D. phaseolorum* (damping off) on soybeans; *Drechslera* (syn. *Helminthosporium,* teleomorph: *Pyrenophora)* spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia* (syn. *Phellinus) punctata, F. mediterranea, Phaeomoniella chlamydospora* (earlier *Phaeoacremonium chlamydosporum), Phaeoacremonium aleophilum* and/or *Botryosphaeria obtusa; Elsinoe* spp. on pome fruits (*E. pyri),* soft fruits (*E*. *veneta:* anthracnose) and vines *(E. ampelina:* anthracnose); *Entyloma oryzae* (leaf smut) on rice; *Epicoccum* spp. (black mold) on wheat; *Erysiphe* spp. (powdery mildew) on sugar beets *(E. betae),* vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum); Fusarium* (teleomorph: *Gibberella)* spp. (wilt, root or stem rot) on various plants, such as F. *graminearum* or *F. culmorum* (root rot, scab or head blight) on cereals (e. g. wheat or barley), F. *oxysporum* on tomatoes, *F. solani* (f*.* sp. glycines now syn. F. virguliforme ) and F. tucumaniae and F. brasiliense each causing sudden death syndrome on soybeans and *F. verticillioides* on corn; *Gaeumannoynces graminis* (take-all) on cereals (e. g. wheat or barley) and corn; *Gibberella* spp. on cereals (e. g. *G. zeae)* and rice (e. g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus)* on corn, cereals and rice; *Hemileia* spp., e. g. *H. vastatrix* (coffee leaf rust) on coffee; *Isariopsis clavispora* (syn. *Cladosporium vitis)* on vines; *Macrophomina phaseolina* (syn. *phaseoli)* (root and stem rot) on soybeans and cotton; *Microdochium* (syn. *Fusarium) nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *_{N}cosphaerella* spp. on cereals, bananas, soft fruits and ground nuts, such as e. g. *M. graminicola* (anamorph: *Septoria tritici,* Septoria blotch) on wheat or M. *fijiensis* (black Sigatoka disease) on bananas; *Peronospora* spp. (dowₙ mildew) on cabbage (e. g. *P. brassicae),* rape (e. g. *P. parasitica),* onions (e. g. *P. destructor),* tobacco (*P*. *tabacina)* and soybeans (e. g. *P. manshurica); Phakopsora pachyrhizi* and *P. meibomiae* (soybean rust) on soybeans; *Phialophora* spp. e. g. on vines (e. g. *P. tracheiphila* and *P. tetraspora)* and soybeans (e. g. *P. gregata:* stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola:* can and leaf spot) and soybeans (e. g. stem rot: *P. phaseoli,* teleomorph: *Diaporthe phaseolorum); Physoderma maydis* (brown spots) on corn; *Phytophthora* spp. (wilt, root, leaf, fruit and stem root) on various plants, such as paprika and cucurbits (e. g. *P. capsici),* soybeans (e. g. *P. megasperma,* syn. *P*. *sojae),* potatoes and tomatoes (e. g. *P. infestans:* late blight) and broad-leaved trees (e. g. *P*. *ramorum:* sudden oak death); *Plasmodiophora brassicae* (club root) on cabbage, rape, radish and other plants; *Plasmopara* spp., e. g. *P. viticola* (grapevine dowₙ mildew) on vines and *P*. *halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat *(P. graminis)* and sugar beets *(P. betae)* and thereby transmitted viral diseases; *Pseudocercosporella herpotrichoides* (eyespot, teleomorph: *Tapesia yallundae)* on cereals, e. g. wheat or barley; *Pseudoperonospora* (dowₙ mildew) on various plants, e. g. *P*. *cubensis* on cucurbits or *P*. *humili* on hop; *Pseudopezicula tracheiphila* (red fire disease or ,rotbrenner', anamorph: *Phialophora)* on vines; *Puccinia* spp. (rusts) on various plants, e. g. *P*. *triticina* (brown or leaf rust), *P*. *striiformis* (stripe or yellow rust), *P*. *hordei* (dwarf rust), *P*. *graminis* (stem or black rust) or *P*. *recondita* (brown or leaf rust) on cereals, such as e. g. wheat, barley or rye, *P*. *kuehnii* (orange rust) on sugar cane and *P*. *asparagi* on asparagus; *Pyrenophora* (anamorph: *Drechslera*) *tritici-repentis* (tan spot) on wheat or *P*. *teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphani-dermatum); Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum)* and soybeans (e. g. *S. rolfsii* or *S. sclerotiorum); Septoria* spp. on various plants, e. g. *S. glycines* (brown spot) on soybeans, *S. tritici* (Septoria blotch) on wheat and *S.* (syn. *Stagonospora)* nodorum (Stagonospora blotch) on cereals; *Uncinula* (syn. *Erysiphe) necator* (powdery mildew, anamorph: *Oidium tuck-eri)* on vines; *Setospaeria* spp. (leaf blight) on corn (e. g. *S. turcicum,* syn. *Helminthosporium turcicum)* and turf; *Sphacelotheca* spp. (smut) on corn, (e. g. *S. reiliana:* head smut), sorghum und sugar cane; *Sphaerotheca fuliginea* (powdery mildew) on cucurbits; *Spongospora subterra-nea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeo-sphaeria] nodorum)* on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara e*/*egans); Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendicula-tus,* syn. *U. phaseoli)* and sugar beets (e. g. *U. betae); Ustilago* spp. (loose smut) on cereals (e. g. *U. nuda* and *U. avaenae),* corn (e. g. *U. maydis:* corn smut) and sugar cane; *Venturia* spp. (scab) on apples (e. g. *V. inaequalis)* and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and II, respectively, and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paeciloₙces* spp. and Zygomycetes such as *Mucor spp.,* and in addition in the protection of stored products and harvest the following yeast fungi are worthy of note: *Candida* spp. and *Saccharomyces cerevisae.*

The compounds I and II, respectively, and compositions thereof, respectively, may be used for improving the health of a plant. The invention also relates to a method for improving plant health by treating a plant, its propagation material and/or the locus where the plant is growing or is to grow with an effective amount of compounds I or II, respectively, and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I and II, respectively, can be present in different crystal modifications whose biological activity may differ. They are likewise subject matter of the present invention.

The compounds I and II, respectively, are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I or II, respectively, as such or a composition comprising at least one compound I or II, respectively, prophylactically either at or before planting or transplanting.

The invention also relates to compositions comprising one compound I and II, respectively, according to the invention. In particular, such composition further comprises an auxiliary as defined below.

The term "effective amount" used denotes an amount of the composition or of the compounds I or II, respectively, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I or II, respectively, used.

The compounds I and II, respectively, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I or II, respectively, and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I or II, respectively, and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I or II, respectively, and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I or II, respectively, and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I or II, respectively, are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I or II, respectively, are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I or II, respectively, are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound I or II, respectively, are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
ix) Microemulsion (ME)
   5-20 wt% of a compound I or II, respectively, are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I or II, respectively, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
xi) Dustable powders (DP, DS)
   1-10 wt% of a compound I or II, respectively, are ground finely and mixed intimately with solid carrier (e.g. finely divided kaolin) ad 100 wt%.
xii) Granules (GR, FG)
   0.5-30 wt% of a compound I or II, respectively, is ground finely and associated with solid carrier (e.g. silicate) ad 100 wt%. Granulation is achieved by extrusion, spray-drying or fluidized bed.
xiii) Ultra-low volume liquids (UL)
   1-50 wt% of a compound I or II, respectively, are dissolved in organic solvent (e.g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and II, respectively, and compositions thereof, respectively, on to plant propagation material, especially seeds include dressing, coating, pelleting, dusting, soaking and in-furrow application methods of the propagation material. Preferably, compound I and II, respectively, or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 g to 10 kg, in particular 0.1 to 1000 g, more particularly from 1 to 1000 g, specificaly from 1 to 100 g and most specificaly from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a composition comprising two or three active ingredients, may be mixed by the user himself in a spray tank and further auxiliaries may be added, if appropriate.

Mixing the compounds I or II, respectively, or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the compounds I and II, respectively,can be used, is intended to illustrate the possible combinations but does not limit them:
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxystrobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxy-methyl)-phenyl)-2-methoxyimino-N-methyl-acetamide, pyribencarb, triclopyricarb/chlorodincarb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate;
   - inhibitors of complex II (e. g. carboxamides): benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1 H-pyrazole-4-carboxamide, N-(2-(1,3,3-trimethyl-butyl)-phenyl)-1,3-dimethyl-5-fluoro-1 H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, N-(7-fluoro-1,1,3-trimethyl-indan-4-yl)-1,3-dimethyl-pyrazole-4-carboxamide, N-[2-(2,4-dichlorophenyl)-2-methoxy-1-methyl-ethyl]-3-(difluoromethyl)-1-methyl-pyrazole-4-carboxamide;
   - other respiration inhibitors (e.g. complex I, uncouplers): diflumetorim, (5,8-difluoro-quinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazole, *2-*[*rel-*(*2S;*3*R)*-3*-*(2*-*chlorophenyl)*-*2-(2,4*-*difluorophenyl)-oxiranylmethyl]-2H-[1,2,4]triazole-3-thiol, 2-[2-chloro-4-(4-chlorophenoxy)phenyl]-1-(1,2,4-triazol-1-yl)pentan-2-ol, 1-[4-(4-chlorophenoxy)-2-(trifluoromethyl)phenyl]-1-cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol, 2-[2-chloro-4-(4-chlorophenoxy)phenyl]-1-(1,2,4-triazol-1-yl)pent-3-yn-2-ol; imidazoles: imazalil, pefurazoate, prochloraz, triflumizol; pyrimidines, pyridines and piperazines: fenarimol, nuarimol, pyrifenox, triforine, [3-(4-chloro-2-fluoro-phenyl)-5-(2,4-difluorophenyl)isoxazol-4-yl]-(3-pyridyl)methanol;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benoₙI, carbendazim, fuber-idazole, thiabendazole, thiophanate-methyl; triazolopyrimidines: 5-chloro-7-(4-methylpiperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethan-il;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)-ethanesulfomyl)-but-2-yl) carbamic acid-(4-fluorophenyl) ester;
   - compounds affecting cell membrane permeability and fatty acides: propamocarb, propamocarb-hydrochlorid
   - fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1 H-pyrazol-1-yl]ethanone; 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate, 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate;
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlor-phenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1H,5H-[1,4]dithiino[2,3-c:5,6-c']dipyrrole-1,3,5,7(2H,6H)-tetraone;
I) Cell wall synthesis inhibitors
   - inhibitors of glucan synthesis: validaₙcin, polyoxin B; melanin synthesis inhibitors: pyroqui-Ion, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, tolprocarb, 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1 -yl]ethanone, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxy-acetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole),
      N-(6-methoxy-pyridin-3-yl) cyclopropanecarboxylic acid amide, 5-chloro-1-(4,6-dimethoxy-pyrimidin-2-yl)-2-methyl-1 H-benzoimidazole, 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate, tert-butyl N-[6-[[(Z)-[(1-methyltetrazol-5-yl)-phenyl-methylene]amino]oxymethyl]-2-pyridyl]carbamate, 2-[2-[(7,8-difluoro-2-methyl-3-quinolyl)oxy]-6-fluoro-phenyl]propan-2-ol, 2-[2-fluoro-6-[(8-fluoro-2-methyl-3-quinolyl)oxy]phenyl]propan-2-ol, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline; 9-fluoro-2,2-dimethyl-5-(3-quinolyl)-3H-1,4-benzoxazepine; picarbutrazox;
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus amyloliquefaciens, B. mojavensis, B. pumilus, B. simplex, B. solisalsi, B. subtilis, B. subtilis* var. *amyloliquefaciens, Candida oleophila, C. saitoana, Clavibacter michiga-nensis* (bacteriophages), *Coniothyrium minitans, Cryphonectria parasitica, Crypto-coccus albidus, Dilophosphora alopecuri, Fusarium oxysporum, Clonostachys rosea* f. *catenulate* (also named *Gliocladium catenulatum), Gliocladium roseum, Lysobacter an-tibioticus, L. enzymogenes, Metschnikowia fructicola, Microdochium dimerum, Micro-sphaeropsis ochracea, Muscodor albus, Paenibacillus polymyxa, Pantoea vagans, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. microflavus, S. violaceusniger, Talaromyces flavus, Trichoderma asperellum, T. atroviride, T. fertile, T. gamsii, T. harmatum, T. harzianum;* mixture of *T. harzianum* and *T. viride;* mixture of *T. polysporum* and *T. harzianum; T. stromaticum, T. virens* (also named *Gliocladium virens), T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: chitosan (hydrolysate), harpin protein, laminarin, Menhaden fish oil, natamycin, Plum pox virus coat protein, potassium or sodium bicarbonate, *Reynoutria sachlinensis* extract, salicylic acid, tea tree oil;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* sp., *Chromobacterium subtsugae,* Cydia pomonella granulosis virus, *Cryptophlebia leucotreta* granulovirus (CrIeGV), *Flavobacterium sp., Helicoverpa armigera* nucleopolyhedrovirus (HearNPV), *Isaria fumosorosea, Heterorhabditis* bacteriophora, *Lecanicillium longisporum, L. muscarium* (formerly *Verticillium lecanii), Metarhizium anisopliae, M. anisopliae* var. *acridum, Nomuraea rileyi, Paecilomyces fumosoroseus, P. lilacinus, Paenibacillus popilliae, Pasteuria* sp., *P. nishizawae, P. penetrans, P. ramose, P. reneformis, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces microflavus;*
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, potassium silicate, sorbitol actanoate, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, Acacia negra extract, extract of grapefruit seeds and pulp, extract of *Chenopodium ambrosiodes,* Catnip oil, Neem oil, Quillay extract, Tagetes oil;
   L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* sp., *B. elkanii, B. japonicum, B. liaoningense, B. lupini, Delftia acidovorans, Glomus intraradices, Mesorhizobium* sp., *Paenibacillus alvei, Penicillium bilaiae, Rhizobium leguminosarum* bv. *phaseoli, R. I.* bv. *trifolii, R. I.* bv. *viciae, R. tropici, Sinorhizobium meliloti;*
   L6) Biochemical pesticides with plant stress reducing, plant growth regulator and/or plant yield enhancing activity: abscisic acid, aluminium silicate (kaolin), 3-decen-2-one, formononetin, genistein, hesperetin, homobrassinlide, humates, jasmonic acid or salts or derivatives thereof, lysophosphatidyl ethanolamine, naringenin, polymeric polyhydroxy acid, *Ascophyllum nodosum* (Norwegian kelp, Brown kelp) extract and *Ecklonia maxima* (kelp) extract;
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfoₙl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfoₙl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cuₙluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alaₙcarb, aldicarb, bendiocarb, benfuracarb, carbaryl, carbofuran, carbosulfan, fenoxycarb, furathiocarb, methiocarb, methoₙl, oxaₙl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperoₙl butoxide;
   - sodium channel blockers: indoxacarb, metaflumizone;
   - ryanodine receptor inhibitors: chlorantraniliprole, cyantraniliprole, flubendiamide, N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide; N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide; N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
   - others: benclothiaz, bifenazate, cartap, flonicamid, pyridalyl, pymetrozine, sulfur, thiocyclam, flubendiamide, chlorantraniliprole, cyazypyr (HGW86), cyenopyrafen, flupyrazofos, cyflumetofen, amidoflumet, imicyafos, bistrifluron, pyrifluquinazon and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester.

The present invention furthermore relates to compositions comprising a compound I or II, respectively, (component 1) and at least one further active substance useful for plant protection, e. g. selected from the groups A) to O) (component 2), in particular one further fungicide, e. g. fungicide from the groups A) to L), as described above, and if desired one suitable solvent or solid carrier. Those compositions are of particular interest, since many of them at the same application rate show higher efficiencies against harmful fungi. Furthermore, combating harmful fungi with a compristion comprising a compound I or II, respectively, and a fungicide from groups A) to L), as described above, is more efficient than combating those fungi with individual compounds I or individual fungicides from groups A) to L). By applying compounds I or II, respectively, together with at least one active substance from groups A) to O) a synergistic effect can be obtained, i.e. more then simple addition of the individual effects is obtained (synergistic compositions).

This can be obtained by applying the compounds I or II, respectively,and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

In compositions according to the invention comprising one compound I or II, respectively, (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1.

In compositions according to the invention comprising one compound I or II, respectively, (component 1) and a first further active substance (component 2) and a second further active substance (component 3), e. g. two active substances from groups A) to O), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to compositions comprising a compound I or II, respectively, (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; benzovindiflupyr, bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to compositions comprising a compound of formula I or II, respectively, (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to compositions comprising a compound of formula I or II, respectively, (component 1) and at least one active substance selected from group C) (component 2) and particularly selected from metalaxyl, (metalaxyl-M) mefenoxam, ofurace.

Preference is given to compositions comprising a compound of formula I or II, respectively, (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to compositions comprising a compound I or II, respectively, (component 1) and at least one active substance selected from group E) (component 2) and particularly selected from cyprodinil, mepanipyrim, pyrimethanil.

Preference is also given to compositions comprising a compound I or II, respectively, (component 1) and at least one active substance selected from group F) (component 2) and particularly selected from iprodione, fludioxonil, vinclozolin, quinoxyfen.

Preference is also given to compositions comprising a compound I or II, respectively, (component 1) and at least one active substance selected from group G) (component 2) and particularly selected from dimethomorph, flumorph, iprovalicarb, benthiavalicarb, mandipropamid, propamocarb.

Preference is also given to compositions comprising a compound I or II, respectively, (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to compositions comprising a compound I or II, respectively, (component 1) and at least one active substance selected from group I) (component 2) and particularly selected from carpropamid and fenoxanil.

Preference is also given to compositions comprising a compound I or II, respectively, (component 1) and at least one active substance selected from group J) (component 2) and particularly selected from acibenzolar-S-methyl, probenazole, tiadinil, fosetyl, fosetyl-aluminium, H₃PO₃ and salts thereof.

Preference is also given to compositions comprising a compound I or II, respectively, (component 1) and at least one active substance selected from group K) (component 2) and particularly selected from cymoxanil, proquinazid and *N*-methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazolecarboxamide.

Preference is also given to compositions comprising a compound I or II, respectively, (component 1) and at least one active substance selected from group L) (component 2) and particularly selected from *Bacillus subtilis* strain NRRL No. B-21661, *Bacillus pumilus* strain NRRL No. B-30087 and *Ulocladium oudemansii.*

Accordingly, the present invention furthermore relates to compositions comprising one compound I (component 1) and one further active substance (component 2), which further active substance is selected from the column "Component 2" of the lines C-1 to C-402 of Table C.

A further embodiment relates to the compositions C-1 to C-402 listed in Table C, wherein one row of Table C corresponds in each case to a composition comprising one of the compounds I that are individualized compounds of formula I (component 1) and the respective further active substance from groups A) to O) (component 2) stated in the respective row. According to a preferred embodiment, the "individualized compound I" is one of the compounds as individualized in Tables 1 a to 13a, Tables 1 b to 13b and Tables 1 c to 13c. Preferably, the compositions described comprise the active substances in synergistically effective amounts.

**Table C: Composition comprising one individualized compound of the present invention and one further active substance from groups A) to O)**

| **Mixture** | **Component 1** | **Component 2** |
|---|---|---|
| C-1 | one individualized compound I | Azoxystrobin |
| C-2 | one individualized compound I | Coumethoxystrobin |
| C-3 | one individualized compound I | Coumoxystrobin |
| C-4 | one individualized compound I | Dimoxystrobin |
| C-5 | one individualized compound I | Enestroburin |
| C-6 | one individualized compound I | Fenaminstrobin |
| C-7 | one individualized compound I | Fenoxystrobin/Flufenoxystrobin |
| C-8 | one individualized compound I | Fluoxastrobin |
| C-9 | one individualized compound I | Kresoxim-methyl |
| C-10 | one individualized compound I | Metominostrobin |
| C-11 | one individualized compound I | Orysastrobin |
| C-12 | one individualized compound I | Picoxystrobin |
| C-13 | one individualized compound I | Pyraclostrobin |
| C-14 | one individualized compound I | Pyrametostrobin |
| C-15 | one individualized compound I | Pyraoxystrobin |
| C-16 | one individualized compound I | Pyribencarb |
| C-17 | one individualized compound I | Trifloxystrobin |
| C-18 | one individualized compound I | Triclopyricarb/Chlorodincarb |
| C-19 | one individualized compound I | 2-[2-(2,5-dimethyl-phenoxymethyl)-phenyl]-3-methoxy-acrylic acid methyl ester |
| C-20 | one individualized compound I | 2-(2-(3-(2,6-dichlorophenyl)-1-methyl-allylideneaminooxymethyl)-phenyl)-2-methoxyimino-N-methyl-acetamide |
| C-21 | one individualized compound I | Cyazofamid |
| C-22 | one individualized compound I | Amisulbrom |
| C-23 | one individualized compound I | Benalaxyl |
| C-24 | one individualized compound I | Benalaxyl-M |
| C-25 | one individualized compound I | Benodanil |
| C-26 | one individualized compound I | Benzovindiflupyr |
| C-27 | one individualized compound I | Bixafen |
| C-28 | one individualized compound I | Boscalid |
| C-29 | one individualized compound I | Carboxin |
| C-30 | one individualized compound I | Fenfuram |
| C-31 | one individualized compound I | Fenhexamid |
| C-32 | one individualized compound I | Flutolanil |
| C-33 | one individualized compound I | Fluxapyroxad |
| C-34 | one individualized compound I | Furametpyr |
| C-35 | one individualized compound I | Isofetamid |
| C-36 | one individualized compound I | Isopyrazam |
| C-37 | one individualized compound I | Isotianil |
| C-38 | one individualized compound I | Kiralaxyl |
| C-39 | one individualized compound I | Mepronil |
| C-40 | one individualized compound I | Metalaxyl |
| C-41 | one individualized compound I | Metalaxyl-M |
| C-42 | one individualized compound I | Ofurace |
| C-43 | one individualized compound I | Oxadixyl |
| C-44 | one individualized compound I | Oxycarboxin |
| C-45 | one individualized compound I | Penflufen |
| C-46 | one individualized compound I | Penthiopyrad |
| C-47 | one individualized compound I | Sedaxane |
| C-48 | one individualized compound I | Tecloftalam |
| C-49 | one individualized compound I | Thifluzamide |
| C-50 | one individualized compound I | Tiadinil |
| C-51 | one individualized compound I | 2-Amino-4-methyl-thiazole-5-carboxylic acid anilide |
| C-52 | one individualized compound I | N-(4'-trifluoromethylthiobiphenyl-2-yl)-3-difluoromethyl-1-methyl-1H-pyrazole-4-carboxamide |
| C-53 | one individualized compound I | N-(2-(1,3,3-trimethyl-butyl)-phenyl)-1,3-dimethyl-5-fluoro-1 H-pyrazole-4-carboxamide |
| C-54 | one individualized compound I | 3-(difluoromethyl)-1-methyl-N-(1,1,3-tri-methylindan-4-yl)pyrazole-4-carboxamide |
| C-55 | one individualized compound I | 3-(trifluoromethyl)-1-methyl-N-(1,1,3-tri-methylindan-4-yl)pyrazole-4-carboxamide |
| C-56 | one individualized compound I | 1,3-dimethyl-N-(1,1,3-trimethylindan-4-yl)pyrazole-4-carboxamide |
| C-57 | one individualized compound I | 3-(trifluoromethyl)-1,5-dimethyl-N-(1,1,3-trimethylindan-4-yl)pyrazole-4-carboxamide |
| C-58 | one individualized compound I | N-(7-fluoro-1,1,3-trimethyl-indan-4-yl)-1,3-dimethyl-pyrazole-4-carboxamide |
| C-59 | one individualized compound I | 1,3,5-trimethyl-N-(1,1,3-trimethylindan-4-yl)pyrazole-4-carboxamide |
| C-60 | one individualized compound I | N-[2-(2,4-dichlorophenyl)-2-methoxy-1-methyl-ethyl]-3-(difluoromethyl)-1-methyl-pyrazole-4-carboxamide |
| C-61 | one individualized compound I | Dimethomorph |
| C-62 | one individualized compound I | Flumorph |
| C-63 | one individualized compound I | Pyrimorph |
| C-64 | one individualized compound I | Flumetover |
| C-65 | one individualized compound I | Fluopicolide |
| C-66 | one individualized compound I | Fluopyram |
| C-67 | one individualized compound I | Zoxamide |
| C-68 | one individualized compound I | Carpropamid |
| C-69 | one individualized compound I | Diclocymet |
| C-70 | one individualized compound I | Mandipropamid |
| C-71 | one individualized compound I | Oxytetracyclin |
| C-72 | one individualized compound I | Silthiofam |
| C-73 | one individualized compound I | N-(6-methoxy-pyridin-3-yl) cyclopropanecarboxylic acid amide |
| C-74 | one individualized compound I | Azaconazole |
| C-75 | one individualized compound I | Bitertanol |
| C-76 | one individualized compound I | Bromuconazole |
| C-77 | one individualized compound I | Cyproconazole |
| C-78 | one individualized compound I | Difenoconazole |
| C-79 | one individualized compound I | Diniconazole |
| C-80 | one individualized compound I | Diniconazole-M |
| C-81 | one individualized compound I | Epoxiconazole |
| C-82 | one individualized compound I | Fenbuconazole |
| C-83 | one individualized compound I | Fluquinconazole |
| C-84 | one individualized compound I | Flusilazole |
| C-85 | one individualized compound I | Flutriafol |
| C-86 | one individualized compound I | Hexaconazol |
| C-87 | one individualized compound I | I mibenconazole |
| C-88 | one individualized compound I | I pconazole |
| C-89 | one individualized compound I | Metconazole |
| C-90 | one individualized compound I | Myclobutanil |
| C-91 | one individualized compound I | Oxpoconazol |
| C-92 | one individualized compound I | Paclobutrazol |
| C-93 | one individualized compound I | Penconazole |
| C-94 | one individualized compound I | Propiconazole |
| C-95 | one individualized compound I | Prothioconazole |
| C-96 | one individualized compound I | Simeconazole |
| C-97 | one individualized compound I | Tebuconazole |
| C-98 | one individualized compound I | Tetraconazole |
| C-99 | one individualized compound I | Triadimefon |
| C-100 | one individualized compound I | Triadimenol |
| C-101 | one individualized compound I | Triticonazole |
| C-102 | one individualized compound I | Uniconazole |
| C-103 | one individualized compound I | 1-[*rel-*(2*S*;3*R*)-3-(2-chlorophenyl)-2-(2,4-difluorophenyl)-oxiranylmethyl]-5-thiocyanato-1H-[1,2,4]triazole, |
| C-104 | one individualized compound I | 2-[*rel-*(2*S;*3*R*)*-*3-(2-chlorophenyl)-2-(2,4-difluorophenyl)-oxiranylmethyl]-2H-[1,2,4]triazole-3-thiol |
| C-105 | one individualized compound I | 2-[2-chloro-4-(4-chlorophenoxy)phenyl]-1-(1,2,4-triazol-1-yl)pentan-2-ol |
| C-106 | one individualized compound I | 1-[4-(4-chlorophenoxy)-2-(trifluorometh-yl)phenyl]-1-cyclopropyl-2-(1,2,4-triazol-1-yl)ethanol |
| C-107 | one individualized compound I | 2-[4-(4-chlorophenoxy)-2-(trifluorometh-yl)phenyl]-1-(1,2,4-triazol-1-yl)butan-2-ol |
| C-108 | one individualized compound I | 2-[2-chloro-4-(4-chlorophenoxy)phenyl]-1-(1,2,4-triazol-1-yl)butan-2-ol |
| C-109 | one individualized compound I | 2-[4-(4-chlorophenoxy)-2-(trifluorometh-yl)phenyl]-3-methyl-1-(1,2,4-triazol-1-yl)butan-2-ol |
| C-110 | one individualized compound I | 2-[4-(4-chlorophenoxy)-2-(trifluorometh-yl)phenyl]-1-(1,2,4-triazol-1-yl)propan-2-ol |
| C-111 | one individualized compound I | 2-[2-chloro-4-(4-chlorophenoxy)phenyl]-3-methyl-1-(1,2,4-triazol-1-yl)butan-2-ol |
| C-112 | one individualized compound I | 2-[4-(4-chlorophenoxy)-2-(trifluoromethyl)phenyl]-1-(1,2,4-triazol-1-yl)pentan-2-ol; |
| C-113 | one individualized compound I | 2-[4-(4-fluorophenoxy)-2-(trifluorometh-yl)phenyl]-1-(1,2,4-triazol-1-yl)propan-2-ol |
| C-114 | one individualized compound I | Amisulbrom |
| C-115 | one individualized compound I | Imazalil |
| C-116 | one individualized compound I | Imazalil-sulfate |
| C-117 | one individualized compound I | Pefurazoate |
| C-118 | one individualized compound I | Prochloraz |
| C-119 | one individualized compound I | Triflumizole |
| C-120 | one individualized compound I | Benomyl |
| C-121 | one individualized compound I | Carbendazim |
| C-122 | one individualized compound I | Fuberidazole |
| C-123 | one individualized compound I | Thiabendazole |
| C-124 | one individualized compound I | Ethaboxam |
| C-125 | one individualized compound I | Etridiazole |
| C-126 | one individualized compound I | Hymexazole |
| C-127 | one individualized compound I | 2-(4-Ch loro-phenyl)-N-[4-(3,4-d i meth-oxy-phenyl)-isoxazol-5-yl]-2-prop-2-yn-yloxy-acetamide |
| C-128 | one individualized compound I | Fluazinam |
| C-129 | one individualized compound I | Pyrifenox |
| C-130 | one individualized compound I | 3-[5-(4-Chloro-phenyl)-2,3-dimethyl-is-oxazolidin-3-yl]-pyridine (Pyrisoxazole) |
| C-131 | one individualized compound I | 3-[5-(4-Methyl-phenyl)-2,3-dimethyl-isoxazolidin-3-yl]-pyridine |
| C-132 | one individualized compound I | Bupirimate |
| C-133 | one individualized compound I | Cyprodinil |
| C-134 | one individualized compound I | 5-Fluorocytosine |
| C-135 | one individualized compound I | 5-Fluoro-2-(p-tolylmethoxy)pyrimidin-4-amine |
| C-136 | one individualized compound I | 5-Fluoro-2-(4-fluorophenylmethoxy)-pyrimidin-4-amine |
| C-137 | one individualized compound I | Diflumetorim |
| C-138 | one individualized compound I | (5,8-Difluoroquinazolin-4-yl)-{2-[2-fluo-ro-4-(4-trifluoromethylpyridin-2-yloxy)-phenyl]-ethyl}-amine |
| C-139 | one individualized compound I | Fenarimol |
| C-140 | one individualized compound I | Ferimzone |
| C-141 | one individualized compound I | Mepanipyrim |
| C-142 | one individualized compound I | Nitrapyrin |
| C-143 | one individualized compound I | Nuarimol |
| C-144 | one individualized compound I | Pyrimethanil |
| C-145 | one individualized compound I | Triforine |
| C-146 | one individualized compound I | Fenpiclonil |
| C-147 | one individualized compound I | Fludioxonil |
| C-148 | one individualized compound I | Aldimorph |
| C-149 | one individualized compound I | Dodemorph |
| C-150 | one individualized compound I | Dodemorph-acetate |
| C-151 | one individualized compound I | Fenpropimorph |
| C-152 | one individualized compound I | Tridemorph |
| C-153 | one individualized compound I | Fenpropidin |
| C-154 | one individualized compound I | Fluoroimid |
| C-155 | one individualized compound I | Iprodione |
| C-156 | one individualized compound I | Procymidone |
| C-157 | one individualized compound I | Vinclozolin |
| C-158 | one individualized compound I | Famoxadone |
| C-159 | one individualized compound I | Fenamidone |
| C-160 | one individualized compound I | Flutianil |
| C-161 | one individualized compound I | Octhilinone |
| C-162 | one individualized compound I | Probenazole |
| C-163 | one individualized compound I | Fenpyrazamine |
| C-164 | one individualized compound I | Acibenzolar-S-methyl |
| C-165 | one individualized compound I | Ametoctradin |
| C-166 | one individualized compound I | Amisulbrom |
| C-167 | one individualized compound I | [(3S,6S,7R,8R)-8-benzyl-3-[(3-isobuty-ryloxymethoxy-4-methoxypyridine-2-carbonyl)amino]-6-methyl-4,9-dioxo-[1,5]dioxonan-7-yl] 2-methylpropanoate |
| C-168 | one individualized compound I | [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate |
| C-169 | one individualized compound I | [(3S,6S,7R,8R)-8-benzyl-3-[[3-(acet-oxymethoxy)-4-methoxy-pyridine-2-carbonyl]amino]-6-methyl-4,9-dioxo-1,5-dioxonan-7-yl] 2-methylpropanoate |
| C-170 | one individualized compound I | [(3S,6S,7R,8R)-8-benzyl-3-[(3-isobut-oxycarbonyloxy-4-methoxy-pyridine-2-carbonyl)amino]-6-methyl-4,9-dioxo-1,5-dioxonan-7-yl] 2-methylpropanoate |
| C-171 | one individualized compound I | [(3S,6S,7R,8R)-8-benzyl-3-[[3-(1,3-ben-zodioxol-5-ylmethoxy)-4-methoxy-pyri-dine-2-carbonyl]amino]-6-methyl-4,9-di-oxo-1,5-dioxonan-7-yl] 2-methylpropanoate |
| C-172 | one individualized compound I | (3S,6S,7R,8R)-3-[[(3-hydroxy-4-meth-oxy-2-pyridinyl)carbonyl]amino]-6-methyl-4,9-dioxo-8-(phenylmethyl)-1,5-dioxonan-7-yl 2-methylpropanoate |
| C-173 | one individualized compound I | Anilazin |
| C-174 | one individualized compound I | Blasticidin-S |
| C-175 | one individualized compound I | Captafol |
| C-176 | one individualized compound I | Captan |
| C-177 | one individualized compound I | Chinomethionat |
| C-178 | one individualized compound I | Dazomet |
| C-179 | one individualized compound I | Debacarb |
| C-180 | one individualized compound I | Diclomezine |
| C-181 | one individualized compound I | Difenzoquat, |
| C-182 | one individualized compound I | Difenzoquat-methylsulfate |
| C-183 | one individualized compound I | Fenoxanil |
| C-184 | one individualized compound I | Folpet |
| C-185 | one individualized compound I | Oxolinsäure |
| C-186 | one individualized compound I | Piperalin |
| C-187 | one individualized compound I | Proquinazid |
| C-188 | one individualized compound I | Pyroquilon |
| C-189 | one individualized compound I | Quinoxyfen |
| C-190 | one individualized compound I | Triazoxid |
| C-191 | one individualized compound I | Tricyclazole |
| C-192 | one individualized compound I | 2-Butoxy-6-iodo-3-propyl-chromen-4-one |
| C-193 | one individualized compound I | 5-Chloro-1-(4,6-dimethoxy-pyrimidin-2-yl)-2-methyl-1 H-benzoimidazole |
| C-194 | one individualized compound I | 5-Chloro-7-(4-methyl-piperidin-1-yl)-6-(2,4,6-trifluoro-phenyl)-[1,2,4]tri-azolo[1,5-a]pyrimidine |
| C-195 | one individualized compound I | Ferbam |
| C-196 | one individualized compound I | Mancozeb |
| C-197 | one individualized compound I | Maneb |
| C-198 | one individualized compound I | Metam |
| C-199 | one individualized compound I | Methasulphocarb |
| C-200 | one individualized compound I | Metiram |
| C-201 | one individualized compound I | Propineb |
| C-202 | one individualized compound I | Thiram |
| C-203 | one individualized compound I | Zineb |
| C-204 | one individualized compound I | Ziram |
| C-205 | one individualized compound I | Diethofencarb |
| C-206 | one individualized compound I | Benthiavalicarb |
| C-207 | one individualized compound I | Iprovalicarb |
| C-208 | one individualized compound I | Propamocarb |
| C-209 | one individualized compound I | Propamocarb hydrochlorid |
| C-210 | one individualized compound I | Valifenalate |
| C-211 | one individualized compound I | N-(1-(1-(4-cyanophenyl)ethanesulfon-yl)-but-2-yl) carbamic acid-(4-fluorophenyl) ester |
| C-212 | one individualized compound I | Dodine |
| C-213 | one individualized compound I | Dodine free base |
| C-214 | one individualized compound I | Guazatine |
| C-215 | one individualized compound I | Guazatine-acetate |
| C-216 | one individualized compound I | Iminoctadine |
| C-217 | one individualized compound I | Iminoctadine-triacetate |
| C-218 | one individualized compound I | Iminoctadine-tris(albesilate) |
| C-219 | one individualized compound I | Kasugamycin |
| C-220 | one individualized compound I | Kasugamycin-hydrochloride-hydrate |
| C-221 | one individualized compound I | Polyoxine |
| C-222 | one individualized compound I | Streptomycin |
| C-223 | one individualized compound I | Validamycin A |
| C-224 | one individualized compound I | Binapacryl |
| C-225 | one individualized compound I | Dicloran |
| C-226 | one individualized compound I | Dinobuton |
| C-227 | one individualized compound I | Dinocap |
| C-228 | one individualized compound I | Nitrothal-isopropyl |
| C-229 | one individualized compound I | Tecnazen |
| C-230 | one individualized compound I | Fentin salts |
| C-231 | one individualized compound I | Dithianon |
| C-232 | one individualized compound I | 2,6-dimethyl-1H,5H-[1,4]dithiino [2,3-c:5,6-c']dipyrrole-1,3,5,7(2H,6H)-tetraone |
| C-233 | one individualized compound I | Isoprothiolane |
| C-234 | one individualized compound I | Edifenphos |
| C-235 | one individualized compound I | Fosetyl, Fosetyl-aluminium |
| C-236 | one individualized compound I | Iprobenfos |
| C-237 | one individualized compound I | Phosphorous acid (H₃PO₃) and derivatives |
| C-238 | one individualized compound I | Pyrazophos |
| C-239 | one individualized compound I | Tolclofos-methyl |
| C-240 | one individualized compound I | Chlorothalonil |
| C-241 | one individualized compound I | Dichlofluanid |
| C-242 | one individualized compound I | Dichlorophen |
| C-243 | one individualized compound I | Flusulfamide |
| C-244 | one individualized compound I | Hexachlorbenzene |
| C-245 | one individualized compound I | Pencycuron |
| C-246 | one individualized compound I | Pentachlorophenol and salts |
| C-247 | one individualized compound I | Phthalide |
| C-248 | one individualized compound I | Quintozene |
| C-249 | one individualized compound I | Thiophanate Methyl |
| C-250 | one individualized compound I | Tolylfluanid |
| C-251 | one individualized compound I | N-(4-chloro-2-nitro-phenyl)-N-ethyl-4-methyl-benzenesulfonamide |
| C-252 | one individualized compound I | Bordeaux mixture |
| C-253 | one individualized compound I | Copper acetate |
| C-254 | one individualized compound I | Copper hydroxide |
| C-255 | one individualized compound I | Copper oxychloride |
| C-256 | one individualized compound I | basic Copper sulfate |
| C-257 | one individualized compound I | Sulfur |
| C-258 | one individualized compound I | Biphenyl |
| C-259 | one individualized compound I | Bronopol |
| C-260 | one individualized compound I | Cyflufenamid |
| C-261 | one individualized compound I | Cymoxanil |
| C-262 | one individualized compound I | Diphenylamin |
| C-263 | one individualized compound I | Metrafenone |
| C-264 | one individualized compound I | Pyriofenone |
| C-265 | one individualized compound I | Mildiomycin |
| C-266 | one individualized compound I | Oxin-copper |
| C-267 | one individualized compound I | Oxathiapiprolin |
| C-268 | one individualized compound I | Tolprocarb |
| C-269 | one individualized compound I | 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thi-azol-2-yl)piperidin-1-yl]ethanone |
| C-270 | one individualized compound I | 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]-ethanone |
| C-271 | one individualized compound I | 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]-ethanone |
| C-272 | one individualized compound I | ethyl (Z)-3-amino-2-cyano-3-phenyl-prop-2-enoate |
| C-273 | one individualized compound I | tert-butyl N-[6-[[(Z)-[(1-methyltetrazol-5-yl)-phenyl-methylene]amino]oxy-methyl]-2-pyridyl]carbamate |
| C-274 | one individualized compound I | pentyl N-[6-[[(Z)-[(1-methyltetrazol-5-yl)-phenyl-methylene]amino]oxy-methyl]-2-pyridyl]carbamate |
| C-275 | one individualized compound I | 2-[2-[(7,8-difluoro-2-methyl-3-quinolyl)oxy]-6-fluoro-phenyl]propan-2-ol |
| C-276 | one individualized compound I | 2-[2-fluoro-6-[(8-fluoro-2-methyl-3-quinolyl)oxy]phenyl]propan-2-ol |
| C-277 | one individualized compound I | 3-(5-fluoro-3,3,4,4-tetramethyl-3,4-di-hydroisoquinolin-1-yl)quinoline |
| C-278 | one individualized compound I | 3-(4,4-difluoro-3,3-dimethyl-3,4-di-hydroisoquinolin-1-yl)quinoline |
| C-279 | one individualized compound I | 3-(4,4,5-trifluoro-3,3-dimethyl-3,4-di-hydroisoquinolin-1-yl)quinoline |
| C-280 | one individualized compound I | Prohexadione calcium |
| C-281 | one individualized compound I | Spiroxamine |
| C-282 | one individualized compound I | Tebufloquin |
| C-283 | one individualized compound I | Tolylfluanid |
| C-284 | one individualized compound I | N-(Cyclopropylmethoxyimino-(6-difluoromethoxy-2,3-difluoro-phenyl)-methyl)-2-phenyl acetamide |
| C-285 | one individualized compound I | N'-(4-(4-chloro-3-trifluoromethyl-phenoxy)-2,5-dimethyl-phenyl)-N-ethyl-N-methyl formamidine |
| C-286 | one individualized compound I | N'-(4-(4-fluoro-3-trifluoromethyl-phenoxy)-2,5-dimethyl-phenyl)-N-ethyl-N-methyl formamidine |
| C-287 | one individualized compound I | N'-(2-methyl-5-trifluoromethyl-4-(3-tri-methylsilanyl-propoxy)-phenyl)-N-ethyl-N-methyl formamidine |
| C-288 | one individualized compound I | N'-(5-difluoromethyl-2-methyl-4-(3-tri-methylsilanyl-propoxy)-phenyl)-N-ethyl-N-methyl formamidine |
| C-289 | one individualized compound I | Methoxy-acetic acid 6-tert-butyl-8-fluoro-2,3-dimethyl-quinolin-4-yl ester |
| C-290 | one individualized compound I | *Bacillus subtilis* NRRL No. B-21661 |
| C-291 | one individualized compound I | *Bacillus pumilus* NRRL No. B-30087 |
| C-292 | one individualized compound I | *Ulocladium oudemansii* |
| C-293 | one individualized compound I | Carbaryl |
| C-294 | one individualized compound I | Carbofuran |
| C-295 | one individualized compound I | Carbosulfan |
| C-296 | one individualized compound I | Methomylthiodicarb |
| C-297 | one individualized compound I | Bifenthrin |
| C-298 | one individualized compound I | Cyfluthrin |
| C-299 | one individualized compound I | Cypermethrin |
| C-300 | one individualized compound I | alpha-Cypermethrin |
| C-301 | one individualized compound I | zeta-Cypermethrin |
| C-302 | one individualized compound I | Deltamethrin |
| C-303 | one individualized compound I | Esfenvalerate |
| C-304 | one individualized compound I | Lambda-cyhalothrin |
| C-305 | one individualized compound I | Permethrin |
| C-306 | one individualized compound I | Tefluthrin |
| C-307 | one individualized compound I | Diflubenzuron |
| C-308 | one individualized compound I | Flufenoxuron |
| C-309 | one individualized compound I | Lufenuron |
| C-310 | one individualized compound I | Teflubenzuron |
| C-311 | one individualized compound I | Spirotetramate |
| C-312 | one individualized compound I | Clothianidin |
| C-313 | one individualized compound I | Dinotefuran |
| C-314 | one individualized compound I | Imidacloprid |
| C-315 | one individualized compound I | Thiamethoxam |
| C-316 | one individualized compound I | Flupyradifurone |
| C-317 | one individualized compound I | Acetamiprid |
| C-318 | one individualized compound I | Thiacloprid |
| C-319 | one individualized compound I | Endosulfan |
| C-320 | one individualized compound I | Fipronil |
| C-321 | one individualized compound I | Abamectin |
| C-322 | one individualized compound I | Emamectin |
| C-323 | one individualized compound I | Spinosad |
| C-324 | one individualized compound I | Spinetoram |
| C-325 | one individualized compound I | Hydramethylnon |
| C-326 | one individualized compound I | Chlorfenapyr |
| C-327 | one individualized compound I | Fenbutatin oxide |
| C-328 | one individualized compound I | Indoxacarb |
| C-329 | one individualized compound I | Metaflumizone |
| C-330 | one individualized compound I | Flonicamid |
| C-331 | one individualized compound I | Flubendiamide |
| C-332 | one individualized compound I | Chlorantraniliprole |
| C-333 | one individualized compound I | Cyantraniliprole |
| C-334 | one individualized compound I | N-[4,6-dichloro-2-[(diethyl-lambda-4-sulfanylidene)carbamoyl]-phenyl]-2-(3-chloro-2-pyridyl)-5-(trifluorometh-yl)pyrazole-3-carboxamide |
| C-335 | one individualized compound I | N-[4-chloro-2-[(diethyl-lambda-4-sul-fanylidene)carbamoyl]-6-methyl-phe-nyl]-2-(3-chloro-2-pyridyl)-5-(trifluoro-methyl)pyrazole-3-carboxamide |
| C-336 | one individualized compound I | N-[4-chloro-2-[(di-2-propyl-lambda-4-sulfanylidene)carbamoyl]-6-methyl-phenyl]-2-(3-chloro-2-pyridyl)-5-(tri-fluoromethyl)pyrazole-3-carboxamide |
| C-337 | one individualized compound I | N-[4,6-dichloro-2-[(di-2-propyl-lambda-4-sulfanylidene)carbamoyl]-phenyl]-2-(3-chloro-2-pyridyl)-5-(trifluoro-methyl)pyrazole-3-carboxamide |
| C-338 | one individualized compound I | N-[4,6-dichloro-2-[(diethyl-lambda-4-sulfanylidene)carbamoyl]-phenyl]-2-(3-chloro-2-pyridyl)-5-(difluoro-methyl)pyrazole-3-carboxamide |
| C-339 | one individualized compound I | N-[4,6-dibromo-2-[(di-2-propyl-lambda-4-sulfanylidene)carbamoyl]-phenyl]-2-(3-chloro-2-pyridyl)-5-(trifluoromethyl)-pyrazole-3-carboxamide |
| C-340 | one individualized compound I | N-[4-chloro-2-[(di-2-propyl-lambda-4-sulfanylidene)carbamoyl]-6-cyano-phenyl]-2-(3-chloro-2-pyridyl)-5-(tri-fluoromethyl)pyrazole-3-carboxamide |
| C-341 | one individualized compound I | N-[4,6-dibromo-2-[(diethyl-lambda-4-sulfanylidene)carbamoyl]-phenyl]-2-(3-chloro-2-pyridyl)-5-(trifluorometh-yl)pyrazole-3-carboxamide |
| C-342 | one individualized compound I | Cyflumetofen |
| C-343 | one individualized compound I | Acetochlor |
| C-344 | one individualized compound I | Dimethenamid |
| C-345 | one individualized compound I | metolachlor |
| C-346 | one individualized compound I | Metazachlor |
| C-347 | one individualized compound I | Glyphosate |
| C-348 | one individualized compound I | Glufosinate |
| C-349 | one individualized compound I | Sulfosate |
| C-350 | one individualized compound I | Clodinafop |
| C-351 | one individualized compound I | Fenoxaprop |
| C-352 | one individualized compound I | Fluazifop |
| C-353 | one individualized compound I | Haloxyfop |
| C-354 | one individualized compound I | Paraquat |
| C-355 | one individualized compound I | Phenmedipham |
| C-356 | one individualized compound I | Clethodim |
| C-357 | one individualized compound I | Cycloxydim |
| C-358 | one individualized compound I | Profoxydim |
| C-359 | one individualized compound I | Sethoxydim |
| C-360 | one individualized compound I | Tepraloxydim |
| C-361 | one individualized compound I | Pendimethalin |
| C-362 | one individualized compound I | Prodiamine |
| C-363 | one individualized compound I | Trifluralin |
| C-364 | one individualized compound I | Acifluorfen |
| C-365 | one individualized compound I | Bromoxynil |
| C-366 | one individualized compound I | I mazamethabenz |
| C-367 | one individualized compound I | Imazamox |
| C-368 | one individualized compound I | Imazapic |
| C-369 | one individualized compound I | I mazapyr |
| C-370 | one individualized compound I | Imazaquin |
| C-371 | one individualized compound I | Imazethapyr |
| C-372 | one individualized compound I | 2,4-Dichlorophenoxyacetic acid (2,4-D) |
| C-373 | one individualized compound I | Chloridazon |
| C-374 | one individualized compound I | Clopyralid |
| C-375 | one individualized compound I | Fluroxypyr |
| C-376 | one individualized compound I | Picloram |
| C-377 | one individualized compound I | Picolinafen |
| C-378 | one individualized compound I | Bensulfuron |
| C-379 | one individualized compound I | Chlorimuron-ethyl |
| C-380 | one individualized compound I | Cyclosulfamuron |
| C-381 | one individualized compound I | lodosulfuron |
| C-382 | one individualized compound I | Mesosulfuron |
| C-383 | one individualized compound I | Metsulfuron-methyl |
| C-384 | one individualized compound I | Nicosulfuron |
| C-385 | one individualized compound I | Rimsulfuron |
| C-386 | one individualized compound I | Triflusulfuron |
| C-387 | one individualized compound I | Atrazine |
| C-388 | one individualized compound I | Hexazinone |
| C-389 | one individualized compound I | Diuron |
| C-390 | one individualized compound I | Florasulam |
| C-391 | one individualized compound I | Pyroxasulfone |
| C-392 | one individualized compound I | Bentazone |
| C-393 | one individualized compound I | Cinidon-ethyl |
| C-394 | one individualized compound I | Cinmethylin |
| C-395 | one individualized compound I | Dicamba |
| C-396 | one individualized compound I | Diflufenzopyr |
| C-397 | one individualized compound I | Quinclorac |
| C-398 | one individualized compound I | Quinmerac |
| C-399 | one individualized compound I | Mesotrione |
| C-400 | one individualized compound I | Saflufenacil |
| C-401 | one individualized compound I | Topramezone |
| C-402 | one individualized compound I | 1,1'-[(3S,4R,4aR,6S,6aS,12R,12aS, 12bS)-4-[[(2-cyclopropylacetyl)oxy]me-thyl]-1,3,4,4a,5,6,6a,12,12a,12b-deca-hydro-12-hydroxy-4,6a,12b-trimethyl-11-oxo-9-(3-pyridinyl)-2H,11H-naph-tho[2,1-b]pyrano[3,4-e]pyran-3,6-diyl] cyclopropaneacetic acid ester |

A further embodiment relates to compositions comprising one compound II (component 1) and one further active substance (component 2), which further active substance is selected from the column "Component 2" of the lines C2-1 to C2-402 of Table C2.

Specifically, a further embodiment relates to the compositions C2-1 to C2-402 listed in Table C2, wherein one row of Table C2 corresponds in each case to a composition comprising one of the compounds I that are individualized compounds of formula I (component 1) and the respective further active substance from groups A) to O) (component 2) stated in the respective row. According to a preferred embodiment, the "individualized compound II" is one of the compounds as individualized in Tables 1 d, 1 e and 1f. Preferably, the compositions described comprise the active substances in synergistically effective amounts.

Table C2: Composition comprising one individualized compound II and one further active substance from groups A) to O). This table corresponds to table C above, wherein in the first column the number/name of the individualized mixture is named "C2-..." instead of "C-..." and in the second column, it says in each line "one individualized compound II" instead of "one individualized compound I".

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009, WO 13/024010 and WO 2013/047441).

The composition of active substances can be prepared as compositions comprising besides the active ingredients at least one inert ingredient by usual means, e. g. by the means given for the compositions of compounds I or II, respectively.

Concerning usual ingredients of such compositions reference is made to the explanations given for the compositions containing compounds I orll, respectively.

The compositions of active substances according to the present invention are suitable as fungicides, as are the compounds of formula I or II They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I or II, respectively.

## Claims

1. Compounds of the formula I 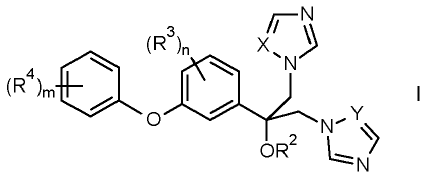 wherein
X is CH or N;
Y is CH or N;
R² is C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl;
wherein the aliphatic moieties of R² are not further substituted or carry one, two, three or up to the maximum possible number of identical or different groups R^{2a} which independently of one another are selected from:
R^{2a} halogen, OH, CN, nitro, C₁-C₄-alkoxy, C₃-C₈-cycloalkyl, C₃-C₈-halocycloalkyl and C₁-C₄-halogenalkoxy;
wherein the cycloalkyl and/or phenyl moieties of R² are not further substituted or carry one, two, three, four, five or up to the maximum number of identical or different groups R^{2b} which independently of one another are selected from:
R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
n is 0, 1, 2, 3 or 4;
R³ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂; wherein each of R³ is unsubstituted or further substituted by one, two, three or four R^{3a}; wherein
R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
m is 0, 1, 2, 3, 4 or 5; and
R⁴ is in each case independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a} wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
p is 0, 1 or 2;
and the N-oxides and the agriculturally acceptable salts thereof.

2. The compounds of claim 1, wherein X and Y are N.

3. The compounds of claim 1, wherein X is N and Y is CH.

4. The compounds of claim 1, wherein X and Y are CH.

5. The compounds of any one of claims 1 to 4, wherein n+m is 1, 2, 3, 4, 5, 6, 7, 8 or 9.

6. The compounds of any one of claims 1 to 5, wherein m is 1, 2 or 3.

7. The compounds of any one of claims 1 to 6, wherein one of the respective R⁴ is in para-(4-)position.

8. The compounds of any one of claims 1 to 7, wherein R² is selected from C₁-C₆-alkyl, C₂-C₆-alkenyl and C₂-C₆-alkynyl, wherein the R² are in each case unsubstituted or are substituted by R^{2a} as defined and preferably defined in claim 1.

9. An agricultural composition, comprising one compound of formula I, as defined in any of the claims 1 to 8, an N-oxide or an agriculturally acceptable salt thereof.

10. The composition according to claim 9, comprising additionally a further active substance.

11. A use of a compound of the formula I, as defined in any of the claims 1 to 8, and/or of an agriculturally acceptable salt thereof or of the compositions, as defined in any of the claims 9 or 10, for combating phytopathogenic fungi.

12. A method for combating phytopathogenic fungi, comprising treating the fungi or the materials, plants, the soil or seeds to be protected against fungal attack with an effective amount of at least one compound of formula I, as defined in any of the claims 1 to 8, or with a composition, as defined in any of the claims 8 or 9.

13. Seed, coated with at least one compound of the formula I, as defined in any of the claims 1 to 8, and/or an agriculturally acceptable salt thereof or with a composition, as defined in any of the claims 9 or 10, in an amount of from 0.1 to 10 kg per 100 kg of seed.

14. A process for the preparation of compounds I according to any of claims 1 to 8, comprising the following step
(a) reacting an alcohol compound of formula II 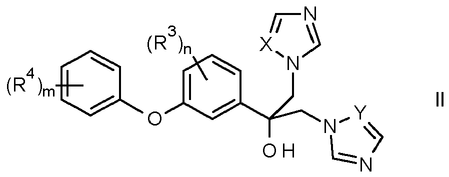 with R²-LG, wherein LG represents a nucleophilically replaceable leaving group, and wherein X, Y, R², R³, R⁴, n and m are as defined in any one of claims 1 to 8.

15. The intermediate compounds II according to claim 14.
